# EUROPEAN PATENT APPLICATION

(11) **EP 0 518 675 A2**
(43) Date of publication of application: **16.12.1992**
(21) Application number: 92305383.9
(22) Date of filing: 11.06.1992
(51) Int. Cl.: C07K 5/02, C07K 5/06, C07D 273/00, C07D 413/12, C07D 267/00, C07D 453/02, C07D 323/00

(54) **Cyclic renin inhibitors**

(30) Priority: 11.06.1991 US 714112
(71) Applicant: MERCK & CO. INC., Rahway New Jersey 07065-0900 (US)
(72) Inventor: Greenlee, William J., Teaneck, NJ 07666 (US); Patchett, Arthur A., Westfield, NJ 07090 (US); Weber, Ann E., Scotch Plains, NJ 07076 (US); Tata, James R., Westfield, NJ 07090 (US)
(74) Representative: Thompson, John Dr.

(57) **Abstract**

Compounds of the formula : 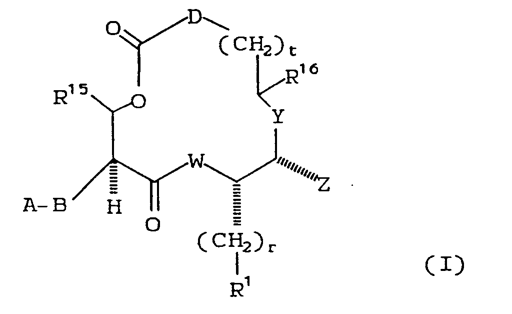
are disclosed. These compounds inhibit the angiotensinogen-cleaving action of the natural proteolytic enzyme, renin, and are useful in treating, preventing or managing renin-associated hypertension, hyperaldosteronism, congestive heart failure, and glaucoma.

## Description

### BACKGROUND OF THE INVENTION

### 1) Field of the Invention

The present invention is concerned with novel compounds I which inhibit the angiotensinogen-cleaving action of the natural proteolytic enzyme, renin, with pharmaceutical compositions containing the novel peptides of the present invention as active ingredients, with methods of treating, preventing or managing renin-associated hypertension, hyper-aldosteronism, and congestive heart failure, with diagnostic methods which utilize the novel compounds I of the present invention, as well as processes therefor. It also includes within its scope methods for treating HIV infections.

Renin is an endopeptidase (molecular weight about 40,000) produced and secreted by the juxtaglomerular cells of the kidney, which cleaves the naturally-occurring plasma glycoprotein, antiotensinogen, specifically at the 10, 11 peptide bond, i.e., between Leu 10 and Leu 11 in the equine substrate, as described by Skeggs et ai, J. Exper. Med. 1957,106,439, or between the Leu 10 and Val 11 in the human renin substrate, as elucidated by Tewksbury et al., Circulation 59, 60, Supp. II: 132, Oct. 1979. Renin cleaves angiotensinogen, its protein substrate, to split off the hemodynamically-inactive decapeptide, angiotensin I, which is converted in the lungs, kidney or other tissue by angiotensin-converting enzyme to the potent pressor octapeptide, angiotensin II. Angiotensin II is then believed to cause constriction of the arterioles and to stimulate release of the sodium-retaining hormone, aldosterone, from the adrenal gland and thereby cause arise in extracellular fluid volume. Thus, the renin-angiotensin system plays an important role in normal cardiovascular homeostasis and in some forms of elevated blood pressure (hypertension).

Inhibitors of angiotensin I converting enzyme have proven useful in the modulation of the renin-angiotensin system. Consequently, specific inhibitors of the limiting enzymatic step that ultimately regulates angiotensin II production, the action of renin on its substrate, have also been sought as effective investigative tools, as well as therapeutic agents in the treatment of hypertension and congestive heart failure.

The compounds of the present invention also exhibit inhibitor activity against HIV protease and are thus useful in the prevention of infection by the human immunodeficiency virus (HIV) and the treatment of consequent pathological conditions such as AIDS. Treating AIDS or preventing infection by HIV is defined as including, but not limited to, treating a wide range of manifestations of HIV infection: AIDS, ARC (AIDS related complex), both symptomatic and asymtomatic, and mere exposure to HIV. For example, the compounds of this invention are useful in preventing infection by HIV after suspected past exposure to HIV by e.g., blood transfusion, accidental needle stick, or exposure to patient blood during surgery.

Several cyclic renin inhibitor designs have been reported in the literature. In general, the aim of the studies reported was to use the conformational constraints imposed by the cyclic structures to help define the conformation of substrates and inhibitors as they bind to renin. None of these publications set forth possible advantages for inhibitors of this type or claim or establish any advantage for these cyclic inhibitors over their acyclic counterparts.

Early cyclic inhibitor designs used 18-membered or 20-membered rings to enclose a Pro-Phe beta-turn postulated to occur in bound substrate, and yielded inhibitors with moderate potency, comparable to that of acyclic analogs (C. L. Nakaie, M. C. F. Oliveira, L. Juliano, J. L. Pesquero and A. C. M. Paiva in Peptides , Structure and Function. proceedings of the Eighth American Peptide Symposium, V. J. Hruby, and D. H. Rich, Eds., Pierce Chemical Co., Rockford, IL, 1983, p. 595; C. R. Nakaie, J. L. Pesquero, M. C. F. Oliveira, L. Juliano and A. C. M. Paiva, in Peptides, Structure and Function. Proceedings of the Ninth American Peptide Symposium, C. M. Dever, V. J. Hruby and K. D. Kopple, Eds., pierce Chemical Co., Rockford, IL, 1985, p. 755).

Pairs of cysteine side-chains (P₂-P₂' and P₄-P₂' pairs) have been linked in high molecular weight cyclic inhibitor structures which are based on the Pᵢ-Pᵢ' Phe-Phe sequence, statine, or a reduced peptide isostere. Only the cyclic inhibitors with a phe-Phe sequence replacing the scissile bond of substrate show potency comparable to acyclic analogs (T. K. Sawyer, D. T. Pals, C. W. Smith, H. S. Saneii, D. E. Epps, D. J. Duchamp, J. B. Hester, R. E. TenBrink, D. J. Staples, A. E. deVaux, J. A. Affholter, G. F. Skala, W. M. Kati, J. A. Lawson, M. R. Schuette, B. V. Kamdar and D. E. Emmert in Peptides, Structure and Function. Proceedings of the Ninth American Peptide Symposium, C. M. Deber, V. J. Hruby and K. D. Kopple, Eds., Pierce Chemical Co., Rockford, IL, 1985, p. 729).

Two cyclic inhibitor designs investigated by Boger et al., incorporated disulfides constructed from P_{Z} toward the carboxy terminus, and these had potency comparable to that of an acyclic analog. An amino-terminal cyclic disulfide inhibitor made by connecting P₅ and P₂ homocysteine sidechains encloses a Pro-phe beta-turn. The optimal ring size for a P₅-P₂ cycle is found in the 16-membered ring inhibitor, and three other disulfide cycles with cysteine at either P₅ or P₂ (or both), were substantially less potent (J. Boger in Aspartic proteinases and Their Inhibitors, V. Kostka, Ed., Walter de Gruyter, Berlin, 1985, p. 401; J. Boger in Proceedings of the Third SCI-RSC Medicinal Chemistry Symposium; Special Publication No. 55 of the Royal Society of Chemistry, R. W. Lambert, Ed., Burlington House, London WIV OBN, 1986, p. 271). Please see also U.S. Patents 4,477,440 and 4,477,441.

A series of renin inhibitors in which the P₁ side-chain of a "reduced peptide" inhibitor is cyclized onto the alpha-nitrogen atom of alanine at P₂ has been reported (H. Sham, G. Bolis, H. H. Stein, S. W. Fesik, P. A. Mar- cotte, J. J. Plattner, C. A. Rempel and J. Greer, J. Med. Chem., 31, 284 (1988), but these have only moderate potency.

Although in some of the cases cited above, the ring-size of the cyclic element of the renin inhibitors cited above is similar to those of the cyclic renin inhibitors disclosed herein, the inhibitors of the present case are structurally distinct, and unlike other cyclic renin inhibitors have low molecular weight, show high in vitro potency against human renin, and are orally active.

### DETAILED DESCRIPTION OF THE INVENTION AND PREFERRED EMBODIMENTS

In accordance with the present invention, there are provided novel compounds of the formula I: 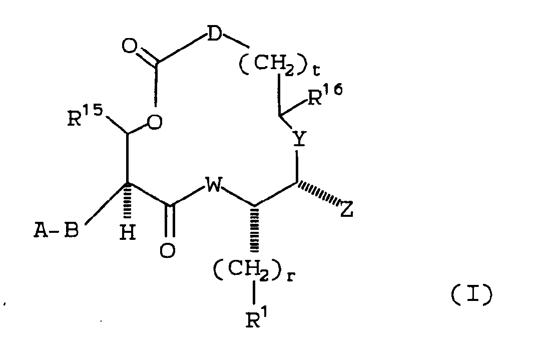
wherein:
A is hydrogen,
Het,
where Het is a saturated or unsaturated 5 to 7-membered monocyclic or 7 to 10-membered bicyclic ring which contains at least one and up to two nitrogen atoms (optionally quaternized or in the N-oxide form),
where Het may optionally be benzofused,
where Het may optionally contain one additional ring atom chosen from among the list consisting of O or S, in sulfide, sulfoxide or sulfone form,
where Het may optionally be substituted with one or two Het substituents independently selected from the group consisting of -OH, C₁-C₄-alkyl, -CF₃, -CN, C₁-C₄-alkoxy, C₁-C₄-alkoxy-C₁-C₄-alkoxy, halo, -NH₂, mono- or di-(C₁-C₄-alkyl)amino, -CO₂H, -CO₂-C₁-C₄-alkyl, -CONR^{2a}R^{2b}, -SO₃H, C₁-C₄-alkyl-CO-, aryl, (where aryl is unsubstituted or mono-, di-, or trisubstituted phenyl or naphthyl wherein the substitutent(s) is/are independently selected from the group consisting of C₁-C₈-alkyl, amino, phenyl-C₁-C₄-alkyl, mono- or di-C₁-C₄-alkyl amino, amino-C₁-C₄-alkyl, mono- or di-C₁-C₄-alkylamino-C₁-C₄-alkyl, guanidyl, guanidyl-C₁-C₄-alkyl, -OH, C₁-C₄-alkoxy, -CONR^{2a}R^{2b}, -CO₂H, -C0₂-C₁-C₄-alkyl, -CF₃, halo, C₁-C₄-alkyl-CO-, C₁-C₄-alkyl-CONH-, tri-(C₁-C₄- alkyl)N⁺X⁻, where X―is a counterion selected from the group consisting of single negatively charged ions, such as chloride, bromide, nitrate, perchlorate, benzoate, maleate, benzenesulfonate, methanesulfonate, tartrate, hemitartrate, and acetate) and mono- or disubstituted C₁-C₄-alkyl, (where the substitutent(s) is/are independently selected from the group consisting of -CO₂H, -CO₂-C₁-C₅-alkyl, C₁-C₅-alkyl-CONH-, -OH, -SO₃H, C₁-C₄-alkyl-S0₂-, C₁-C₄-alkyl-SO-, -S0₂NHCO-C₁-C₄-alkyl, C₁-C₅-alkyl-OCONH- and aryl as defined above),
where if one or both N are quaternized in Het, then each nitrogen atom may be quaternized with a Het substitutent cited above selected from the group consisting of -C₁-C₄-alkyl, -CF₃, aryl, and mono- or disubstituted C₁-C₄-alkyl with the corresponding counterion being X― as defined above,
where Het may have in the alternative to the above Het substituents, a Het substituent selected from the group consisting of -(CH₂)q- and -(CH₂)₂O(CH₂)₂- which forms a quaternary spirocyclic ring with the N atom wherein q is 3-to-6 and the counterion is X‾ as defined above,
where Het may be substituted both with one Het substituent chosen from among those listed above and also with up to four Het substituents selected from the group consisting of C₁-C₂-alkyl substituents (for example where A is 3,3,5,5-tetramethyl-4-benzylpiperidin-4-yl), and Het-C₁-C₄-alkyl (where Het is as defined above without optional substitution and where the alkyl group is optionally substituted with one or two substituents independently selected from the group consisting of hydroxyl, -C0₂H, -C0₂-C₁-C₄-alkyl, -S0₃H, and aryl where aryl is as defined above),
aryl,
where aryl is defined above,
R²CO-,
where R² is unsubstituted or mono- or disubstituted C₁-C₄-alkyl where the substituent(s) is/are selected from the group consisting of C₁-C₄-alkyl, -S0₃H, aryl or aryl-CO- (where aryl is as defined above), Het or Het-CO- (where Het is as defined above), R^{2a}O-, R^{2a}OCO-, R²ₐR^{2b}N-, R²ₐR^{2b}NCO-, R²aR^{2b}NCONH-, R2a_{R}2bNS0₂-, (R²ₐO)(R^{2b}O)PO-, R²_{c}S-, R²_{c}SO-, R2cSO₂-, R²_{c}CONH-, R2cOCONH-, and -N(R¹⁷R¹⁸R¹⁹)⁺X‾(where R²a and R^{2b} are independently hydrogen, C₁-C₄-alkyl, aryl as defined above, Het as defined above, R²_{c} is C₁₋₄-alkyl, aryl as defined above or Het as defined above, R¹⁹ is C₁-C₄-alkyl, R¹⁷ and R¹⁸ are independently aryl as defined above, Het as defined above or C₁-C₄-alkyl optionally substituted with a substituent chosen from the group consisting of aryl as defined above, Het as defined above, -OH, -NH₂, -NH-C₁-C₄-alkyl, -N(C₁-C₄-alkyl)₂, -CO₂H, -C0₂-C₁-C₄-alkyl, -S0₃H, -CO-NH-SO₂-C₁-C₄-alkyl, and -CO-NH-SO₂-aryl, and X‾ is as defined above),
R²⁻ (where R² is defined above),
R²0CO- (where R² is as defined above),
R²SO₂- (where R² is as defined above),
Aryl-CO- (where aryl is as defined above),
Het-CO- (where Het is as defined above),
R2a_{R2bN}-_{CO}- (where R²a and R^{2b} are as defined above),
R²e(CH₂)ᵣN-CO- where r, R²a is as defined above and R²e is het-CO where Het is as defined above or Het SO₂-,
R²aR^{2b}N-S0₂- (where R²a and R^{2b} are as defined above), and
C₁-C₄-alkyl-(OCH₂CH₂)ₓOCO- (where x is 1 to 3); B is -CH₂-CH[(CH₂)rR³]CON(R")--N(A¹)CH[(CH₂)ᵣR³]CO-N(R¹¹), -O-CH[(CH₂)ᵣR³]CO-N(R¹¹), -N(A¹)CH[(CH₂)ᵣR³]-CO-O-, -O-CH[(CH₂)ᵣR³]CO-O- or -N(A¹)CH[(CH₂)ᵣR³]CH(OH)CH₂-,
where r is 0-to-2,
A¹ is hydrogen or C₁-C₄-alkyl,
R³ is hydrogen, C₁-C₄-alkyl,
C₃-C₇-cycloalkyl, aryl as defined above, Het as defined above or 4-(morpholin-4-yl)ethoxy-phenyl, and
R¹¹ is hydrogen or C₁-C₄-alkyl;
A and B together may alternatively be:
   G-CH₂CH[(CH₂)ᵣR³]-Q-N(R¹¹)-, G-CH₂CH[(CH₂)ᵣR³]-CO-O-, Het-S(O)ₘCH[(CH₂)ᵣR³]CON(R¹¹)-, (where r, R³, R¹¹ and Het are as defined above and Q is -CO- or -S0₂-), R^{2d}CON(R¹¹)-, R^{2d}OCON(R¹¹)-, R^{2d}CO-O-, R^{2d}SO₂N(R¹¹)-, (where R^{2d} is Het as defined above, aryl as defined above, or C₁-C₄-alkyl or C₂-C₄-alkenyl substituted with Het, Het-O-, aryl, or aryl-O-, each as defined above), 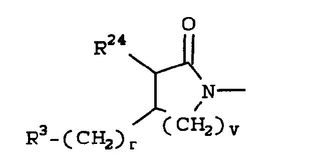
      or 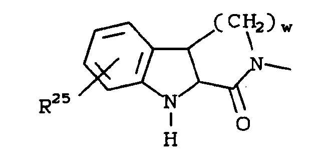
      (where v is 1-to-3, w is 1 or 2, R²⁵ is C₁-C₄-alkyl, amino, mono- or di-C₁-C₄-alkylamino, -OH, C₁-C₄-alkoxy, -CO₂H, -C0₂-C₁-C₄-alkyl, -CONR^{2a}R^{2b}, -CF₃, halo, -NHCO-O-C₁-C₄-alkyl, -N(C₁-C₄-alkyl)CO-O-C₁-C₄-alkyl, -NHCO-C₁-C₄-alkyl or -N(C₁-C₄-alkyl)CO-C₁-C₄-alkyl, R³ and r are as defined above, R²⁴ is hydrogen, C₁-C₄-alkyl, or is A-N(H)- where A is independently selected from the definition of A as defined above);
   G is
   R²⁰⁻S(O)ₘ- (where m is 0-to-2 and R²⁰ is C₃-C₇-cycloalkyl, aryl as defined above, Het as defined above or C₁-C₄-alkyl optionally substituted with one or two substituents chosen from the group consisting of C₁-C₄- alkoxy, -OH, -CO₂H, -C0₂-C₁-C₄-alkyl, -NH₂, -NH(C₁-C₄-alkyl), -N(C₁-C₄-alkyl)₂ and (C₁-C₄-alkyl)CO-O-), R¹⁷R¹⁸NS0₂- (where R¹⁷ and R¹⁸ are as defined above), 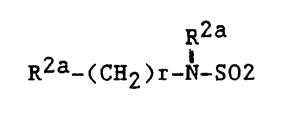
      where r, R²a and R²e are as defined above, or 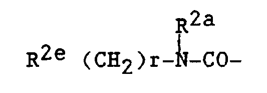
      where r, R²a and R²e are as defined above; R²⁰CO- (where R²⁰ is as defined above), R²⁰OCO- (where R²⁰ is as defined above) or -CH(OH)CH₂Het (where Het is defined above);
   A and B together may be -J-CH[(CH₂)ᵣ-R³]-K-, where
   K is
      -CH₂-,
      -CH(OH)-,
      -CO-,
      -NH-,
      -O-,
      -S-,
      -SO-,
      -SO₂-,
      -NO-,
      -P(O)O-;
   J is
   R²⁶⁻CO-(CH₂)_{d} (where d is 0 to 4, R²⁶ is -OH, -0-C₁-C₆-alkyl, -NR¹⁸R¹⁸, Het), R²⁷-SO₂ (where R²⁷ is -C₁-C₄-alkyl, aryl, Het), R²⁸, where R²⁸ is aryl, Het, C₁-C₄-alkyl optionally substituted with aryl, Het, -CO₂H, -C0₂-C₁-C₄-alkyl, -S0₂-C₁-C₄-alkyl, -SO₂Ar, -SO₂Het), R²⁸⁻ NH-CO- where R²⁸ is as defined above; R¹ is
   C₁-C₄-alkyl, aryl as defined above, unsubstituted, di-, or trisubstituted C₃-C₇-cydoalkyl, (where the substituents is/are selected from the group consisting of C₁-C₄-alkyl, trifluoromethyl, -OH, C₁-C₄-alkoxy, or halo) or a 5- or 6-membered ring saturated heterocycle containing one or two heteroatoms selected from the group consisting of N, O or S, optionally substituted with one or two substituents (where the substituents is/are selected from the group consisting of C₁-C₄-alkyl, C₁-C₄-alkoxy, halo, -NH₂ or -OH);
   R¹⁵ is
   C₁-C₄-alkyl, aryl as defined above, imidazol-4-yl, thiazol-4-yl or thiazol-5-yl;
   t is 1-to-4;
   R¹⁶ is
   hydrogen or
   C₁-C₄-alkyl optionally substituted with a substituent chosen from among the group consisting of C₁-C₄- alkyl, C₃-C₇-cycloalkyl, aryl as defined above, Het as defined above, -OH, -S0₃H, -CO₂H, CO₂-C₁-C₄-alkyl, -CO-Het, -NR¹⁷R¹⁸, -NHR¹⁸, -N(R¹⁷R¹⁸R¹⁹)⁺X⁻ (where X-, R¹⁷, R¹⁸ and R¹⁹ as defined above), -S(O)ₘ-R²¹ (where m is as defined above and R²¹ is Het, aryl or C₁-C₄-alkyl the alkyl optionally substituted with a substituent chosen from among the group consisting of aryl, Het, -NH₂, -OH, -NH-C₁-C₄-alkyl or -N(C₁-C₄-alkyl)₂ ), -SO₂NH₂, -SO₂NR¹⁷R¹⁸ (where R¹⁷ and R¹⁸ are as defined above), -SO₂NHR^{1B} (where R¹⁸ is as defined above) and -CH₂(OCH₂CH₂)ₓ-OC₁-C₄-alkyl (where x is as defined above);
   Y is -OCO-, -CH₂CO- or -CH₂CH(OH)- (where the Y substituent is inserted into formula I clockwise from left to right);
   W is O or NR²³, where R²³ is -H or C₁-C₄-alkyl;
   Z is -NH₂, -OH, -OP0₃H₂, -OCOR²², -OCO-OR²² (where R²² is 5-indanyl or C₁-C₆-alkyl optionally substituted with Ph, -S0₃H, -CO₂H, -P0₃H₂, -NH₂, -NH(C₁-C₄-alkyl), -N(C₁-C₄-alkylh, -N(C₁-C₄-alkyl)₃⁺X⁻ where X- is defined above), -OCHR22a-_{OCOR}22b (where R²²a and R^{22b} are C₁-C₄-alkyl), 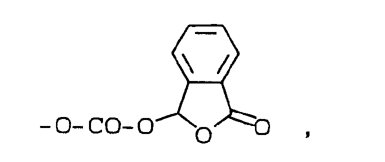
      or -O-CO-CH₂O-(CH₂CH₂O)ₓC₁-C₄-alkyl or -O-CO-O(CH₂CH₂O)ₓ-C₁-C₄-alkyl (where x is as defined above); and
   D is absent, -CH₂0-, or -CH₂S-.

Heterocyclic substituents in which nitrogen is the heteroatom are preferred, and of these, those containing a single nitrogen atom are preferred. Fully saturated heterocyclic substituents are also preferred. Thus, piperidine is a preferred heterocyclic substituent. Other preferred heterocyclic substituents are: pyrryl, pyrrolinyl, quinuclidinyl, isoquinuclidinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolidinyl, isoxazolyl, isoxazolidinyl, morpholinyl, thiazolyl, thiazolidinyl isothiazolyl, isothiazolidinyl, indolyl, quinolinyl, isoquinolinyl, benzimidazolyl, benzothiazolyl, benzoxazolyl, furyl, thienyl and benzothienyl.

The term "halo" means fluoro, chloro, bromo and iodo.

Among the substituents for A, B, R¹, R¹¹, R¹⁵, R¹⁶, R²³, Z, and r preferred groups are recognized as follows. Preferred A¹ are -H or -CH₃.

Preferred A are:
Boc- ,
Et OC-,
i-PrSO₂- ,
CH₃(OCH₂CH₂)₃OCO-,

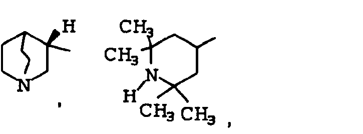

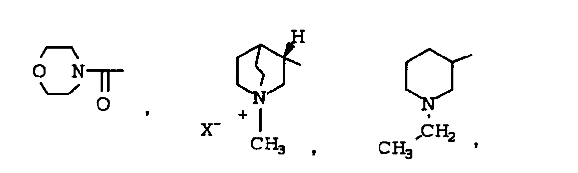

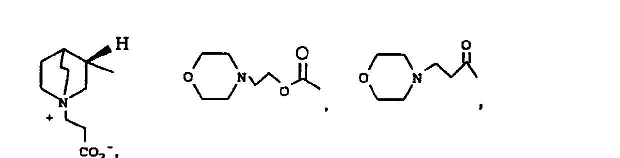

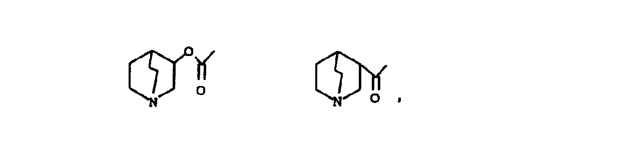

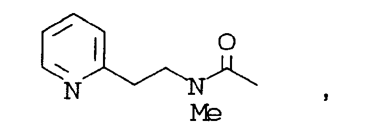

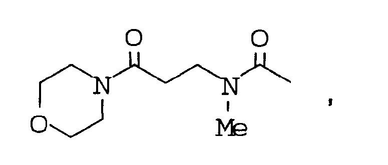

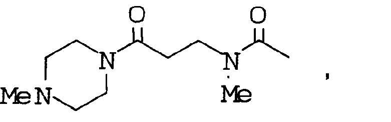

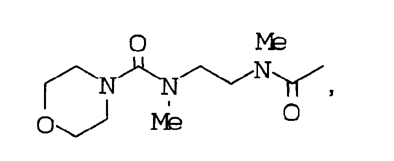

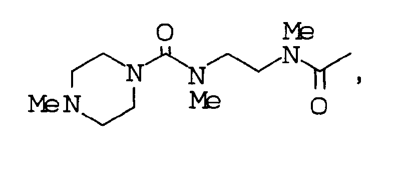

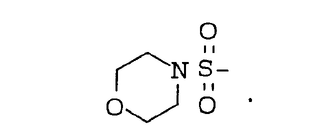

Preferred B are:

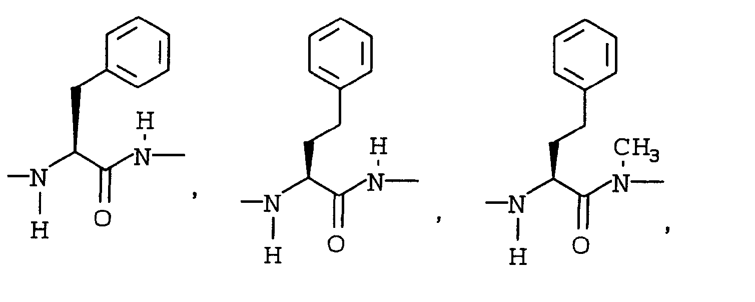

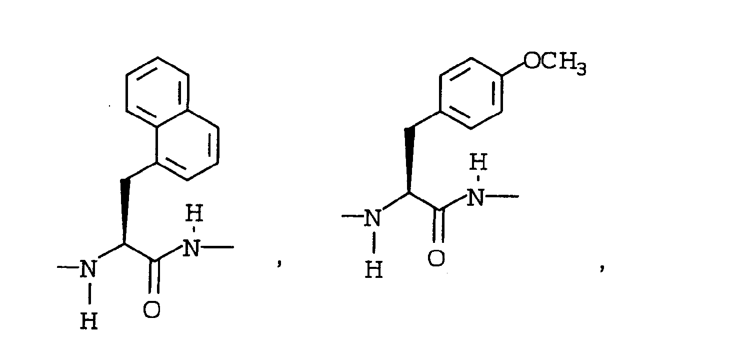

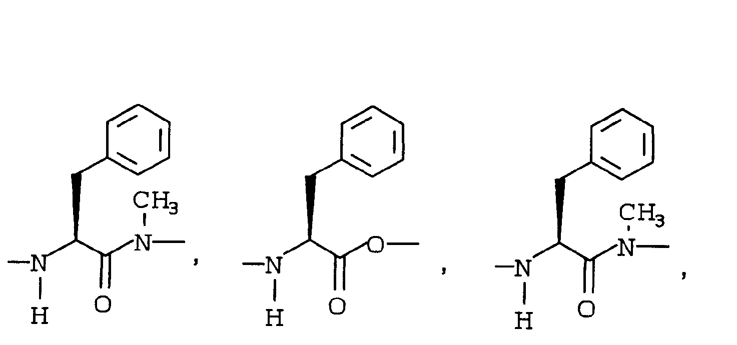

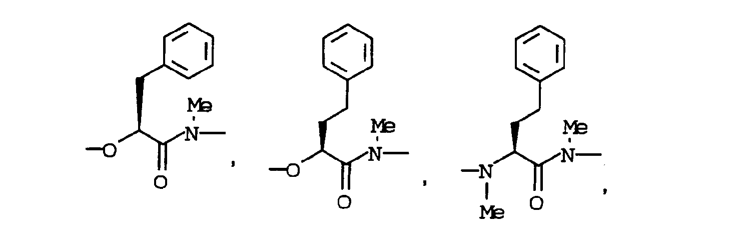

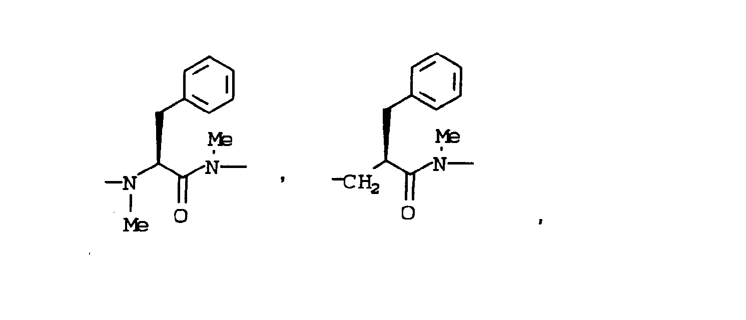

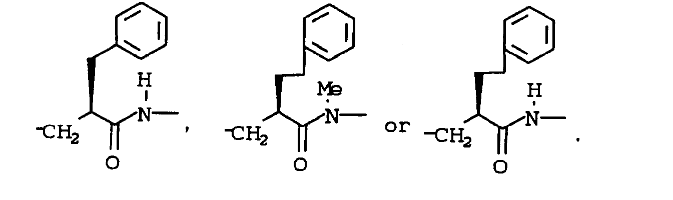

Preferred A and B taken together are: 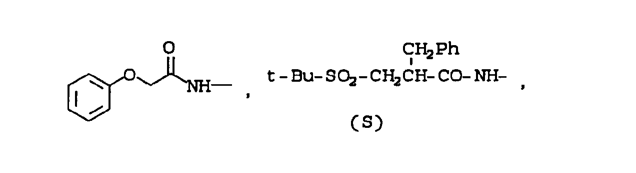 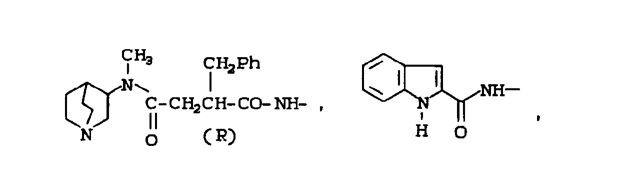 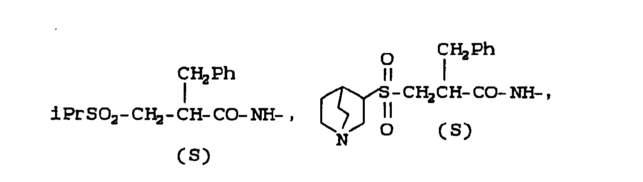 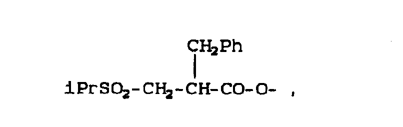 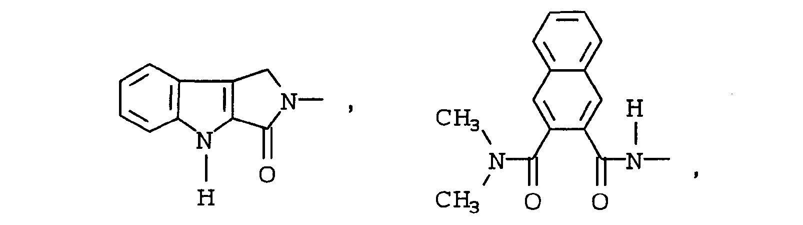 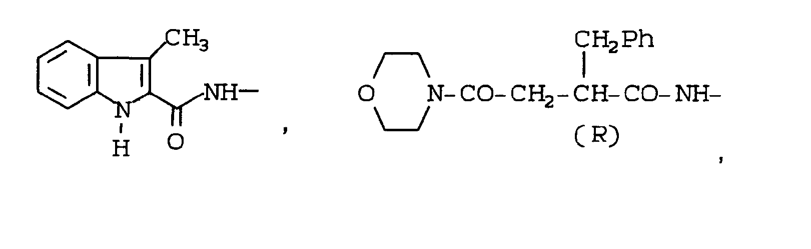 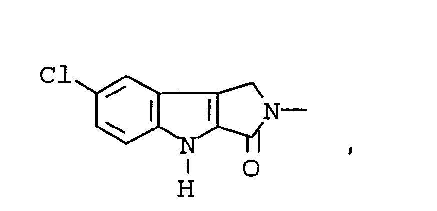 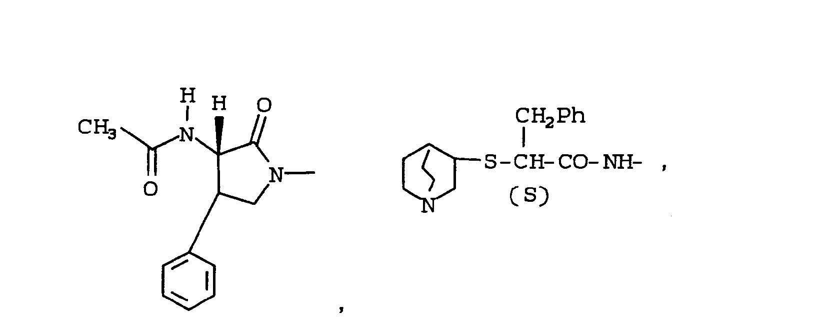 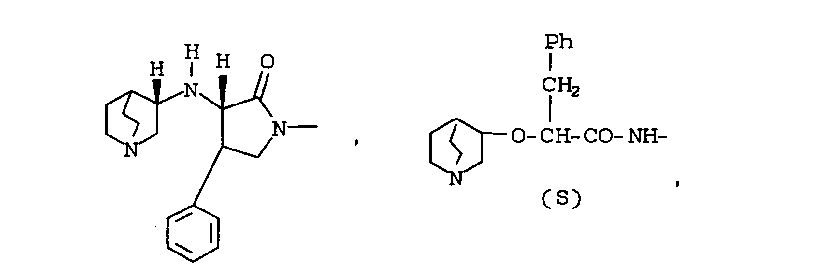 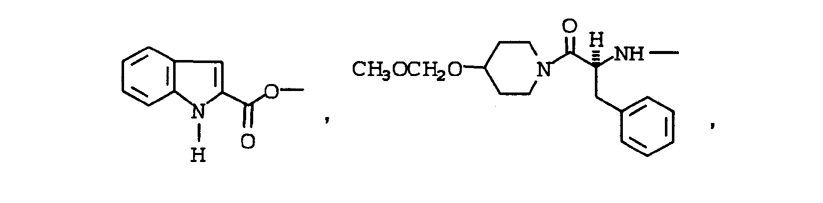 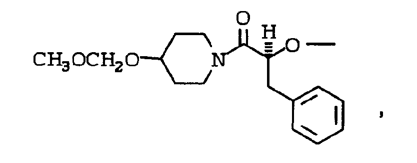 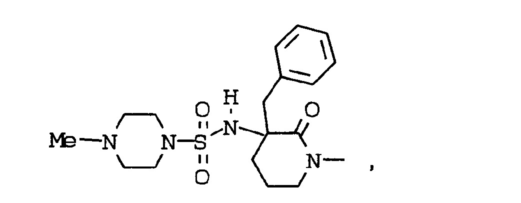 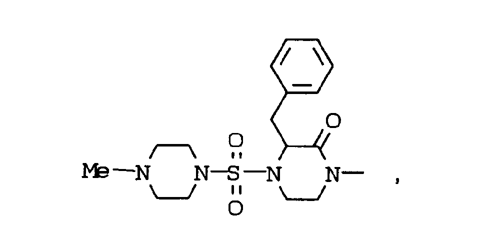 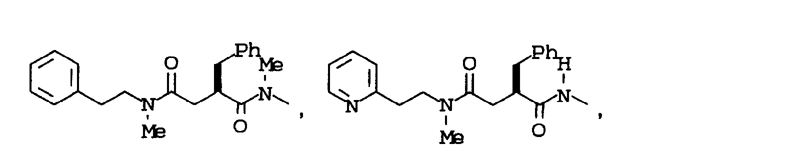 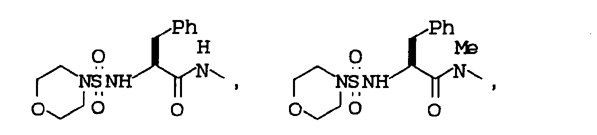 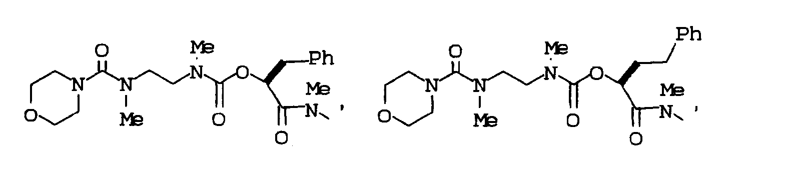 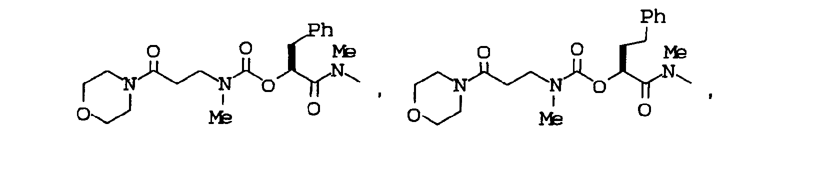 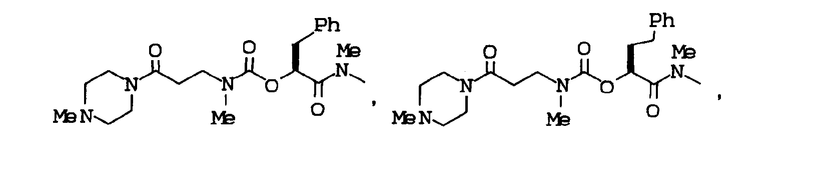 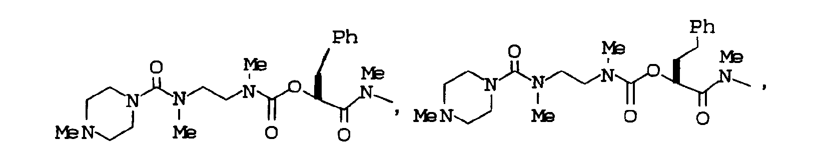 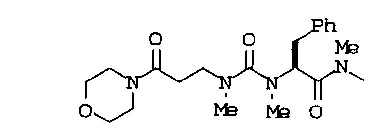
, or

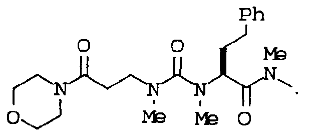

Preferred R¹⁵ are -H, -CH₃, i-Pr and n-Pr. Preferred R¹⁶ are -H, n-butyl, isobutyl, isopropyl, 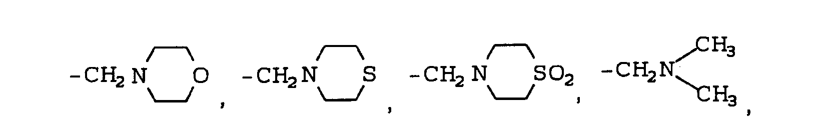 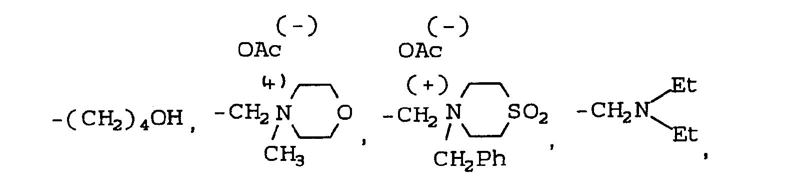
and

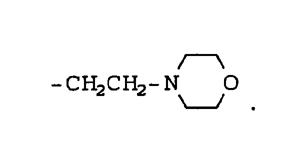

Preferred R²³ are hydrogen or methyl, a preferred r is 1 and preferred Z are:
-OH, -OCOCH₂CH₂CO₂H,
-OCOCH₂N(C₁-C₄-alkyl)₂,
-OCO-CH₂NH₂,
-OCOCH₂CH₂NH₂,
-OCO(C₁-C₄-alkyl),
-NH₂,
-OCOCH(n-Bu)NH₂,
-OCOCH(i-Pr)NH₂,
-OCOO(CH₂CH₂0)₃CH₃,
-OP0₃H₂, and
-OCOCH₂CH₂P0₃H₂.

The preferred ring systems for the compounds of this invention include the following: 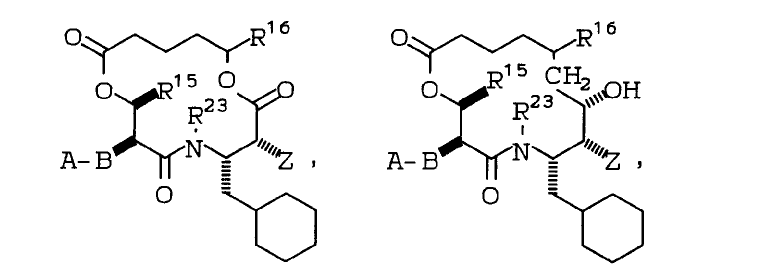 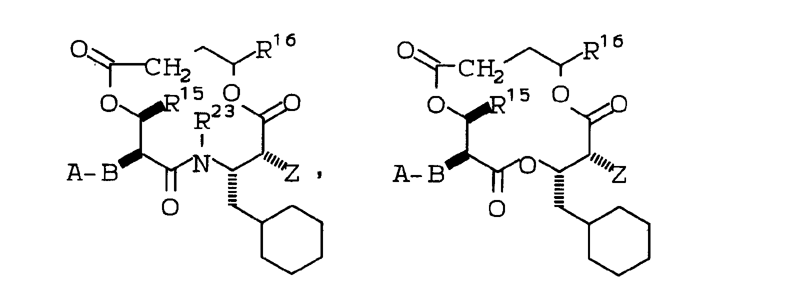
and 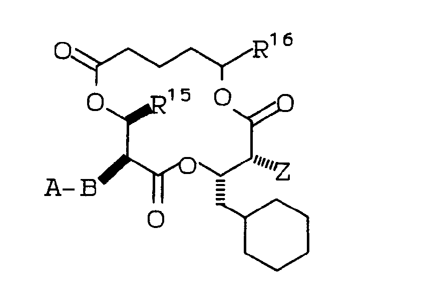
where A, B, R¹⁵, R¹⁶, R²³, Z and r are as defined above.

The preferred compounds of the present invention include those in Tables I-X:

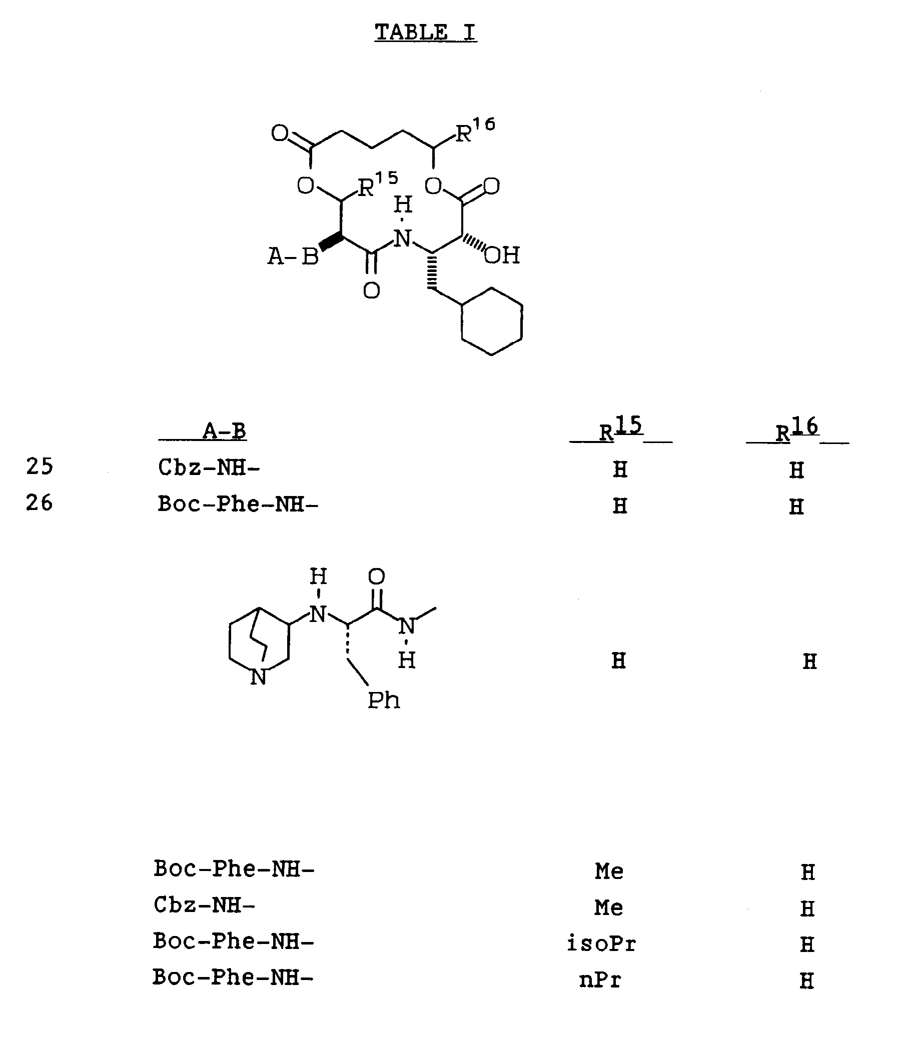

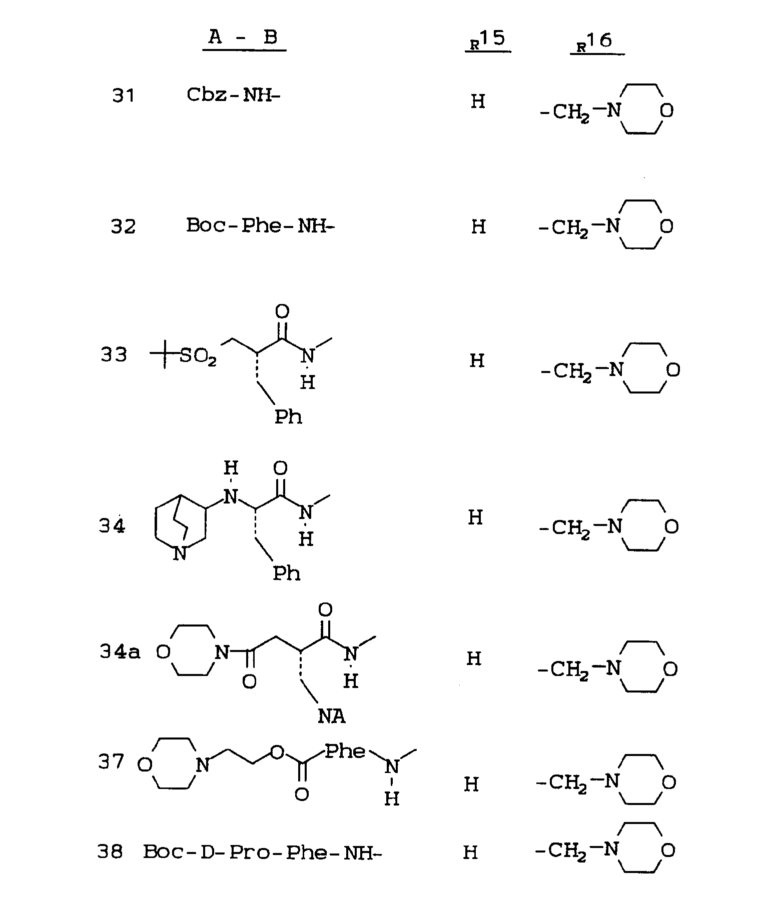

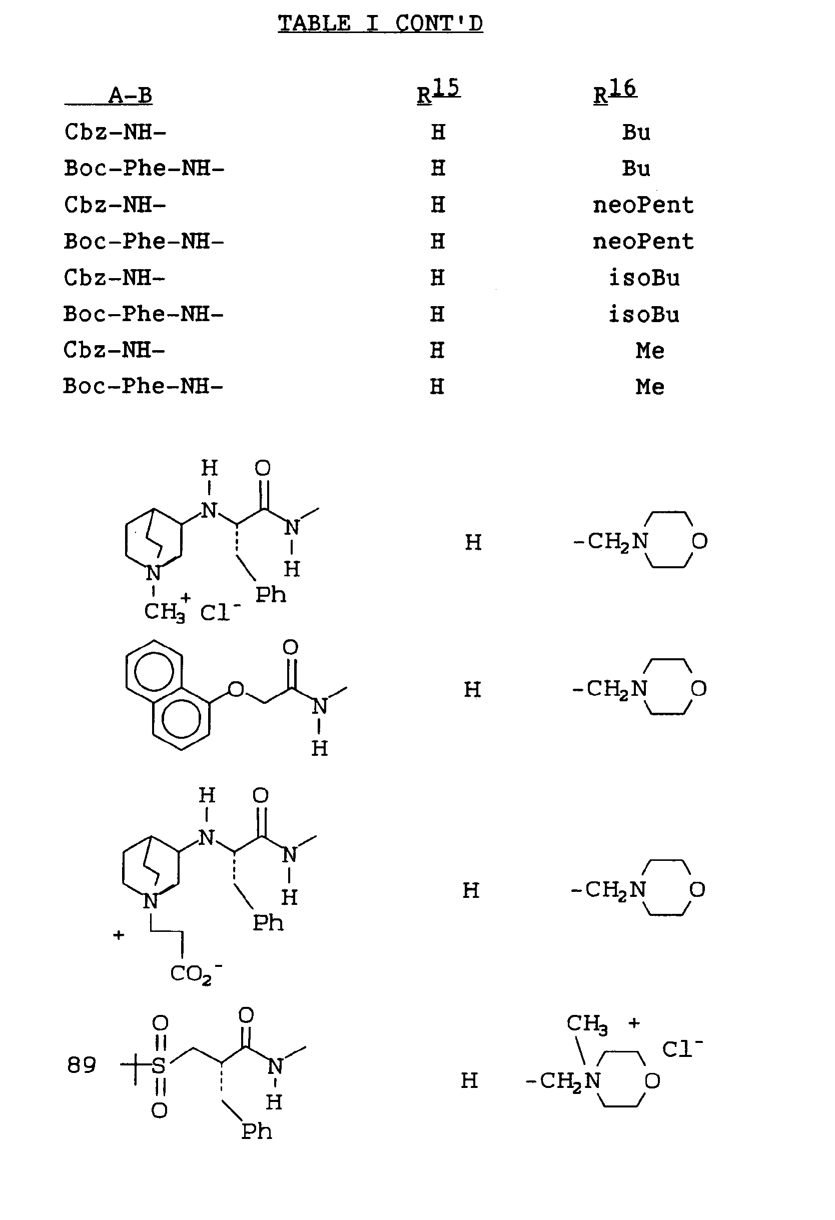

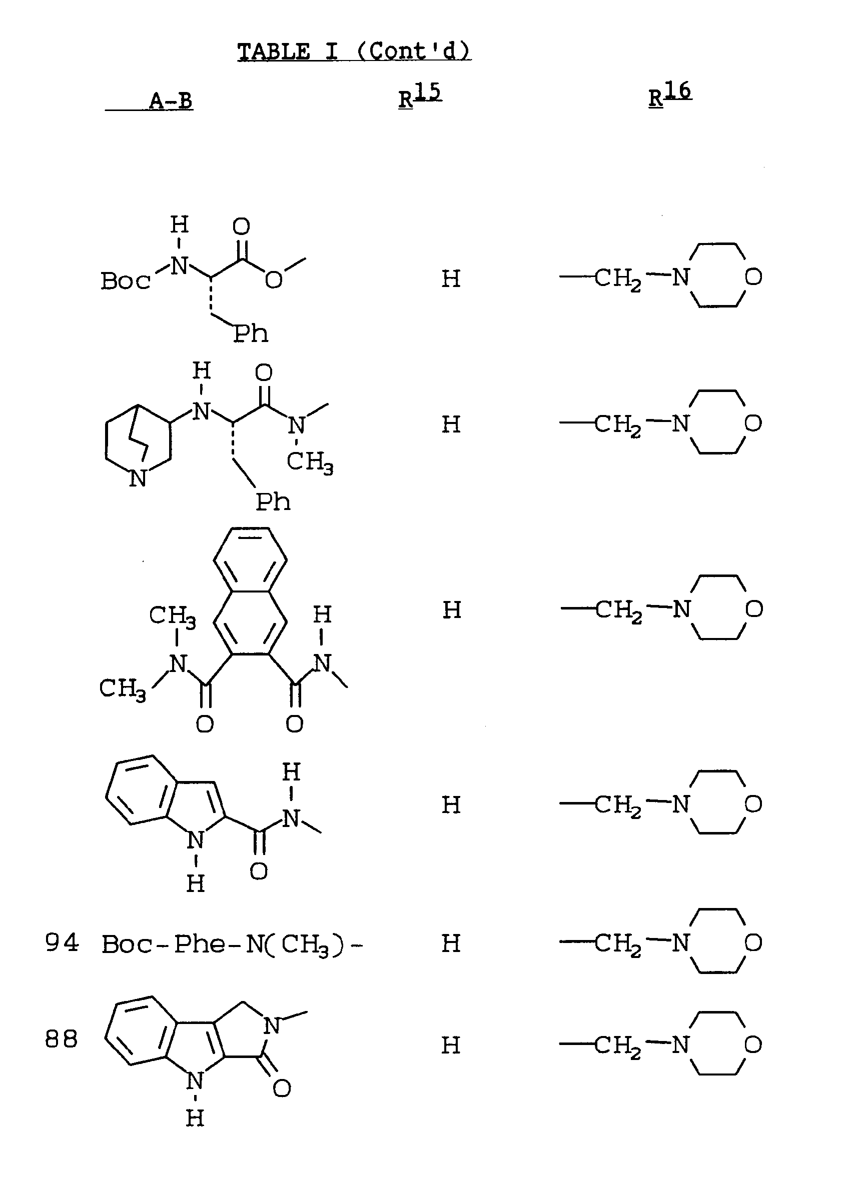

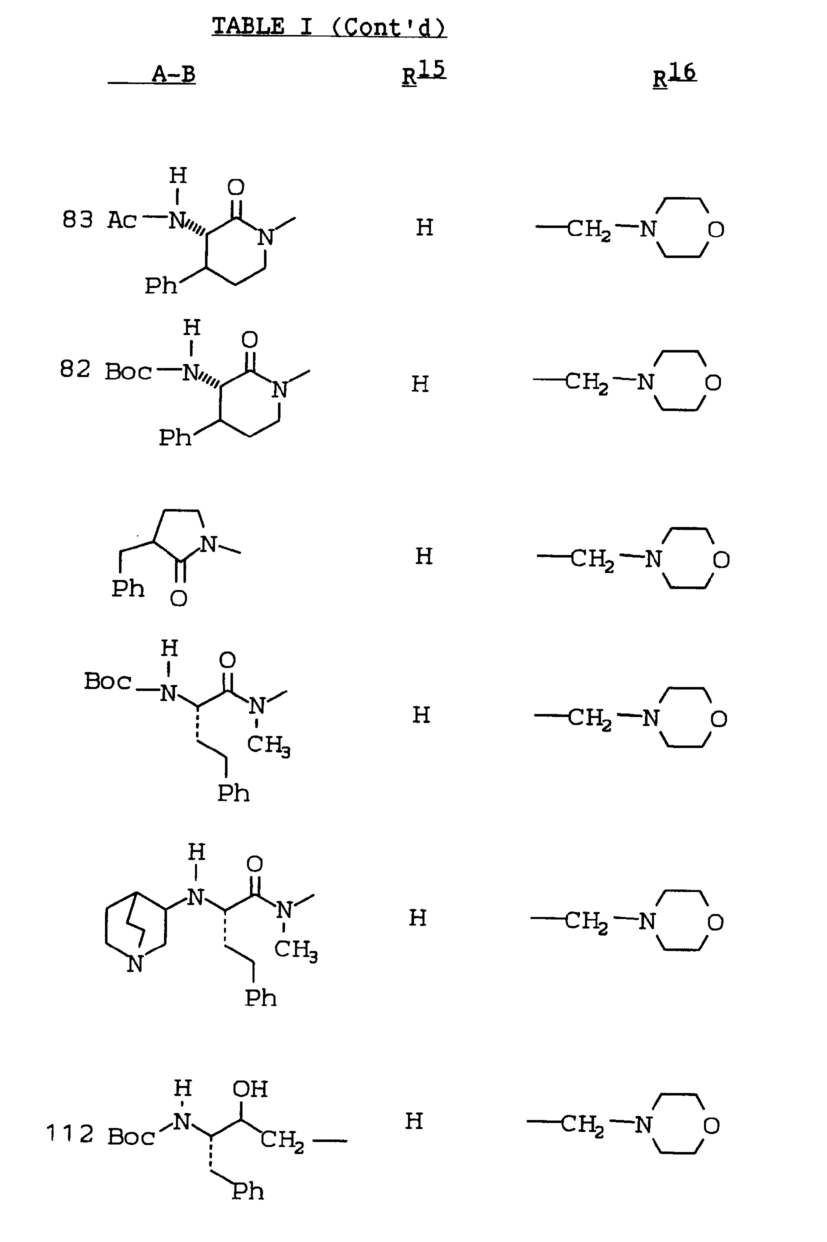

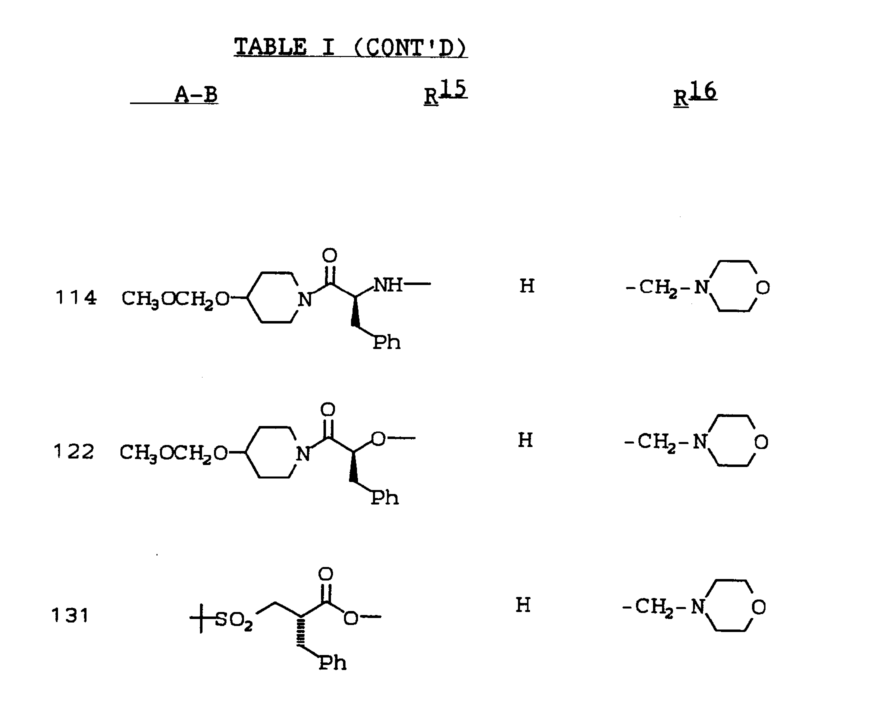

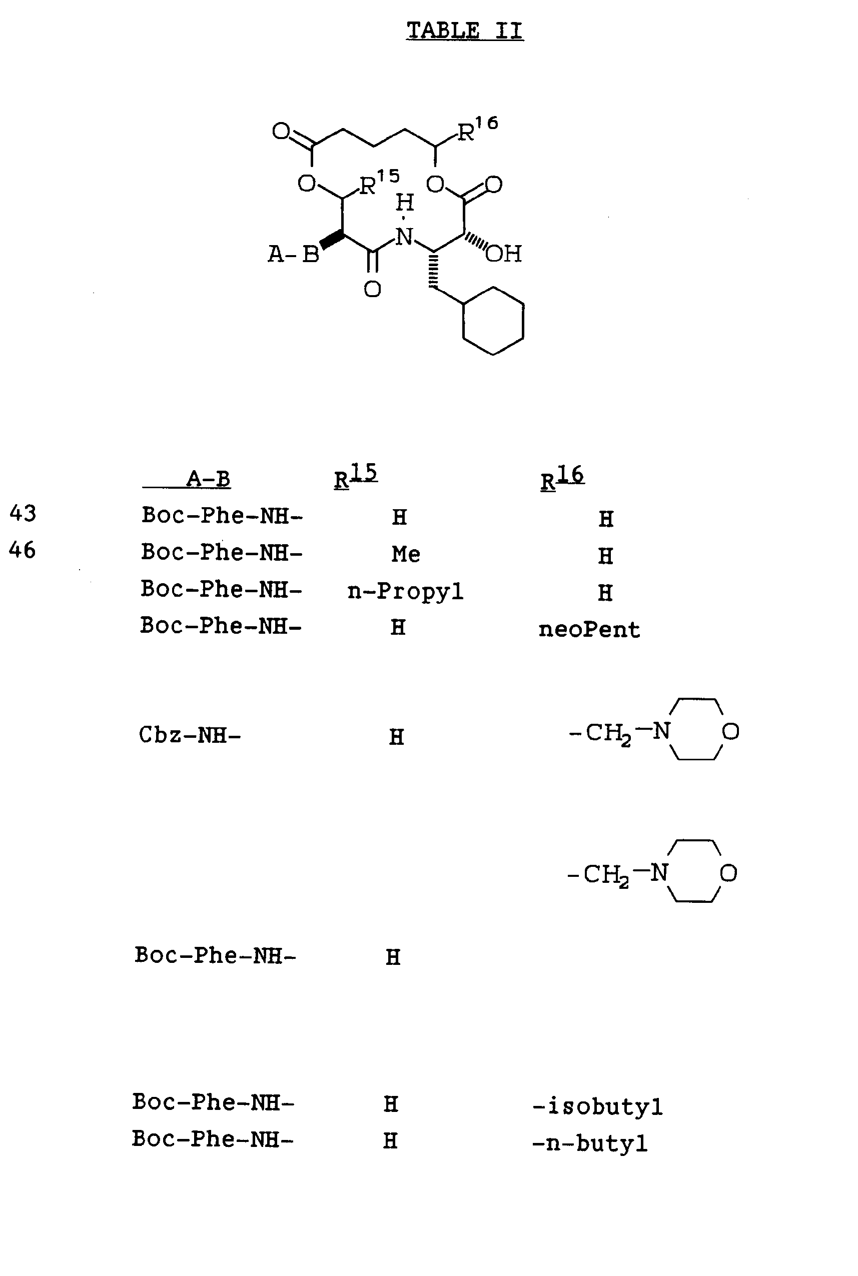

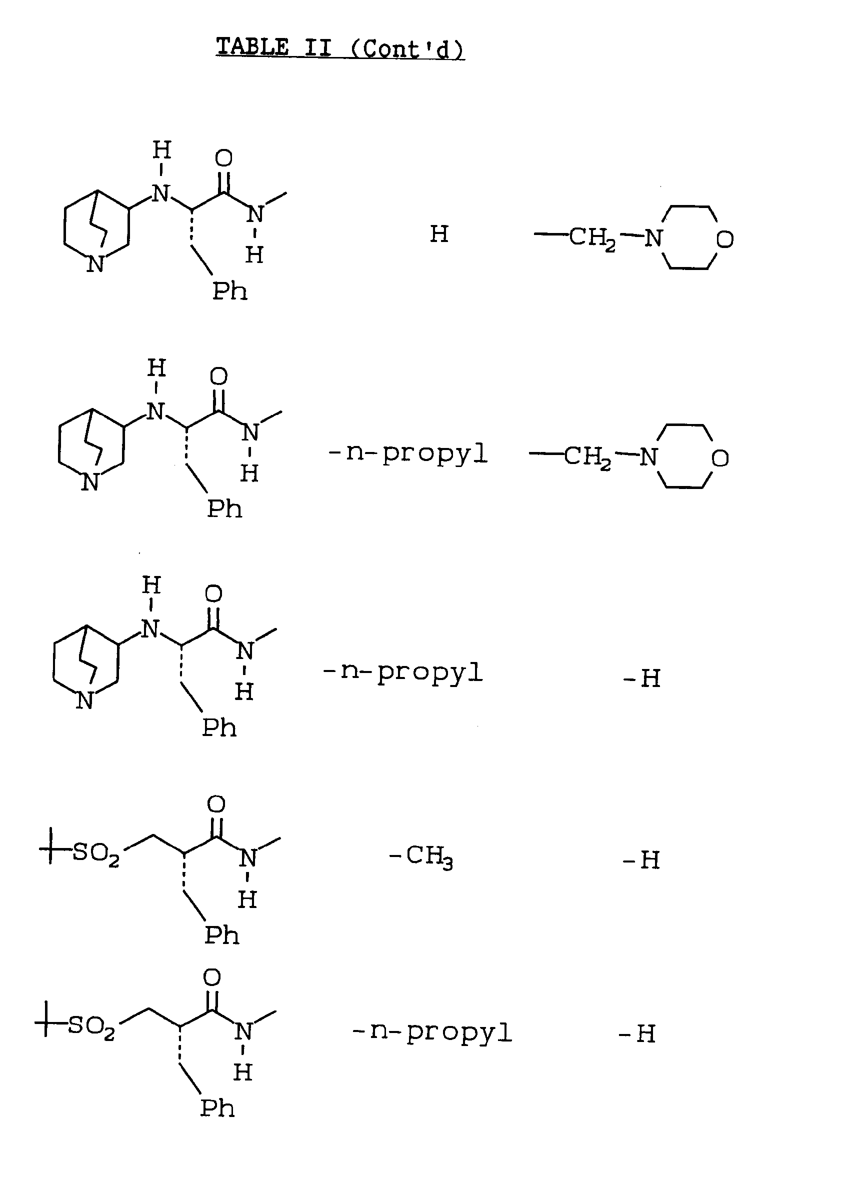

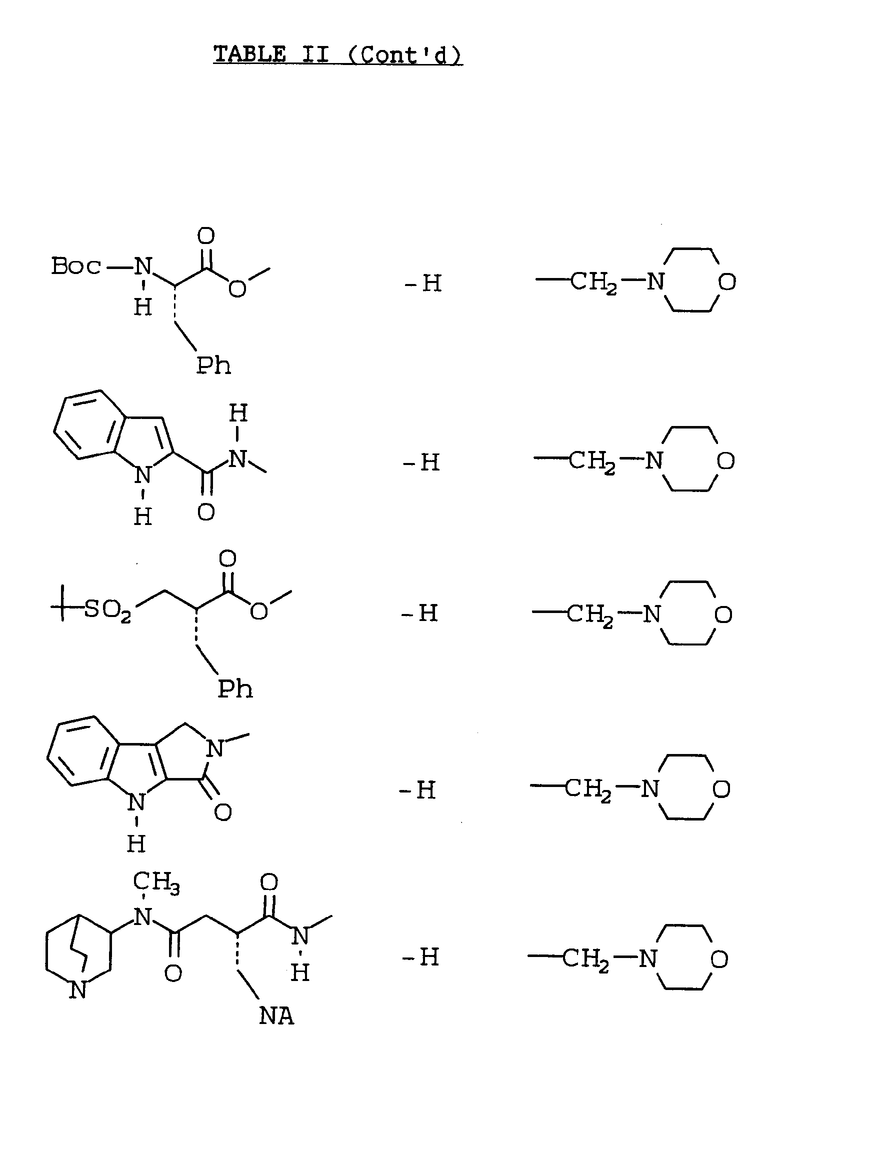

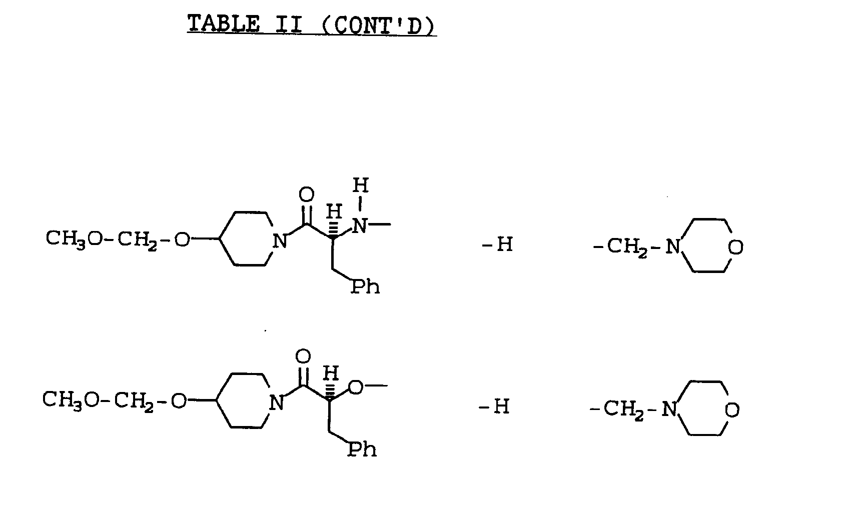

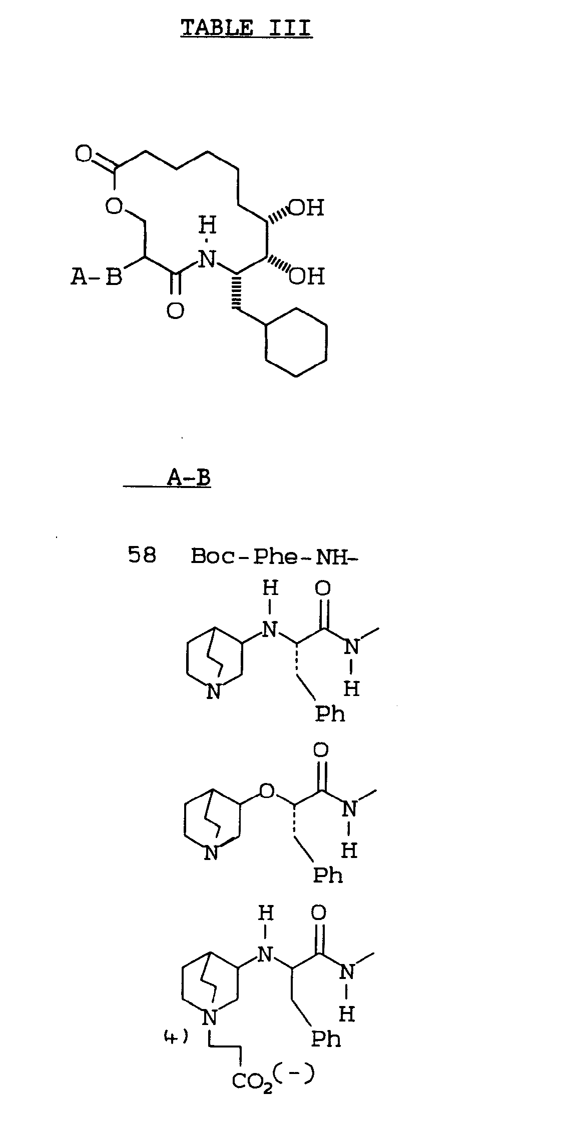

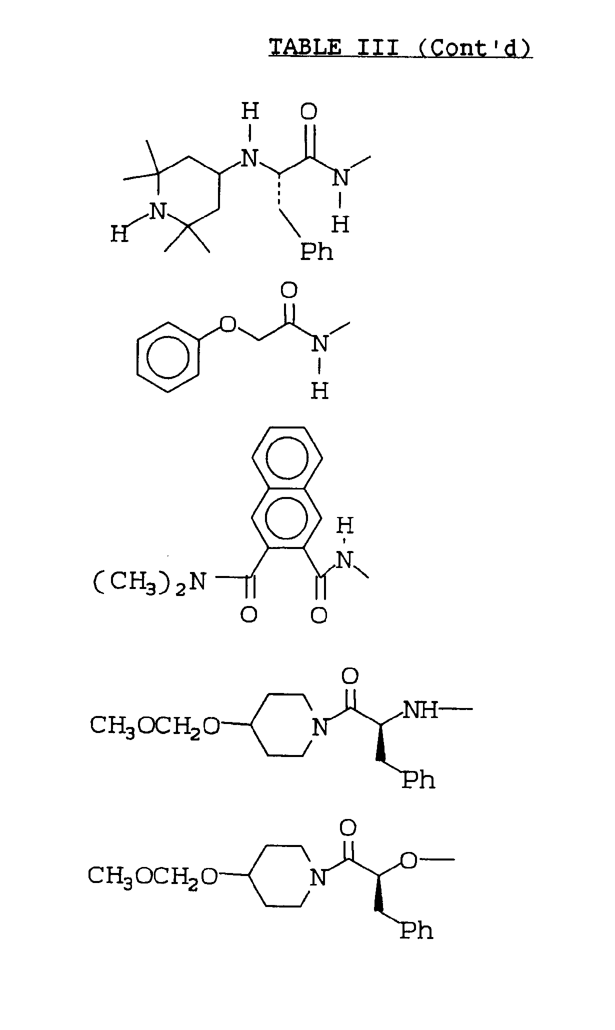

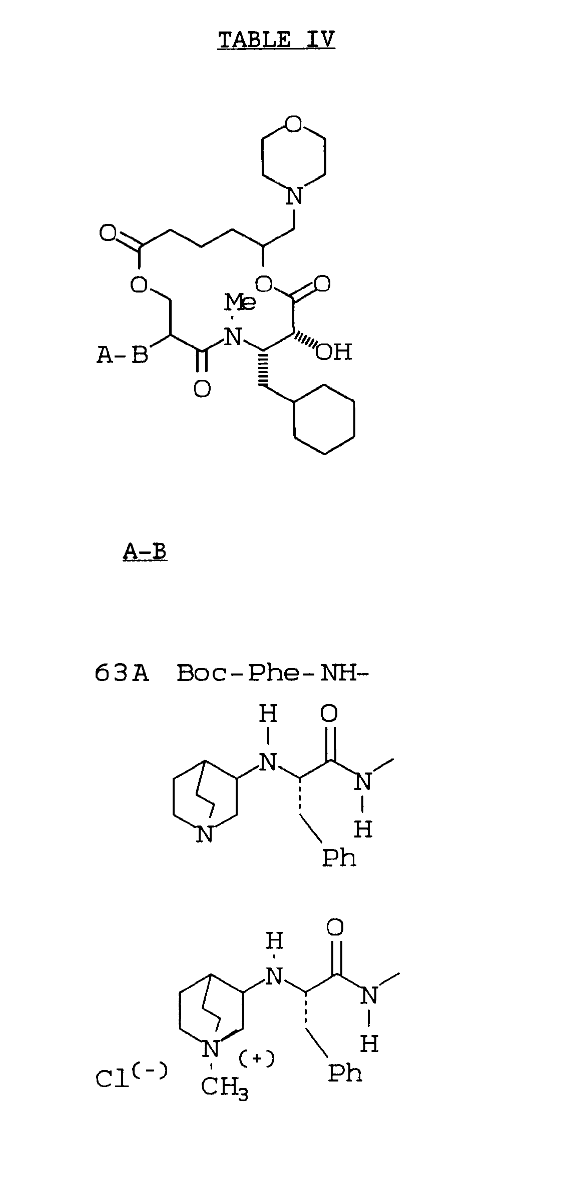

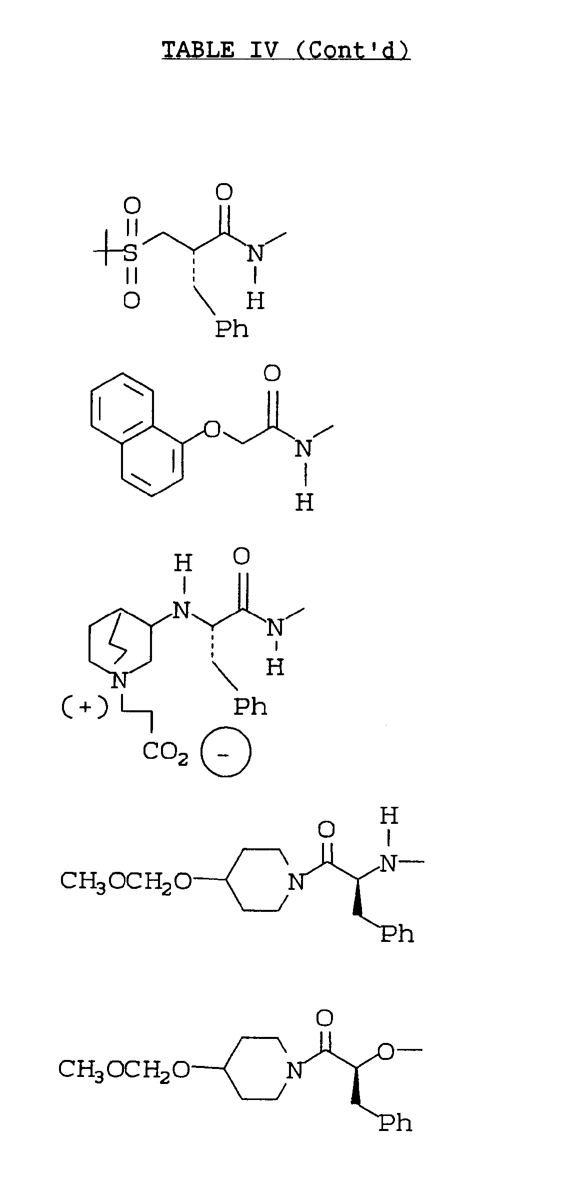

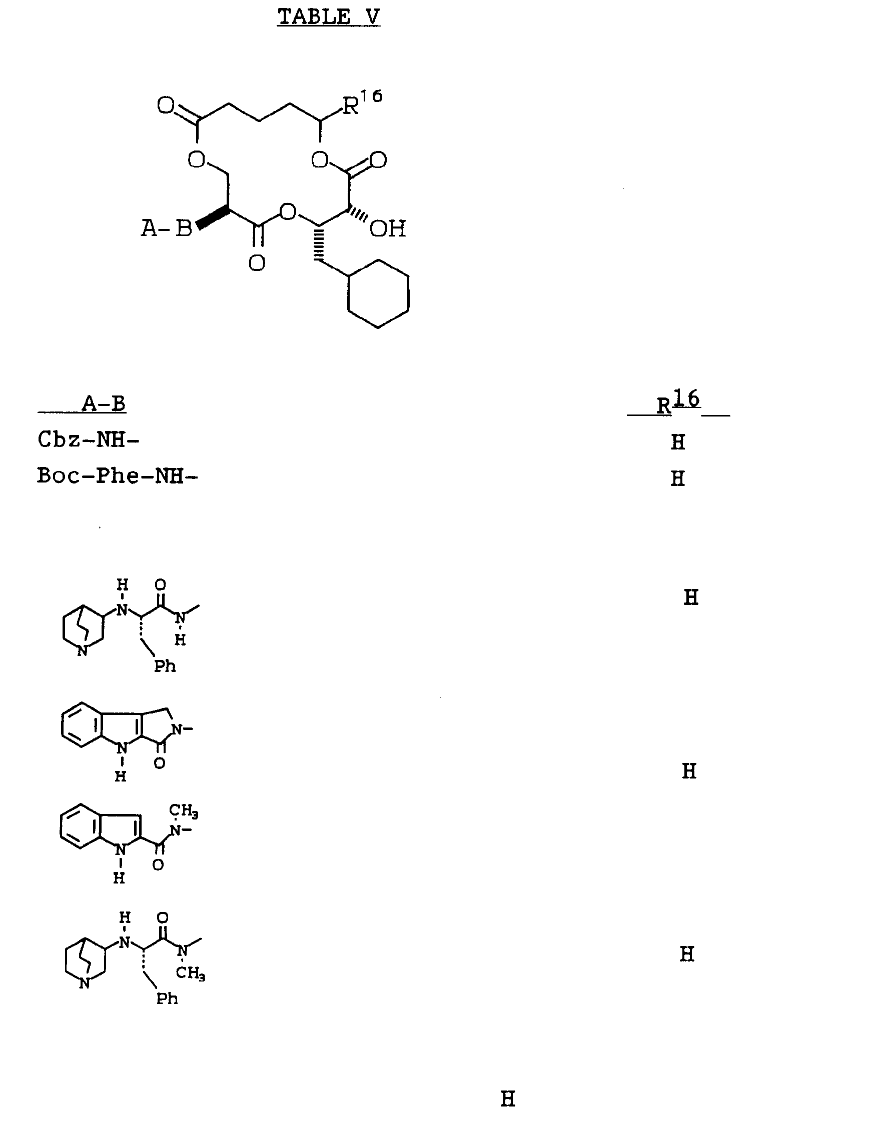

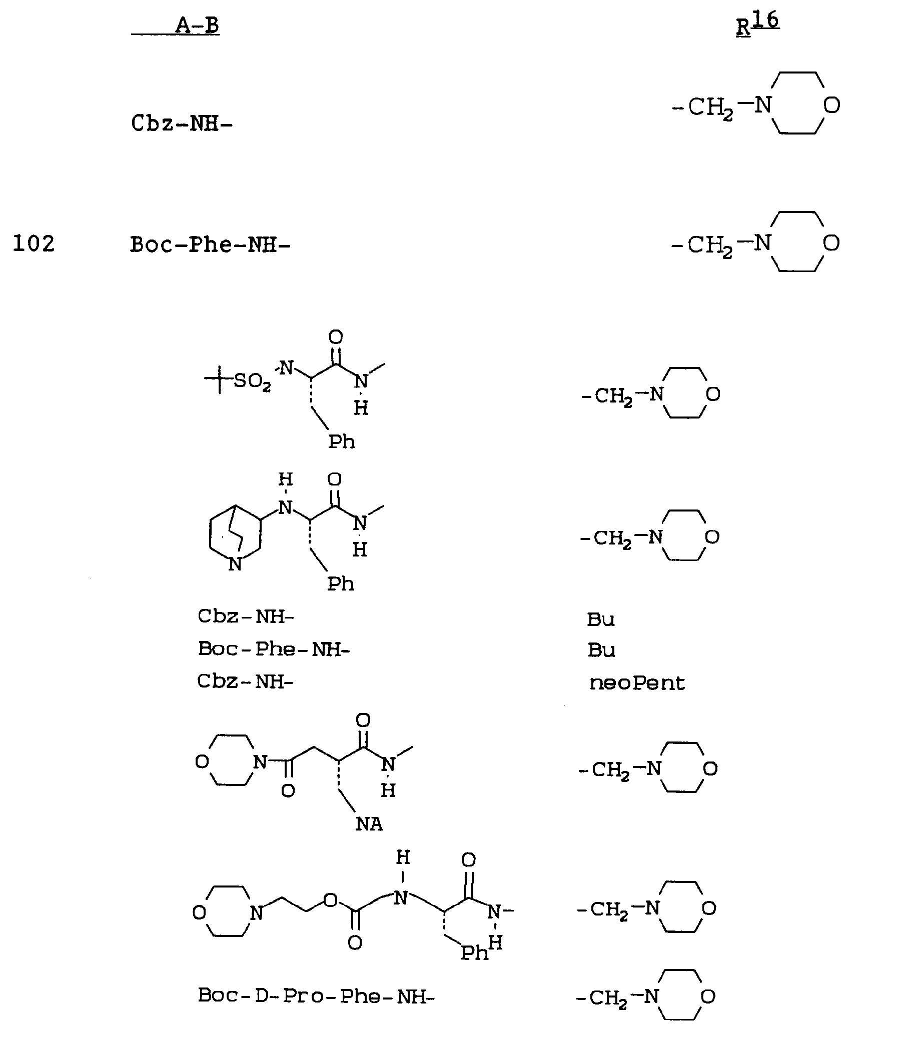

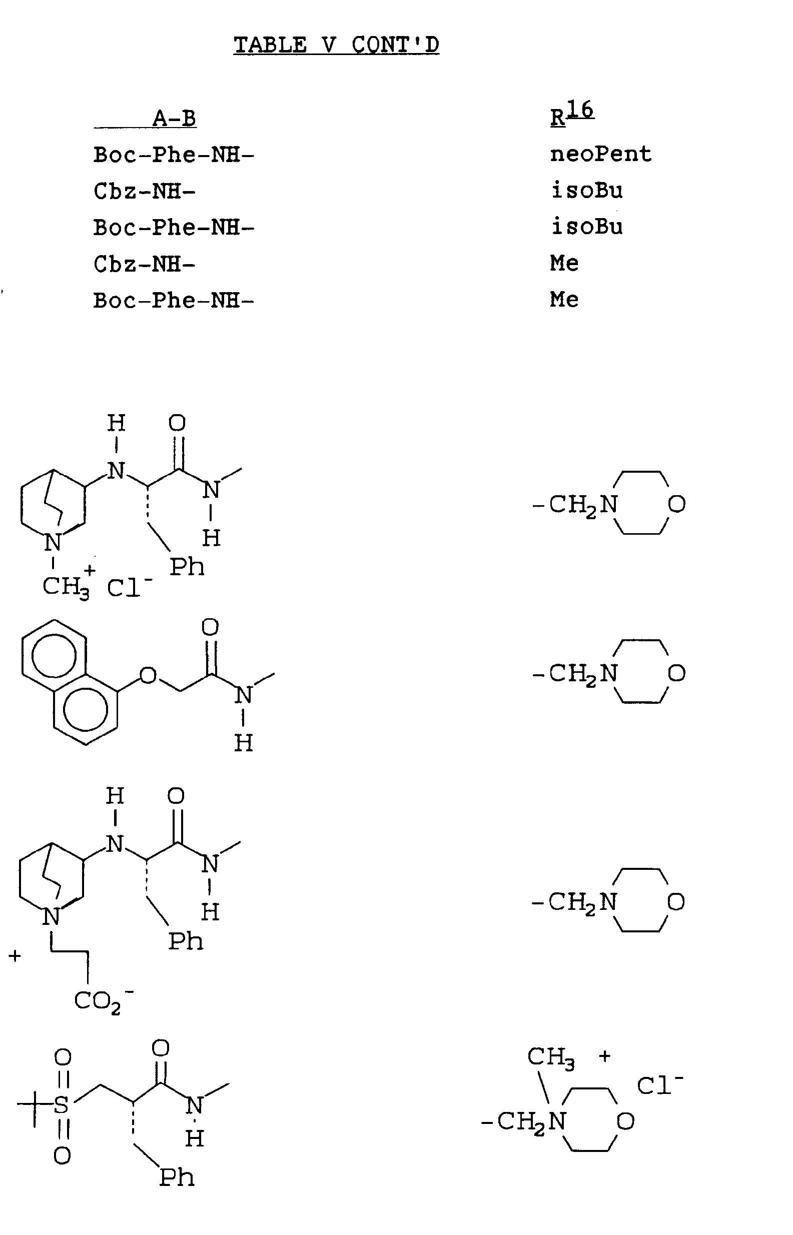

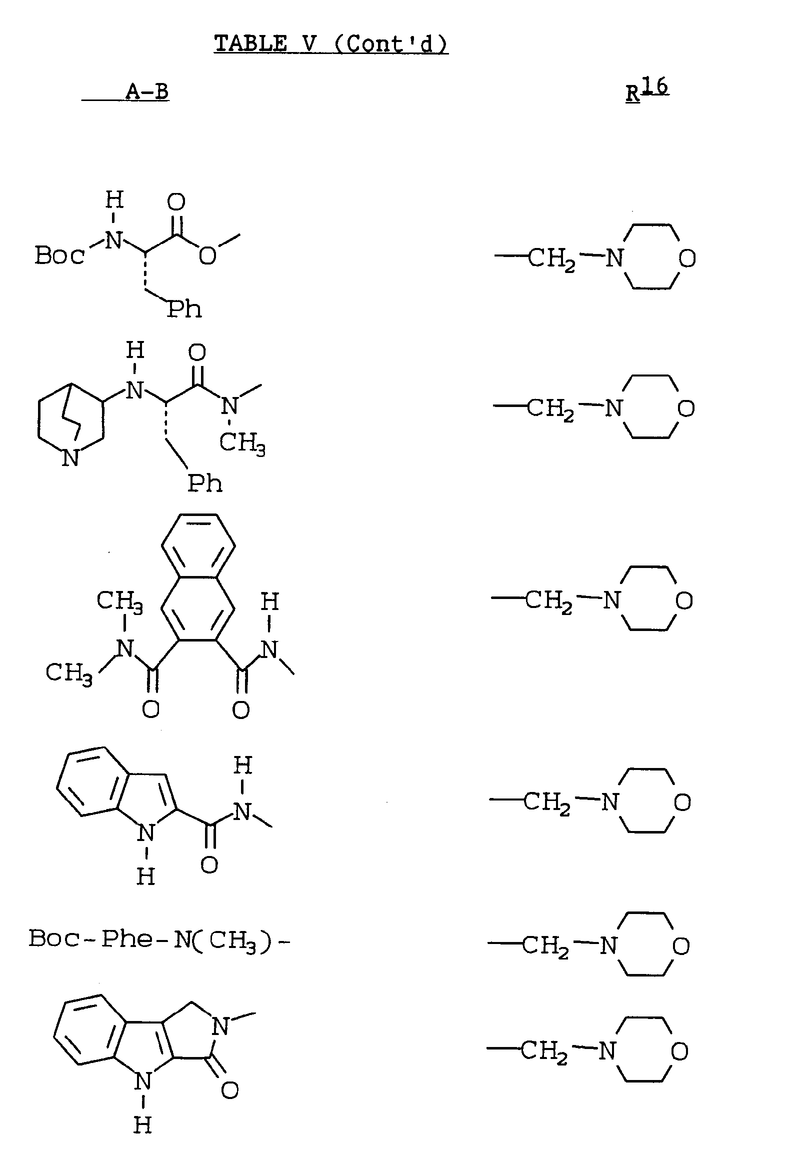

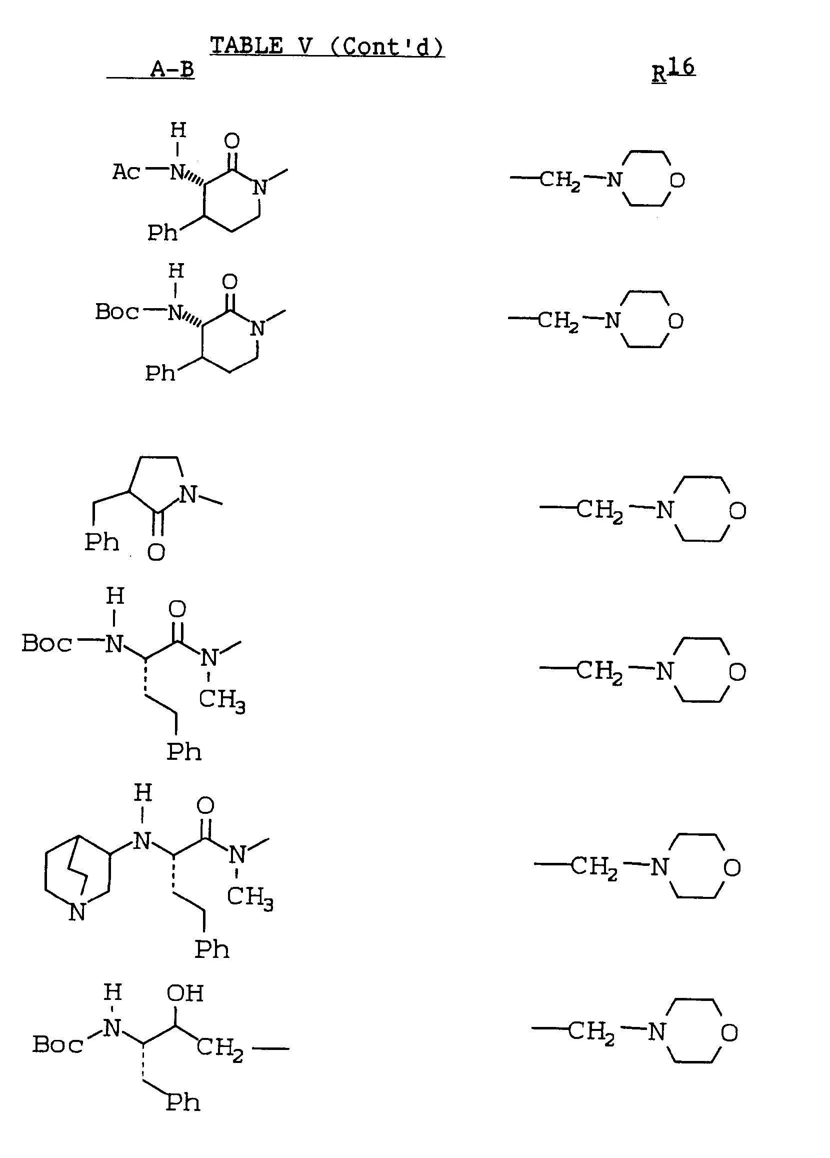

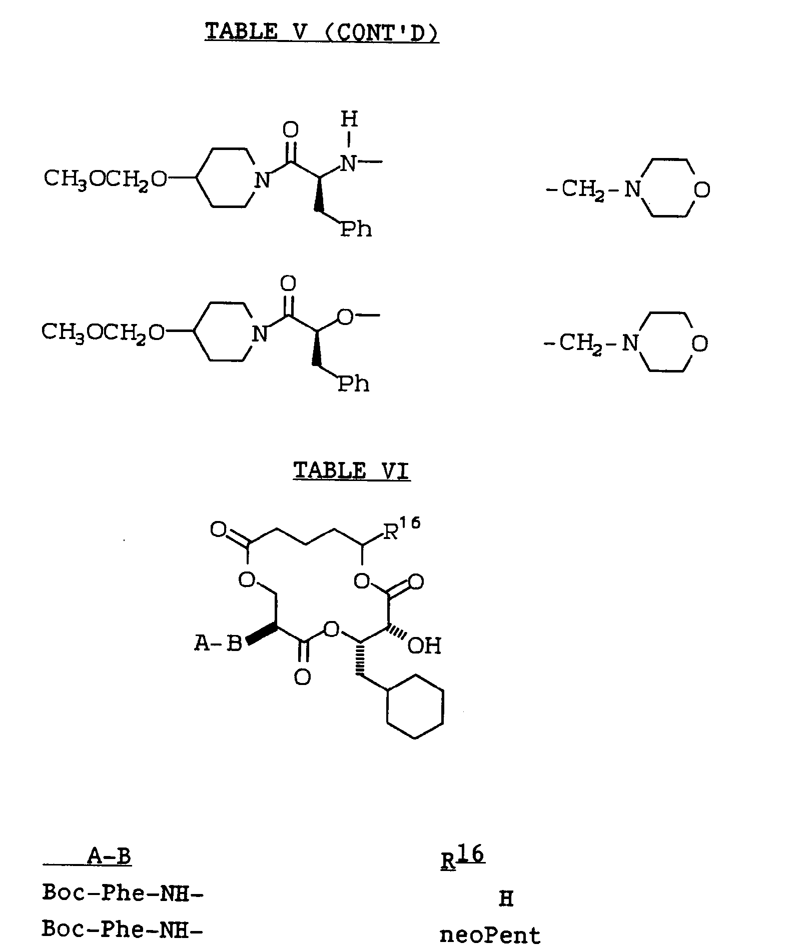

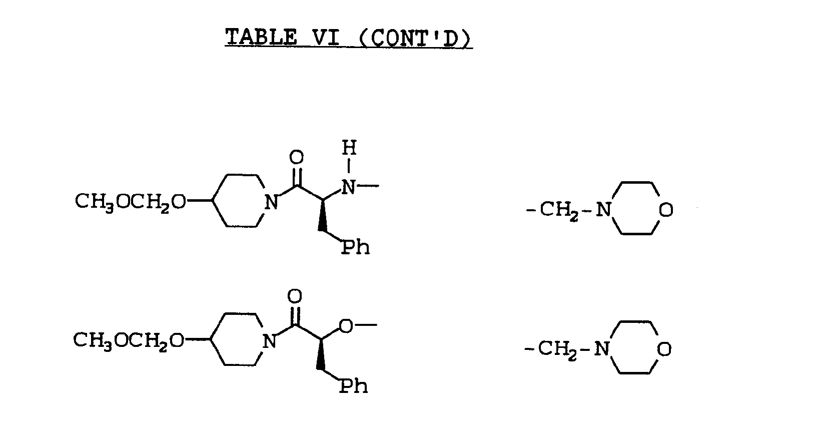

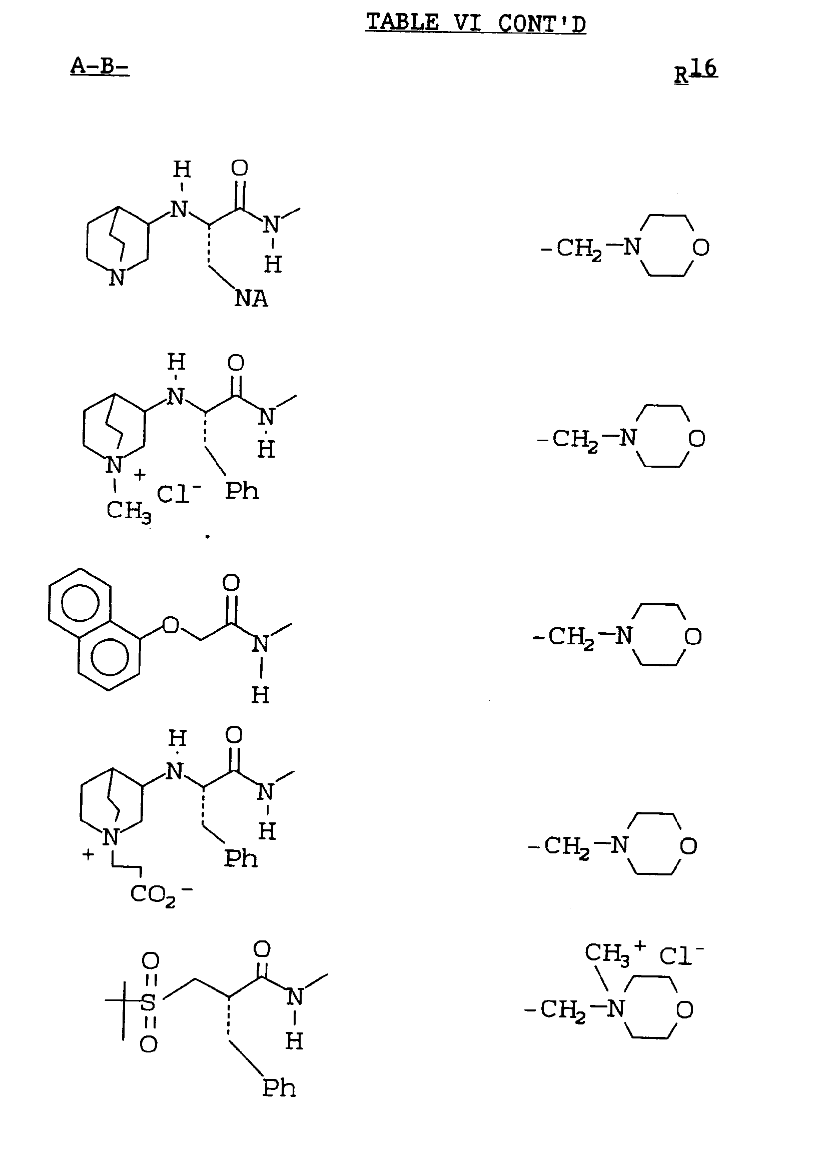

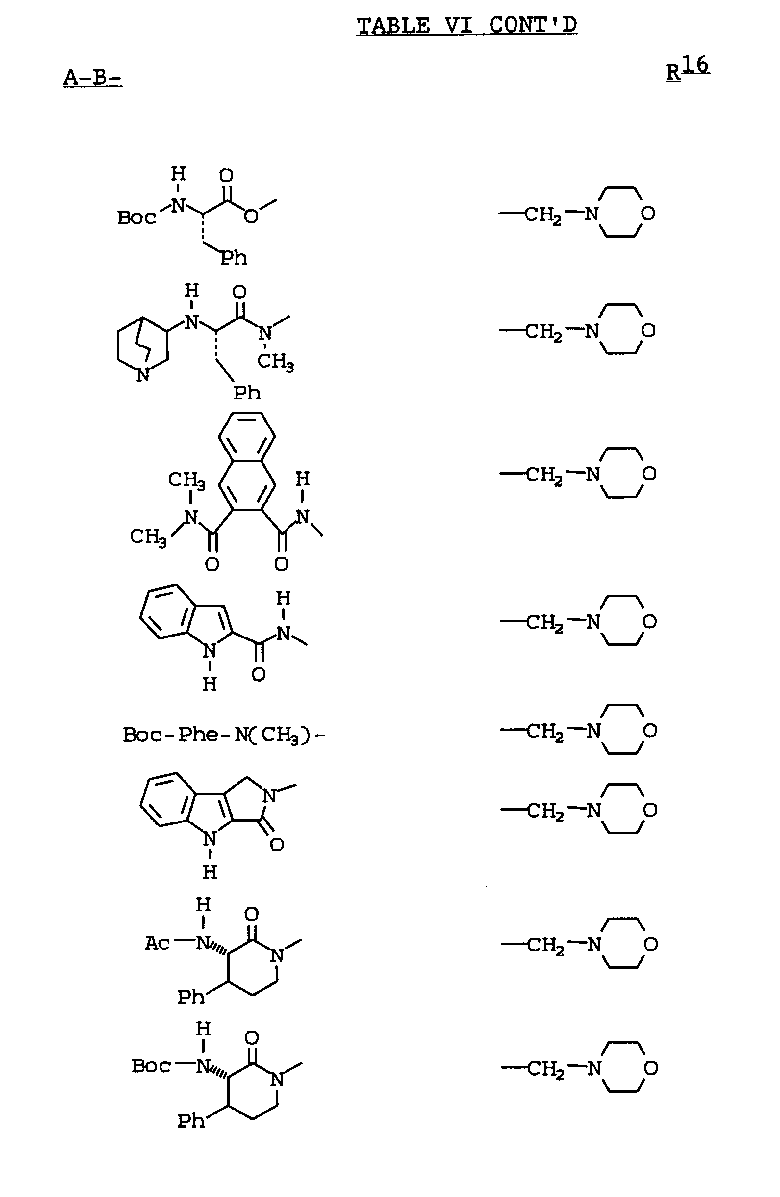

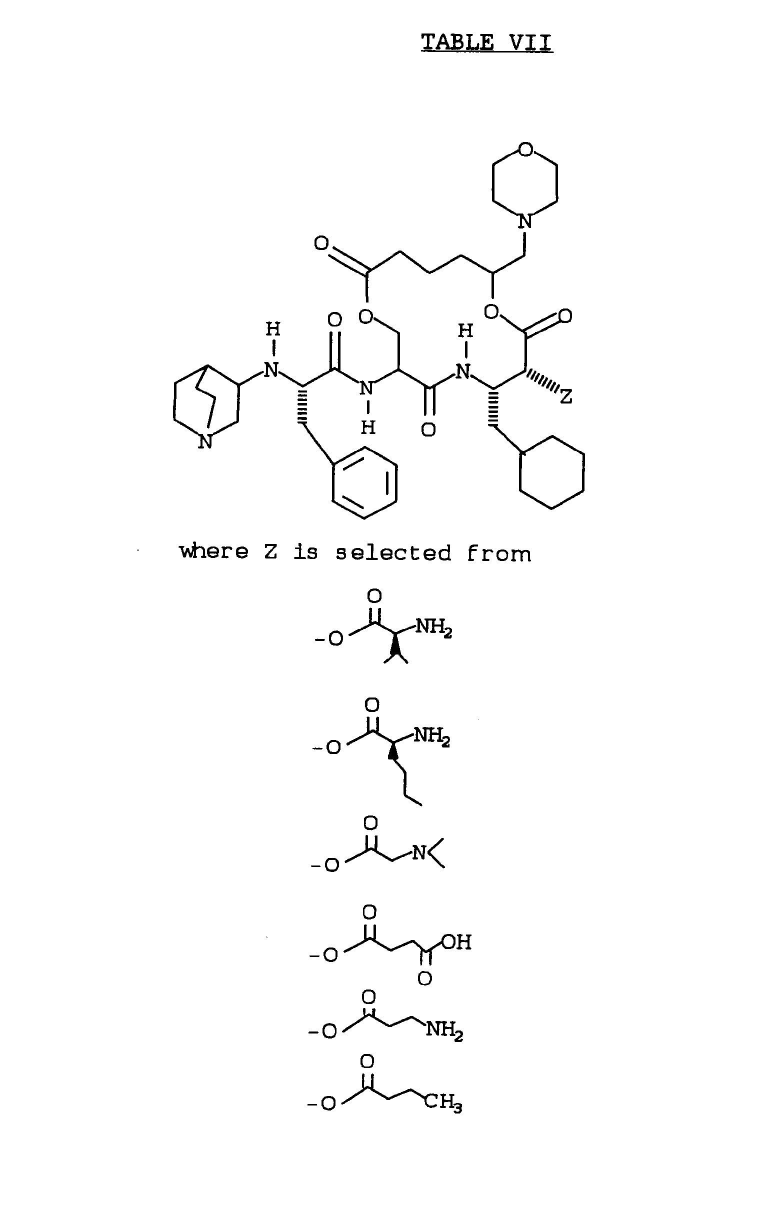

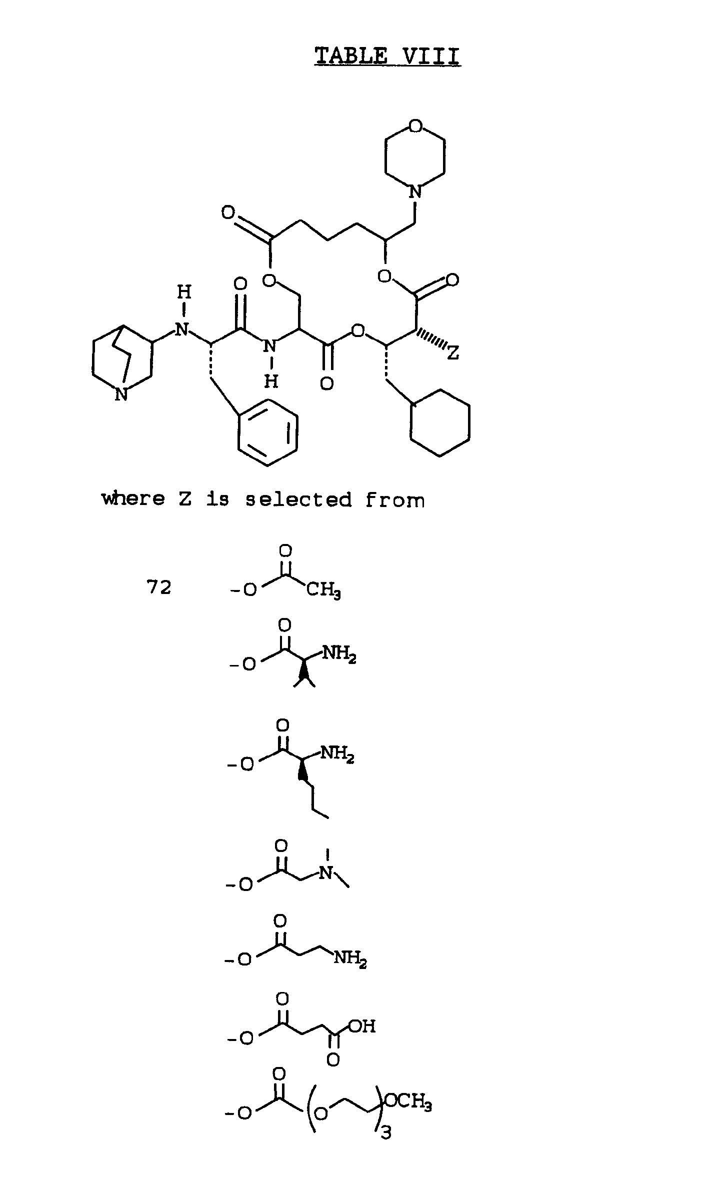

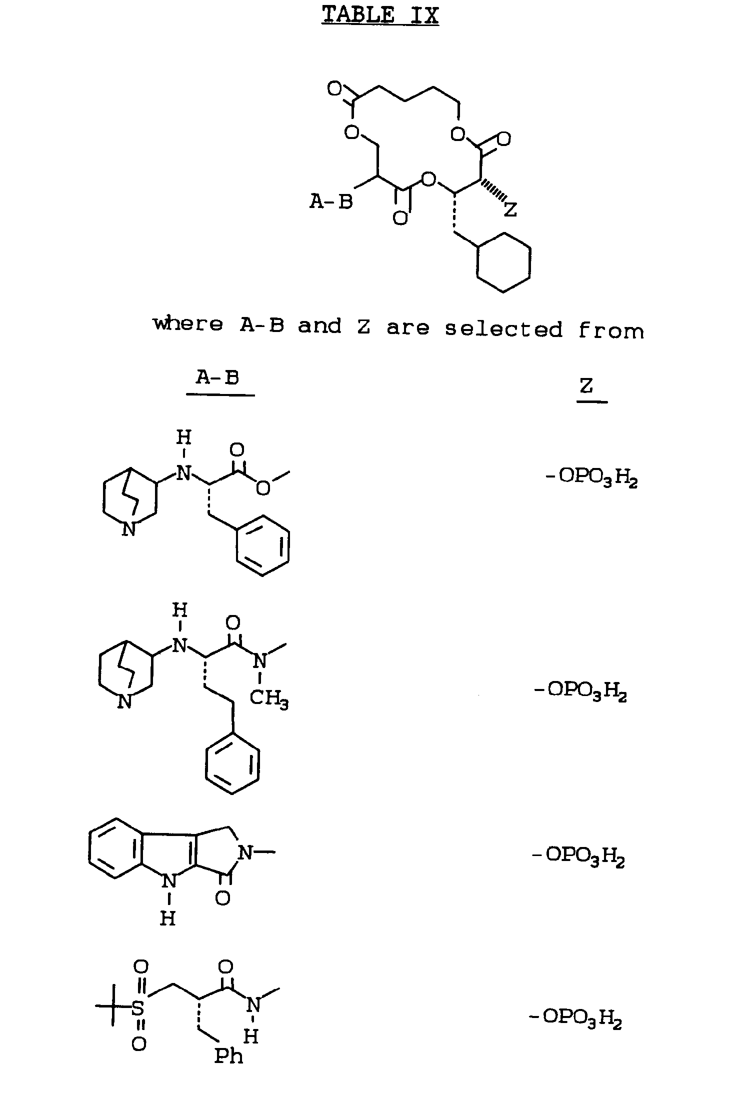

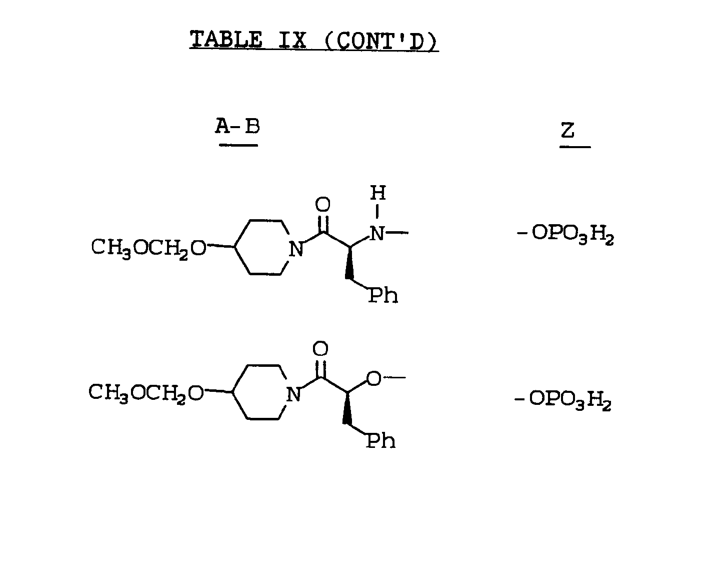

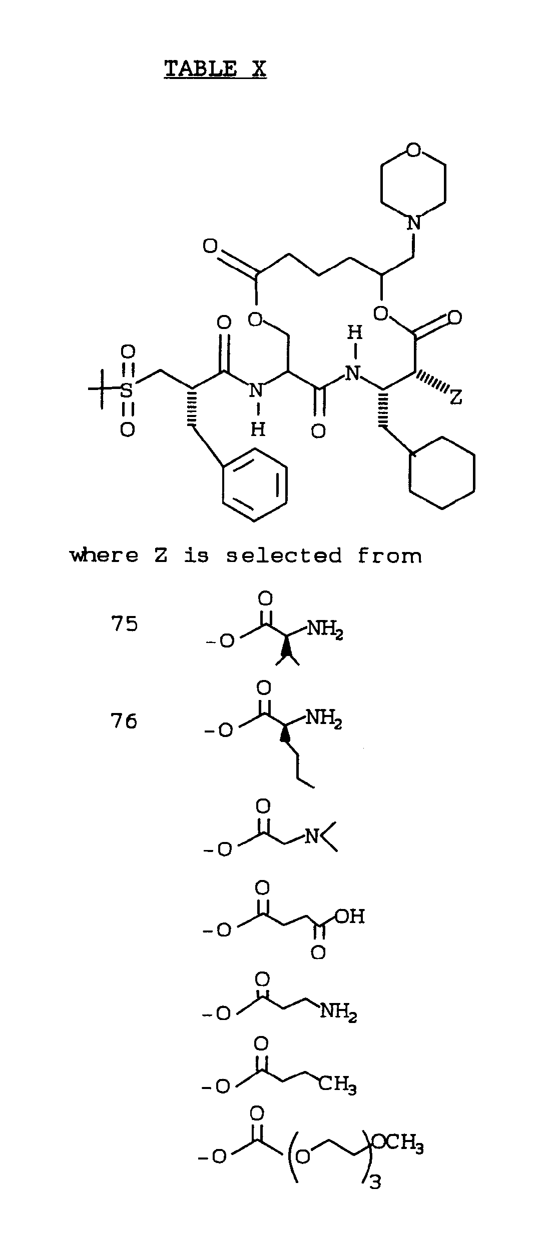

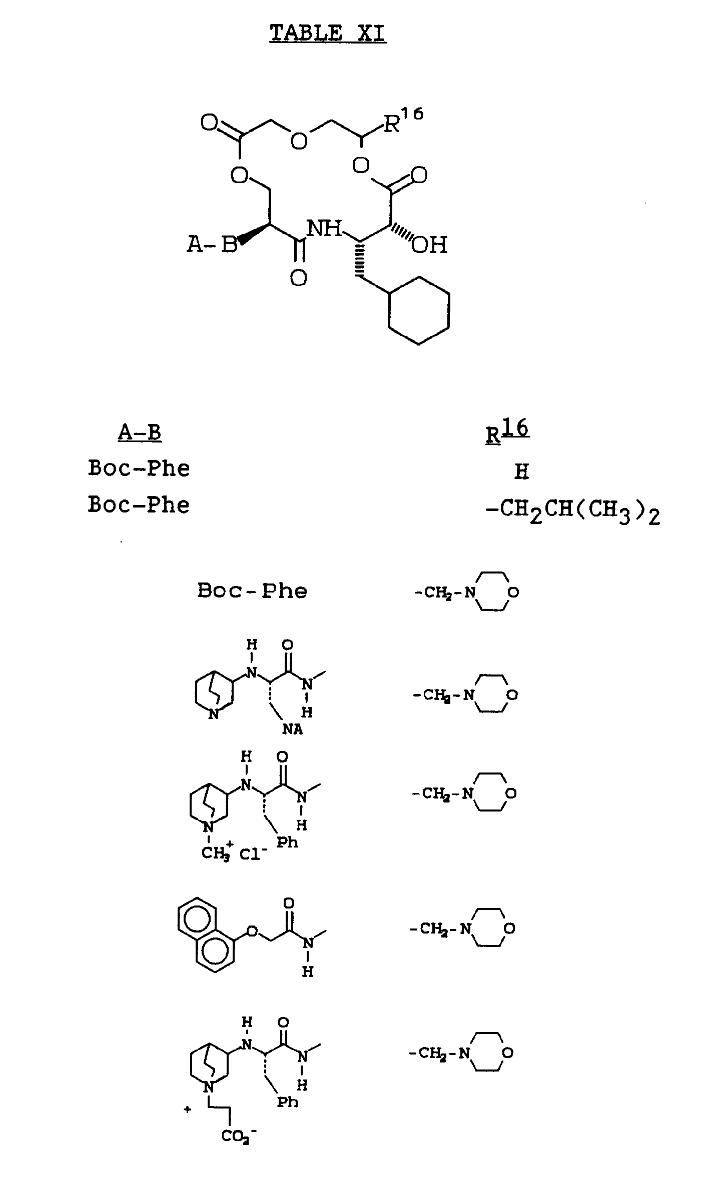

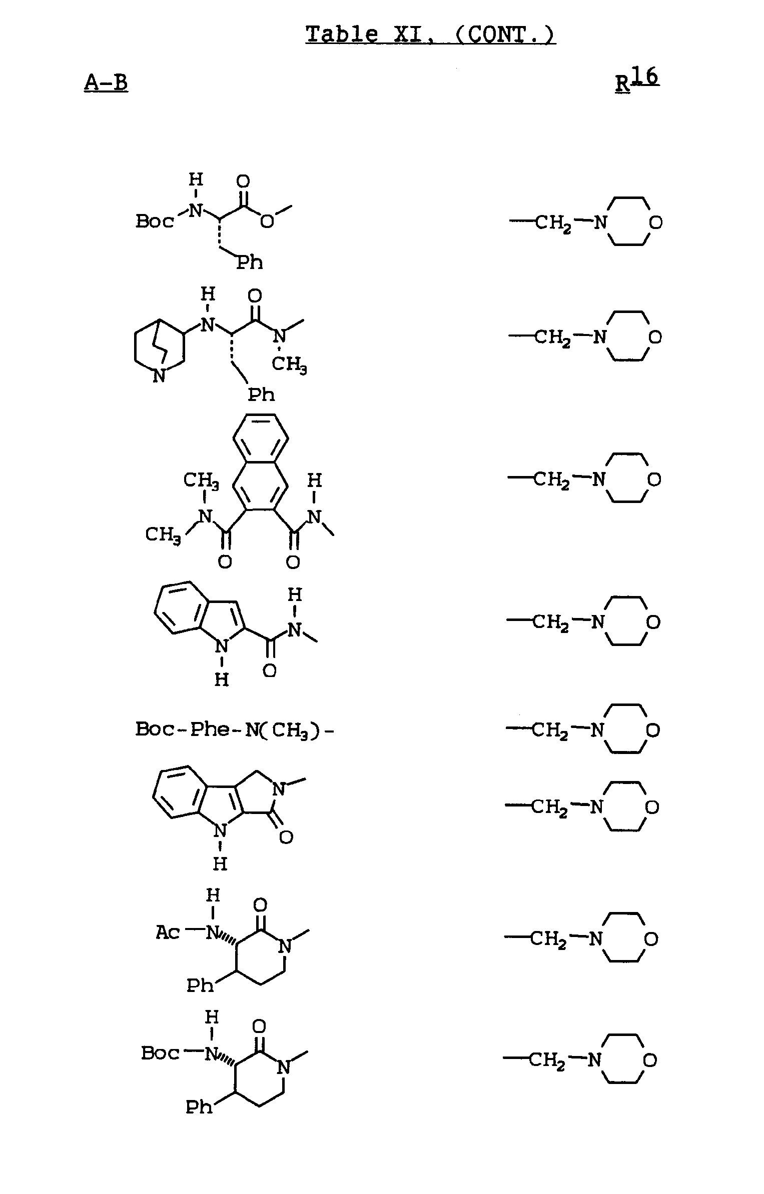

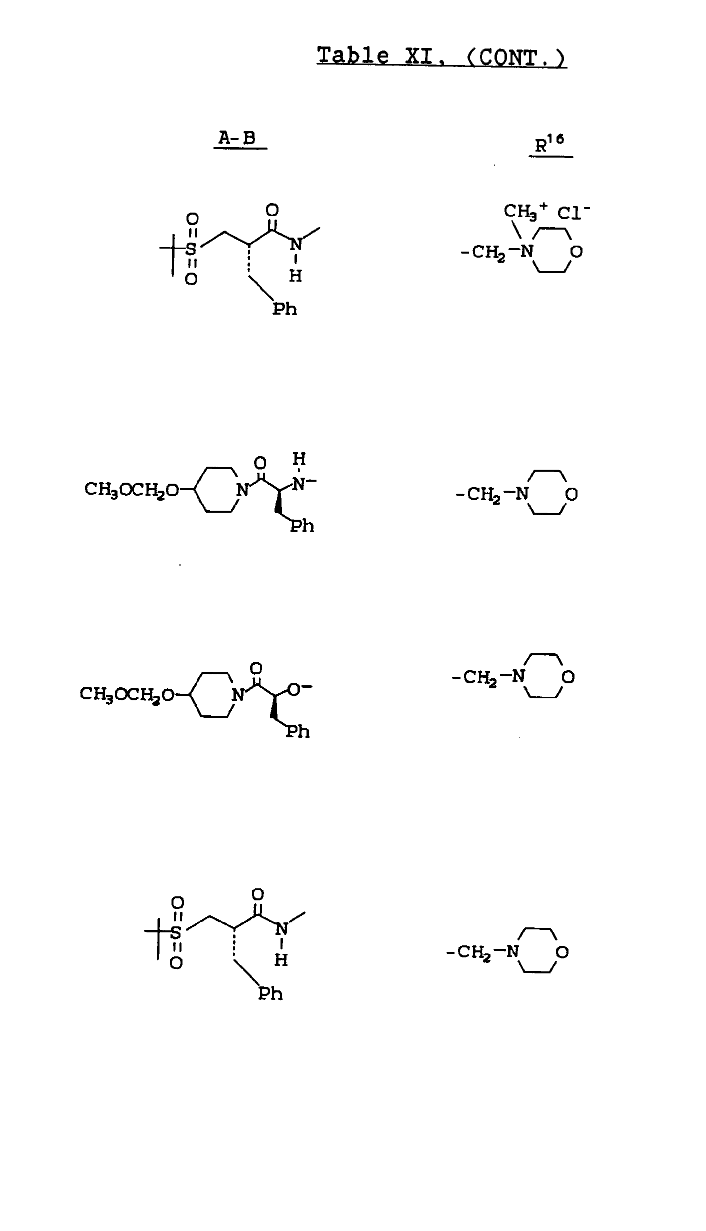

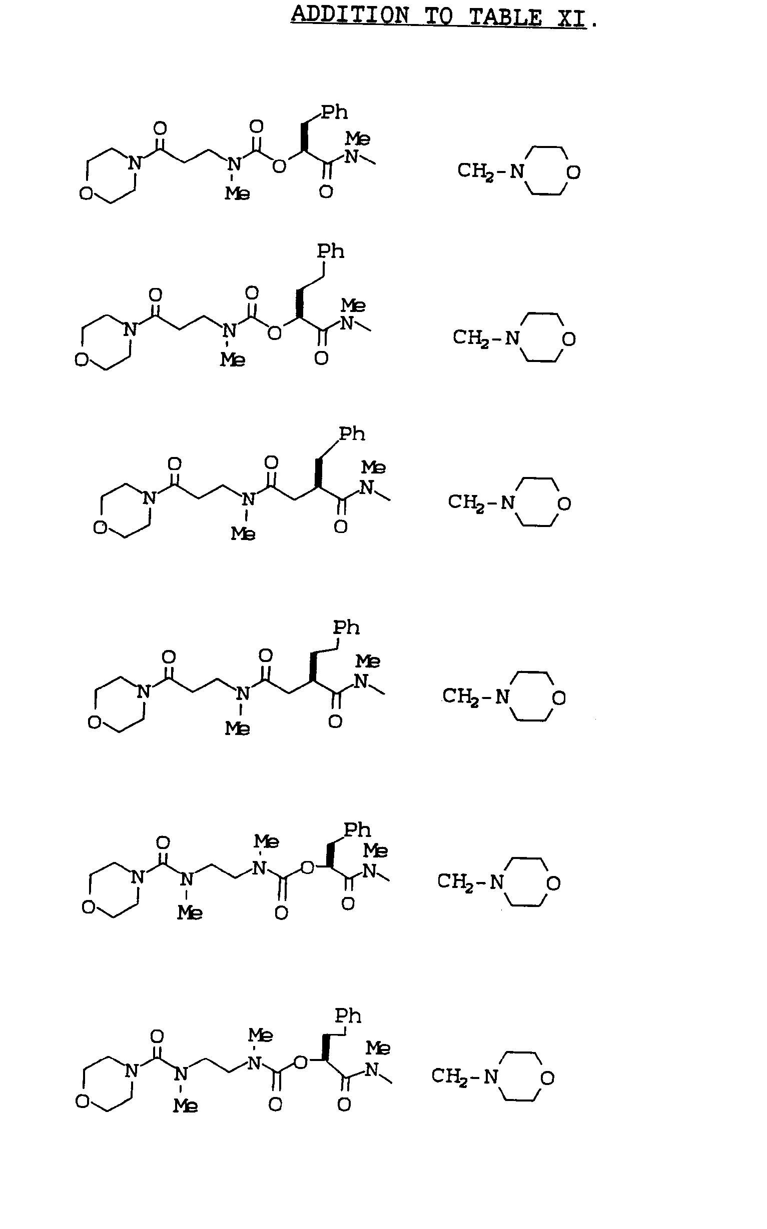

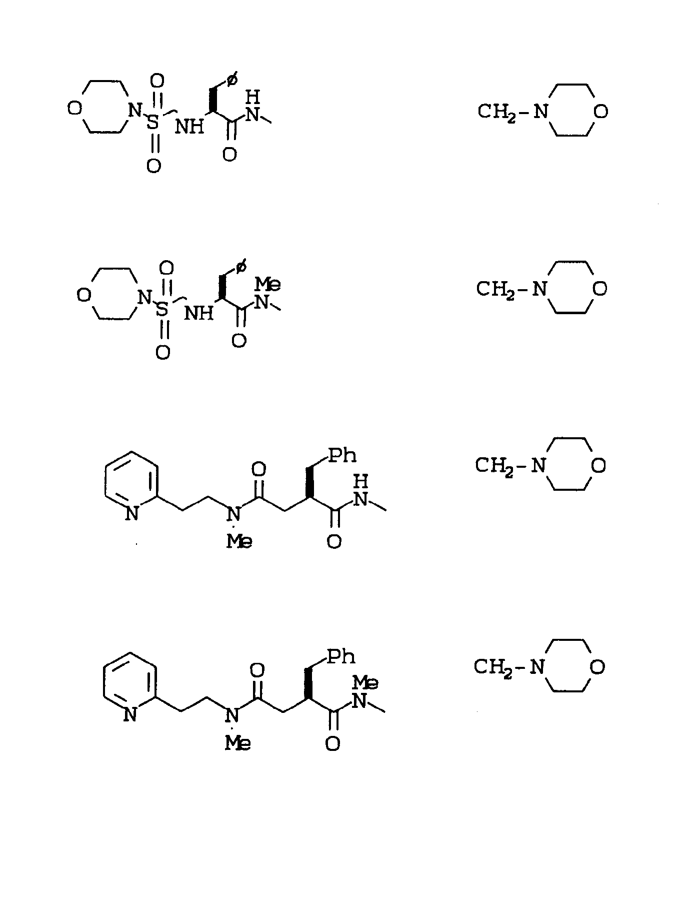

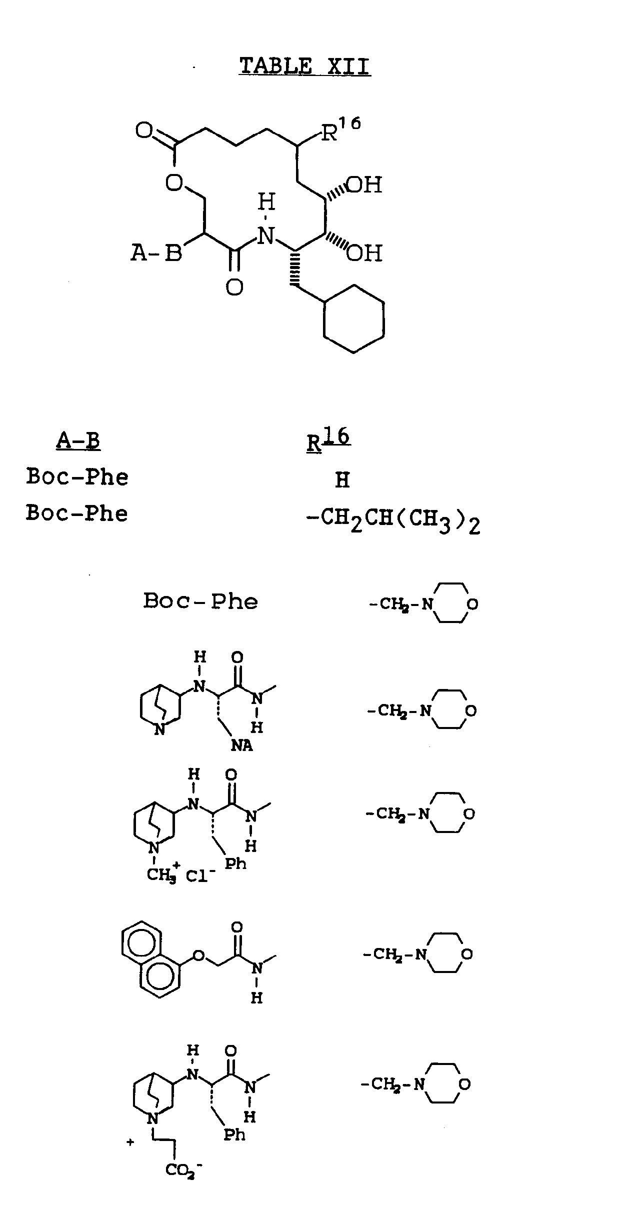

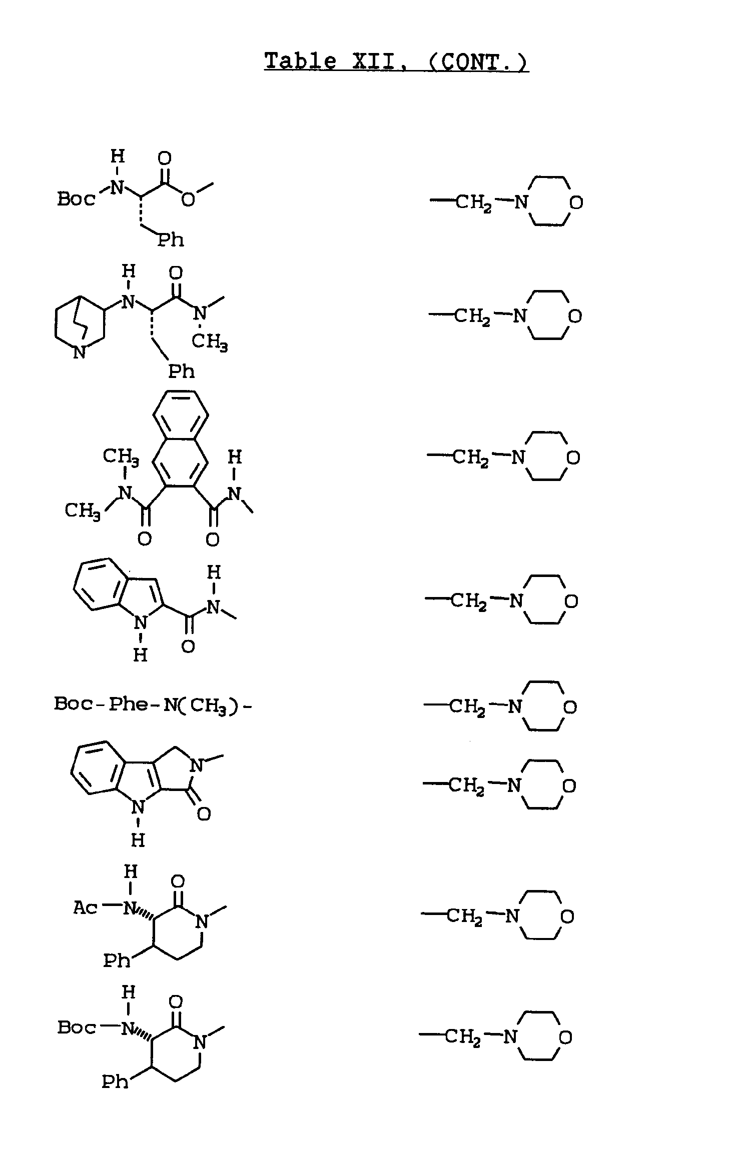

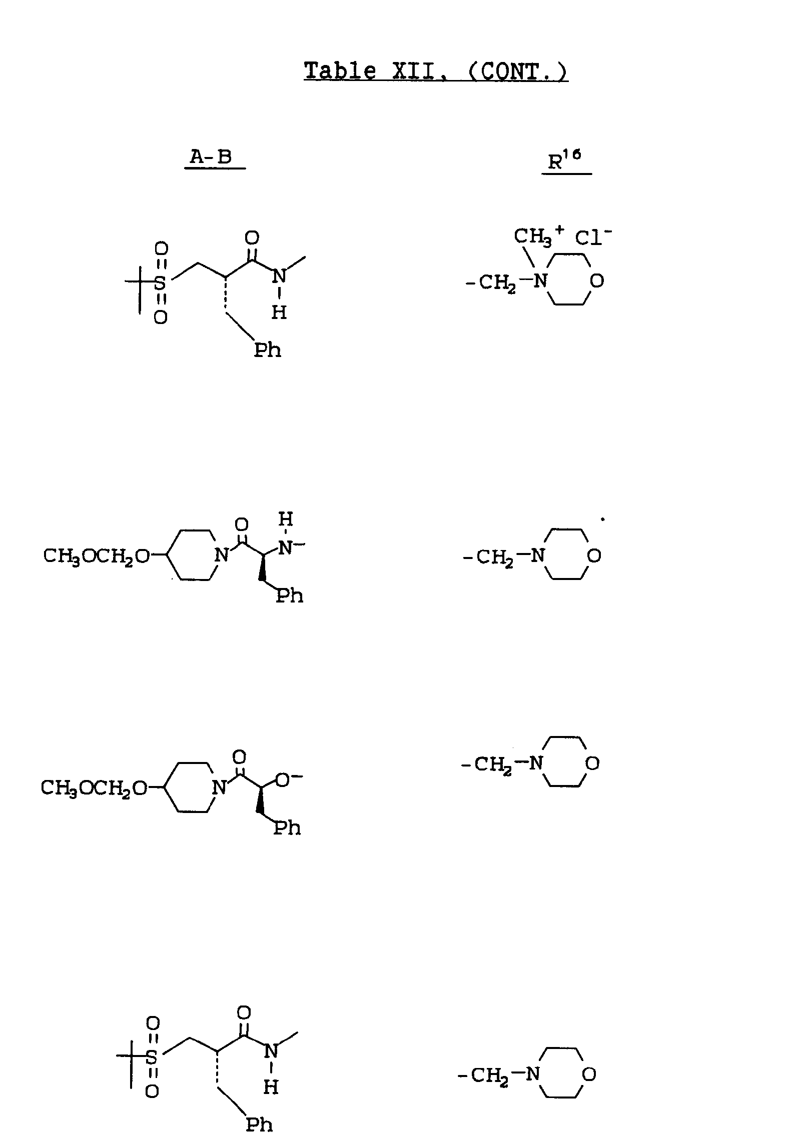

The abbreviations used herein have the following meaning:

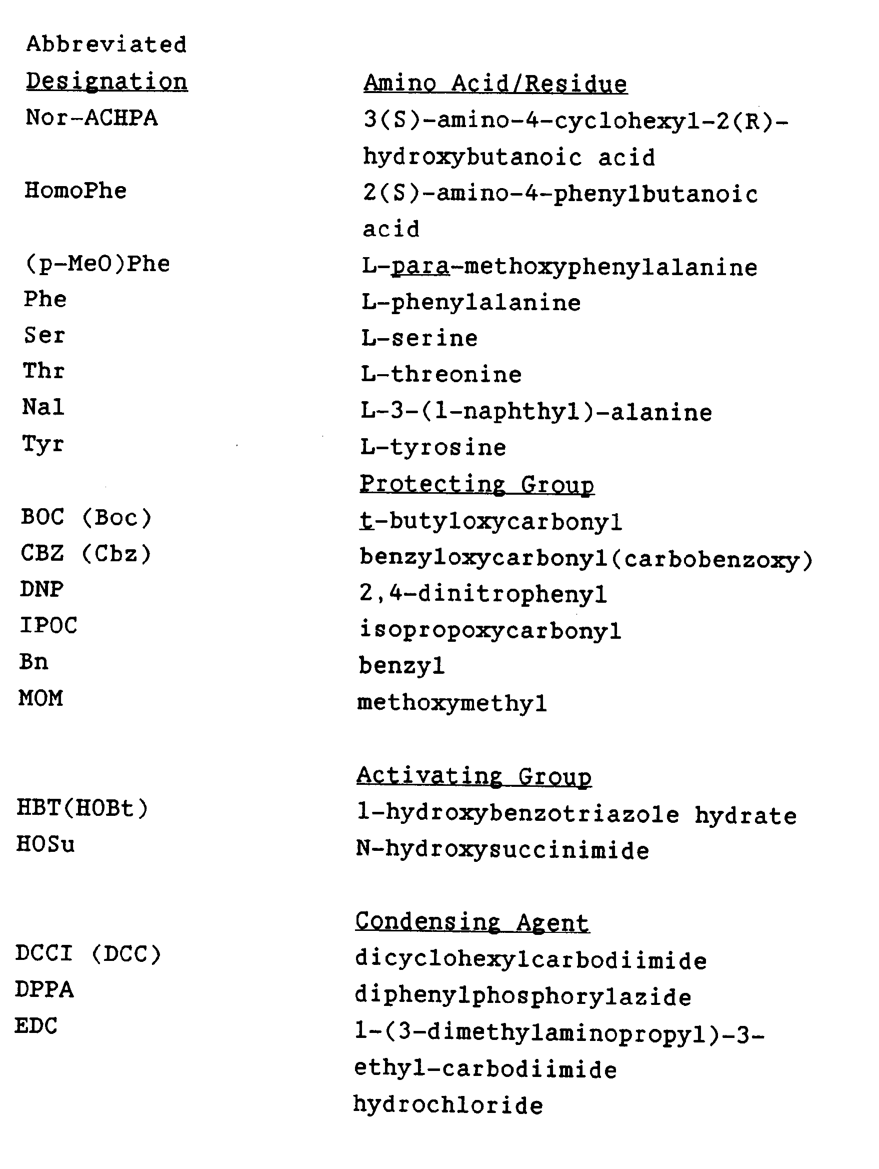

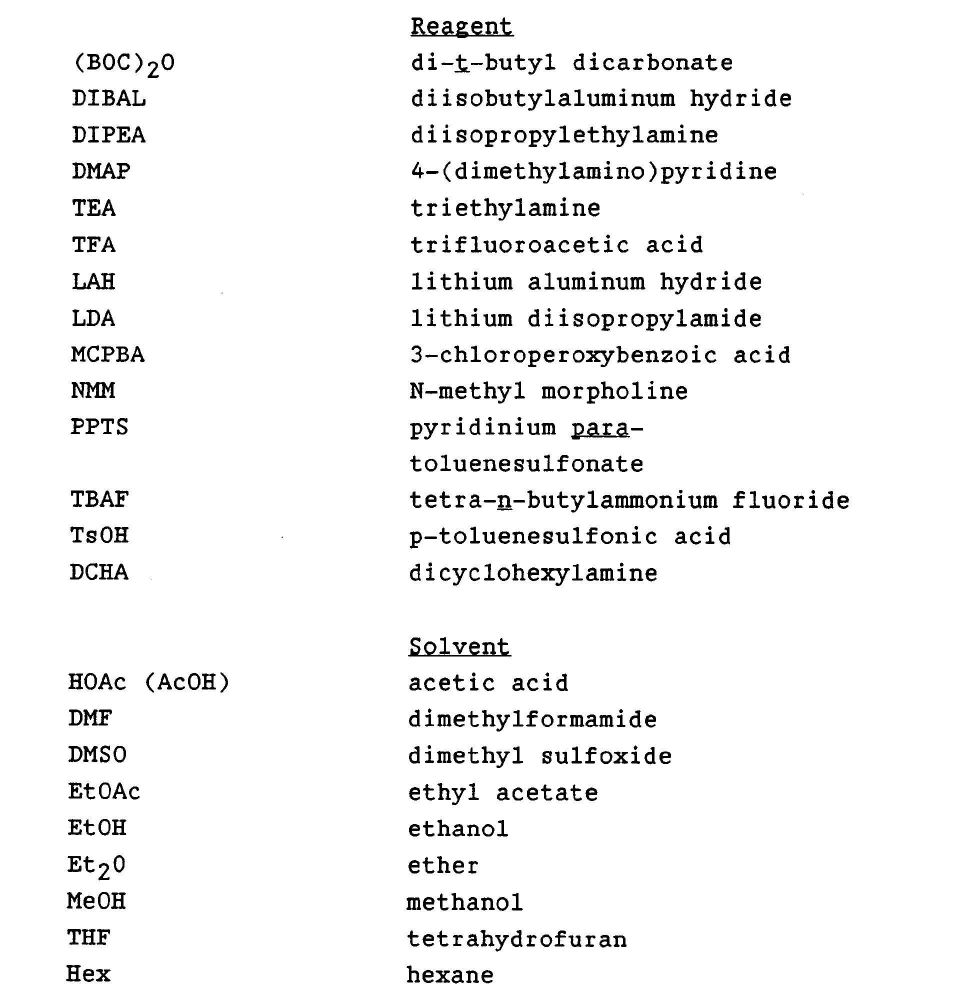

As can be seen, a unique aspect and essential feature of the present invention is the incorporation of certain cyclic elements thereby inparting enhanced oral absorption as renin inhibitors.

The Formula I compounds can be used in the form of salts derived from inorganic or organic acids and bases when there is an acidic or basic function. Included among such acid addition salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucohepta- noate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pal- moate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups can be quarternized with such agents as lower alkyl halides, such as methyl, ethyl, propyl, and butyl chloride, bromides and iodides: dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained.

The novel compounds of the present invention inhibit the angiotensinogen-cleaving action of the natural proteolytic enzyme, renin, and possess an excellent degree of activity in treating renin- associated hypertension, hyperaldosteronism, glaucoma and congestive heart failure.

The compounds of the invention are useful in treating hypertension. They are also of value in the management of acute and chronic congestive heart failure. These compounds may also be expected to be useful in the treatment of secondary hyperaldosteronism, primary and secondary pulmonary hyperaldosteronism, primary and secondary pulmonary hypertension, renal failure such as diabetic nephropathy, glomerulonephritis, scleroderma, glomerular sclerosis, proteinuria of primary renal disease, end stage renal disease, renal transplant therapy, and the like, renal vascular hypertension, left ventricular dysfunction, diabetic retinopathy and in the management of vascular disorders such as migraine, Raynaud's disease, luminal hyperplasia, and to minimize the atherosclerotic process. The application of the compounds of this invention for these and similar disorders will be apparent to those skilled in the art. The compounds of this invention are also useful to treat elevated intraocular pressure and to enhance retinal blood flow.

For these purposes the compounds of the present invention may be administered parenterally, by inhalation spray, orally, or rectally in dosage unit formulations containing conventional non-toxic pharmaceutically acceptable carriers, adjuvants and vehicles. The term parenteral as used herein includes subcutaneous injections, intravenous, intramuscular, intrasternal injection of infusion techniques. In addition to the treatment of warm- blooded animals such as mice, rats, horses, dogs, cats, etc., the compounds of the invention are effective in the treatment of humans.

The pharmaceutical compositions may be in the form of a sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanedioi. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectibles.

The inhibitors of this invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Dosage levels of the order of 2 to 35 grams per day are useful in the treatment of the above indicated conditions. For example, renin-associated hypertension and hyperaldosteronism are effectively treated by the administration of from 30 milligrams to 0.5 grams of the compound per kilogram of body weight per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

Thus, in accordance with the present invention there is further provided a pharmaceutical composition for treating renin-associated hypertension and hyperaldosteronism, comprising a pharmaceutical carrier and a therapeutically effective amount of Compound I.

The renin-inhibitory compounds of the present invention may also be utilized in diagnostic methods for the purpose of establishing the significance of renin as a causative or contributory factor in hypertension or hyper- aldosteronism in a particular patient. For this purpose the novel inhibitors of the present invention may be administered in a single dose of from 0.1 to 10 mg per kg of body weight.

Both in vivo and in vitro methods may be employed. In the in vivo method, a novel compound of the present invention is administered to a patient, preferably by intravenous injection, although parenteral administration is also suitable, at a hypotensive dosage level and as a single dose, and there may result a transitory fall in blood pressure. This fall in blood pressure, if it occurs, indicates supranormal plasma renin levels.

An in vitro method which may be employed involves incubating a body fluid, preferably plasma, with a novel compound of the present invention and, after deprotein ization, measuring the amount of angiotensin 11 produced in nephrectomized, pentolinium-treated rats. Another in vitro method involves mixing the plasma or other body fluid with a novel compound of the present invention and injecting the mixture into a test animal. The difference in pressor response with and without added peptide is a measure of the renin content of the plasma.

The following method was used for in vitro evaluation of the renin inhibitors of Formula I: The human plasma renin IC₅₀ values for inhibitors of Formula I were determined at pH 7.4 following the procedure described in J. Boger, L.S. Payne, D.S. Perlow, N.S. Lohr, M. Poe, E.H. Blaine, E. H. Elm, T.W. Schorn, B.I. Lamont, T.-Y. Lin, M. Kawai, D.H. Rich and D.F. Veber, J. Med. Chem., 28, 1779 (1985).

The following methods were used for in vivo evaluation of the renin inhibitors of Formua I: Intravenous evaluation of renin inhibitors in conscious sodium-deficient Rhesus monkeys: Rhesus monkeys, male and female, weighing 2.6 - 4.5 Kg, were surgically prepared with chronic arterial and venous catheters and vascular access ports for direct monitoring of mean arterial pressure (MAPI) and heart rate (HR). The animals were maintained on a low sodium diet (1.2 mmol Na/day) plus fruit for a week, and administered LASIX (furosemide) at2.5 mg/Kg, intramuscularly the evening prior to the experiment. The animals had been trained to sit quietly in the chairs with water AD LIBIUM for the duration of the experiment. The inhibitors were administered by bolus injection using 0.5% acetic acid-5% dextrose in water as the vehicle (0.4 ml/Kg), and MAP and HRwere measured. Blood samples were withdrawn at different time intervals beginning at the nadir of hypotensive response. PRA was determined as described above. The responsiveness of the animal during the experiment was verified with the standard inhibitor, SCRIP (Iva-His-Pro-Phe-His-Sta-Leu-Phe-NH₂, IC₅₀ = 3.7 nM). The i.v. dose of the standard inhibitor required to lower blood pressure by 50% of the maximal response was determined (ED₅₀ = 0.039 umoles/Kg). Inhibitors were tested at doses which were derived by comparing their IC₅₀ values to that of SCRIP. A projected ED₅₀ dose for each inhibitor was calculated using the following formula: ED₅₀ (Test Inhibitor, umoles/Kg) = ED₅₀ (SCRIP) X [IC₅₀ (Test Inhibitor)/IC₅₀ (SCRIP)], where the IC₅₀ values were determined against human plasma renin. In order to assure initial complete inhibition of endogenous monkey renin after i.v. administration, a multiple of projected ED₅₀ dose was chosen for each inhibitor. Percent inhibition of monkey PRA, changes in MAP and HR were calculated and plotted against time. The data points are averages of two or more monkey experiments.

Protocol for oral administration of renin inhibitors in conscious sodium-deficient Rhesus monkeys: Rhesus monkeys of either sex were surgically prepared and sodium depleted for administration of compounds orally, as above. The animals were fitted with a nasogastric feeding tube for oral administration of inhibitors. The inhibitors were administered orally as a solution (2.5 ml/Kg) in 0.1 M citric acid, and MAP and HR were measured over time. Plasma samples were collected at different time intervals up to 6 hours, and plasma renin activity (PRa) (ngAl/ml/hr) was determined using the RIA method (Travenol genetech's RIA Kit). Percent inhibition of primate PRA and peak changes in MAP and HR were calculated. All data points are an average of 2 - 5 monkey experiments.

The compounds of the present invention are prepared in accordance with the following reaction schemes and experimental procedures.

### SECTION A: PREPARATION OF INTERMEDIATES

The following carboxylic acids, useful in preparing macrocyclic inhibitors of Formula I may be prepared by methods described in the following references:

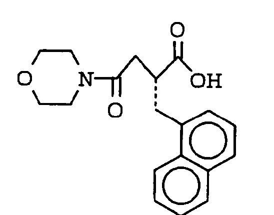

K. lizuka et al., J. Med. Chem., 31, 704 (1988);

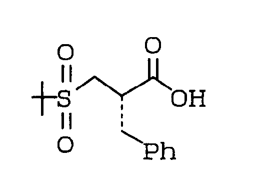

P. Buhlmayer et al., J. Med. Chem., 31, 1839 (1988);

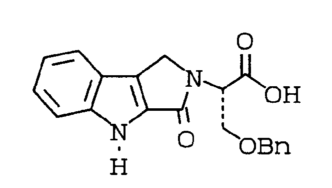

D.J. Kempf et al., "Design and Synthesis of Rigid Heterocyclic Phenylalanine Replacements for Incorporation into Renin Inhibitors," Proceedings of 11th Am. Peptide Symposium, Salk Institute, University of California, San Diego, July 9-14, 1989, ESCOM Scientific Publishers, BV Leiden, The Netherlands.

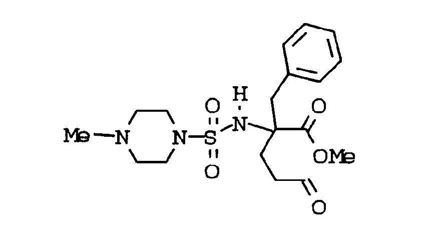

B. De, et. al., European Patent Application No. EP0365992, published May 2, 1990.

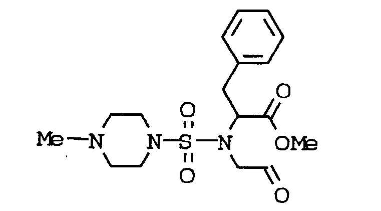

B. De, et. al., European Patent Application No. EP0365992, published May 2, 1990.

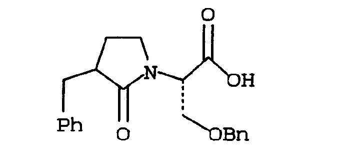

S. Thaisrivongs, et al, J. Med. Chem., 31, 1371(1988).

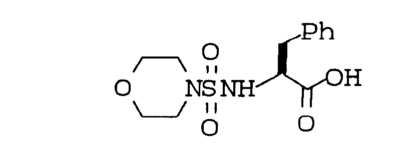

J.M. Hamby et al., EPO 380805 A1 published August 8, 1990.

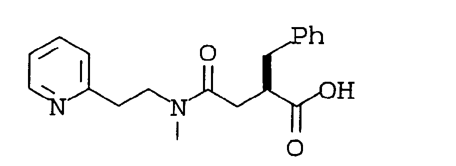

S.H Rosenberg et al., EP 0 410 260 A2 published January 30, 1991.

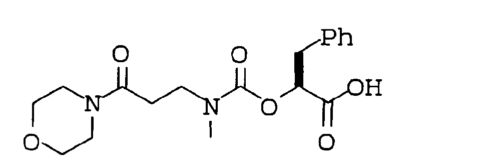

K. Hemmi. et al., USP 4,921,855 published May 1, 1990.

### Synthesis of norACHPA acetonide, 3

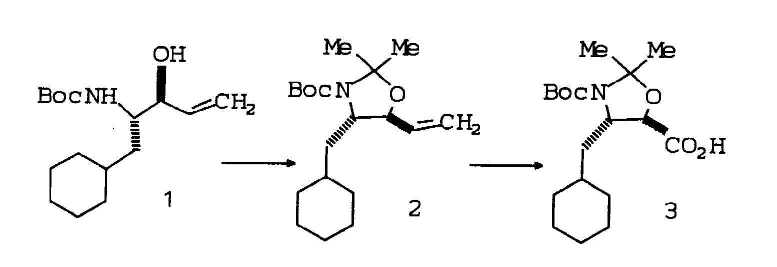

### (4S,5S)-3-tert-Butoxycarbonyl-4-cyclohexylmethyl-2,2-dimethyl-5-vinyloxazolidine (2)

A solution of 34.6 g (122 mmol, 1.0 equiv) of (3S,R,4S)-3-tert-butoxycarbonylamino-5-cyclohexyl-3-hydroxy-1-pentene (1, 6:1 S/R mixture at C-3, prepared according to the procedure of Rosenberg, S.H.; Plattner, J.J.; Luly, J.R.; Eur. Patent Appl. 0 230 266, 1987) and 1.16 g (6.10 mmol, 0.05 equiv) of p-toluenesulphonic acid monohydrate in 530 mL of dichloromethane was cooled to -78 °C and 63.5 g (75.0 mL, 61.0 mmol, 5 equiv) of dimethoxypropane was added. The reaction mixture was stirred at -22 °C overnight and then quenched by the addition of 1.23 g (1.70 mL, 12.2 mmol, 0.1 equiv) of triethylamine. The solution was washed sequentially with 250 mL portions of saturated aqueous sodium bicarbonate solution and 1 N aqueous sodium bisulfate solution, dried over anhydrous magnesium sulfate, and concentrated to give 43 g of an oil. Purification by silica gel chromatography (Water's Prep 500, 4% ethyl acetate/hexane) gave 25.9 g (66% yield, >97% diastereomeric purity by 300 MHz ¹H NMR) of the title compound as an oil: R_{f} 0.25 (5% ethyl acetate/hexane); ¹H NMR (300 MHz, CDCI₃) δ 5.95 (ddd, 1 H, J = 7.1, 10.3, 17.1 Hz), 5.33 (d, 1 H, J = 17.1 Hz), 5.23 (d, 1 H, J = 10.3 Hz), 4.26 (dd, 1 H, J = 3.5, 7.1 Hz), 3.81 (br s, 1 H), 1.98-0.85 (m, 19 H), 1.47 (s, 9 H); MS(FAB) 378 (M+1+matrix (dithiothreitol) - Boc).

Anal. calcd. for C₁₉H₃₃NO₃: C, 70.55; H, 10.28; N, 4.33. Found: C, 70.45; H, 9.99; N, 4.29.

### (4S,5R)-3-tert-Butoxycarbonyl-4-cyclohexylmethyl-2,2-dimethyloxazolidine-5-carboxylic acid (norACHPA, 3)

To a solution of 25.9 g (80.1 mmol, 1.0 equiv) of (4S,5S)-3-tert-butoxycarbonyl-4-cyclohexylmethyl-2,2-dimethyl-5-vinyloxazolidine (2) in 1500 mL of acetone at room temperature was added in four portions over 3 h a solution of 102.8 g (480 mmol, 6.0 equiv) of sodium periodate and 1.07 g (4.01 mmol, 0.05 equiv) of 50% ruthenium dioxide on carbon in 1500 mL of water. After the final addition, the reaction was judged complete by TLC analysis and excess reagent was quenched by the addition of 14 mL of isopropyl alcohol. The resultant mixture was filtered through celite and concentrated. The aqueous residue was diluted with 2 L of 1:1 1 N aqueous sodium bisulfate and 1 N aqueous sodium bisulfite and extracted with four 750-mL portions of dichloromethane. The combined organic phases were dried over anhydrous magnesium sulfate and decolorized with activated charcoal. Concentration gave 25.9 g (95%) of a slightly green solid. An analytical sample was prepared by recrystallization from ethyl acetate/hexane: R_{f} 0.30 (10% MeOH/CH₂CI₂); ¹H NMR (300 MHz, CDCI₃) D 4.38 (s, 1 H), 4.35 (br s, 1 H), 1.93 (br d, J = 12 Hz), 1.80-0.85 (m, 12 H), 1.66 (s, 3 H), 1.58 (s, 3 H), 1.48 (s, 9 H); MS(FAB) 342 (M+1), 286, 242.

Anal. calcd. for C₁₈H₃₁NO₅: C, 63.32; H, 9.15; N, 4.10. Found: C, 63.38; H, 9.25; N, 4.04.

### N^{α}-(Quinuclidin-3(RS)-yl)-Phe-t-butyl ester hydrochloride (4)

To a solution of 9.00 g (56.25 mmol) 3-quinuclidinone and 4.15 g (18.75 mmol) Phe-O-t-Bu in 50 ml methanol was added over a 12 hour period a solution of 2.95 g (46.9 mmol) sodium cyanoborohydride in 13 ml methanol. After stirring for an additional 8 hours, 5.78 g (50.0 mmol) pyridine hydrochloride was added and after 1 1/2 hours stirring, sodium chloride was removed by filtration. The filtrate was concentrated to a foam which was treated with 15 ml methanol and 50 ml ethyl acetate to give a slurry of the byproduct 3-hydroxy qui- nuclidine hydrochloride (74% of excess) which was removed by filtration. The filtrate was concentrate to an oil and charged with 10 ml methanol to a 5 X 200 cm column of LH-20 and eluted with methanol. The product fraction contained 6.54 g of a mixture of diastereomers in a 55:45 ratio as established by HPLC.

### N^{α}-(Quinuclidin-3(S)-yl)-Phe-t-butyl ester hydrochloride (4S)

A solution of 7.0 g of the isomer mixture (from Example 1) in 25 ml water was treated with 2.62 g sodium bicarbonate bringing the pH to 9.0. The clear solution was lyophilized and the crystalline residue was extracted with 50 ml of acetonitrile. Evaporation of the solvent and treatment with 25 ml ether gave crystals which were filtered off, washed with ether, and dried. The yield was 2.49 g (65%) of an isomer established by x-ray crystal structure analysis to be the S,S-diastereomer hydrochloride.

### N^{α}-(Quinuclidin-3(S)-yl)Phe·2 HCI (5S)

A solution of 1.91 g of 4S in 3 ml concentrated hydrochloric acid was left for 3 hours and then concentrated to an amorphous mass. To remove excess HCI the material was redissolved in 10 ml water and concentrated to yield 1.98 g of the dihydrochloride.

### [N^{α}-(N-methylquinuclidin-3(S)-yl)Phe-O-t-Bu]⁺I⁻(6S)

A solution of 4S in 2 ml methanol was treated with 310 µl. (5.0 mmol) methyl iodide and 68.3 mg (1.26 mmol) sodium methylate. After 2 hours at room temperature the reaction mixture was concentrated and charged with 4 ml of methanol to a 2.5 X 210 cm column of LH-20 and eluted with methanol. The product fractions contained 366 mg of product with an NMR spectrum consistent with the assigned structure.

### N^{α}-(N-Methylquinuclidin-3(S)-yl)-phenylalanine⁺CI⁻·HCl

A solution of 366 mg (775 µM) of 6S in 1 ml of water and 2 ml of conc. hydrochloric acid was aged for 2 hours, concentrated and charged with 2 ml methanol to 2.5 X 210 cm LH20 column and eluted with methanol. The product fraction contained 254 mg of product with NMR and mass spectra consistent with the structure.

### N^{α}-(Quinuclidin-3(RS)-yl)Nal-OCH₃·HCl (8)

A solution of 2.20 g (8.28 mmol) of 3-(1-naphthyl)-Ala-OCH_{a"}HCI and 4.02 g (25 mmol) of 3-quinuclidinone hydrochloride in 30 ml of methanol was treated over the course of 11 hours with a solution of 1.20 g (20.7 mmol) of sodium cyanoborohydride in 7.5 ml of methanol. After the addition was complete the reaction mixture was allowed to stir for 4 days and then treated with 2.42 g (20.9 mmol) pyridine hydrochloride and after stirring for 3 hours, the solvent was removed using a rotary evaporator. The residue was stirred with 10 ml methanol and the insoluble sodium chloride was removed by filtration and washed with 5 ml methanol. The filtrate was treated with 60 ml ethyl acetate and the solution was seeded with 3-RS-quinuclidinol hydrochloride. The alcohol byproduct was removed by filtration and the filtrate was concentrated in vacuum to an oil. A second crop of this byproduct was removed by crystallization with a solvent mixture consisting of 50 ml ethyl acetate, 50 ml of acetonitrile, and 2 ml of methanol. The filtrate was concentrated in vacuo to 5.36 g of an amorphous residue. This was dissolved in 5 ml of methanol and chromatographed over a 5 X 200 cm column of LH-20 eluting with methanol. The product-containing fractions were combined and concentrated, yielding 4.4 g of product.

### N^{α}-(Quinuclidin-3(S)-yl)Nal-OCH₃·HCl (8S)

Using mixtures of acetonitrile and ether for crystallization, a total of 440 mg of the 3(S)-diastereomer was obtained from the above mixture (8).

### N^{α}-(Quiniclidin-3(RS)-yl)Nal-OH dihydrochloride (9)

Na-(Quiniclidin-3(RS)-yl)Nal-OMe-HCI (8) (0.5g) was dissolved in 6N HCI (10 ml), and the mixture was refluxed for 4 hours and then allowed to stand at room temperature overnight. The mixture was then concentrated in vacuo to dryness, and the residue was dried in a vaccum dessicatorover NaOH and dryness, and the residue was dried in a vaccum descicator over NaOH and P₂0₅ overnight to give the desired product as a foam (0.55 g). ¹H NMR (300 MHz, CD₃OD): δ 1.9-2.2 (m, 3H), 2.45 (m, 2H), 3.16-3.95 (m. 7H), 4.2-4.5 (m, 3H), 7.35-7.7 (m, 4H), 7.88 (dd, 2H), 8.3 (d, 1 H), MS(FAB): m/e 325 (MH⁺).

### N^{α}-(2,2,6,6-Tetramethylpiperidin-4-yl)-Phe-O-t-Bu (9)

A solution of 11.55 g (60.2 mmol) 2,2,6,6-tetramethylpiperidin-4-one hydrochloride and 4.44 g (20 mmol) Phe-O-t-Bu in 40 ml of methanol was treated over an eight hour period with a solution of 3.19 g (50.8 mmol) sodium cyanoborohydride in 6 ml of methanol. After stirring overnight a solution of 8.21 g (71.0 mmol) pyridine hydrochloride in 20 ml of methanol was added and stirring continued for 1 1/2 hour. Sodium chloride was removed by filtration, and the filtrate was concentrated to an oil. The byproduct 2,2,6,6-tetramethylpiperidin-3-ol (69.5% of excess) crystallized on addition of 40 ml ethyl acetate and 40 ml of acetonitrile, and was removed by filtration. The filtrate was concentrated to an amorphorus mass which was charged with 10 ml methanol to a 5 X 200 cm LH-20 column and eluted with methanol. Evaporation of the solvent from the product-containing fractions and crystallization from 10 ml acetonitrile afforded 5.34 g (61.5%) of product, which had NMR and mass spectra in accord with assigned structure.

### N^{α}(1-Ethylpiperidin-3(RS)-yl)Phe-O-t-Bu (10)

A solution of 8.18 g (50.0 mmol) 1-ethyl-3-piperidone HCI, 5.15 g (20.0 mM) Phe-O-t-Bu and 1.64 g (19.3 mM) sodium acetate in 250 ml methanol was treated over a 14 hour period with a solution of 1.88 g (30.0 mmol) sodium cyanoborohydride in 10 ml methanol. After stirring overnight, 3.47 g (30.0 mmol) pyridine hydrochloride was added, and after 2 hour stirring sodium chloride was removed by filtration and the reaction mixture was concentrated to an oil. This was dissolved in 16 ml methanol and chromatographed on a 5 X 200 cm LH-20 column eluted with methanol. The product fraction contained 4.01 g (67.2%) of a mixture of diastereomers with NMR and mass spectra in accord with the assigned structure.

### Methyl 2-Hydroxy-3-phenylpropionate (11)

To a stirred solution of phenylalanine (16.5 g, 0.1 mole) in 2N sulfuric acid at 0°C, was added sodium nitrite (10.5 g, 1.5 equiv) in small portions over a period of 0.5 hours and the mixture stirred overnight. Aqueous phase was extracted with ether (5 X 250 mL) and the ethereal extracts were washed with saturated aqueous solution of sodium chloride, dried over anhydrous magnesium sulfate and concentrated to give phenyllactic acid (1 equiv) in methanol (15 equiv) at 0°C and the mixture stirred at room temperature overnight. Removal of volatiles in vacuo and chromatographic purification of the oil (20-25% ethyl acetate in hexane) gives methyl 2-hydroxy-3-phenylpropionate (11). H NMR (300 MHz, CDCl₃): 8 7.33-7.196 (m, 5H), 4.451 (dd, 1 H), 3.764 (s, 3H), 3.1225 (dd, 4.45 Hz, 13.95 Hz, 1H), 2.9575 (dd, 7 Hz, 14 Hz, 1H), 2.787 (br s, 1H).

### Methyl 2-Methanesulfonyloxy-3-phenylpropionate (12)

A dichloromethane solution of methyl 2-hydroxy-3-phenylpropionate (11) is treated with triethylamine (1.1 equiv) and methanesulfonyl chloride (1.1 equiv) at 0°C. Upon completion of reaction, the mixture is dissolved in dichloromethane/ ether and washed with saturated aqueous solution of sodium chloride, dried and concentrated. Purification of crude material by flash column chromatography (40% ethyl acetate in hexane) gives methyl 2-methanesulfonyloxy-3-phenyl-propionate (1.6 g, 93%). ¹H NMR (300 MHz CDCl₃): 8 7.358-7.233 (m, 5H), 5.173 (dd, 4.26 Hz, 8.8 Hz, 1 H), 3.793 (s, 3H), 3.301 (dd, 4.23 Hz, 14.38 Hz, 1 H), 3.1295 (dd, 8.8 Hz, 14.3 Hz, 1 H), 2.766 (s, 3H).

### 3-Acetylthioquinuclidine (13)

To a THF (300 mL) solution of triphenylphosphine (42 g, 160 mmol, 2 equiv) at 0°C was added diisopropyl azodicarboxylate (32 mL, 162 mmol) to produce a pale yellow solid. A THF (300 mL) solution of 3-quinuclidinol (10.2 g, 80.2 mmol) and thiolacetic acid was added dropwise to the yellow reaction mixture and stirred overnight. THF was removed in vacuo and the residue was dissolved in ether (500 mL) and extracted with 10% HCI (4 X 150 mL). The aqueous acidic phase was back extracted with ether/ethyl acetate (75 mL/25 mL) and then neutralized to pH 7 by the addition of sodium bicarbonate cautiously in small portions. The aqueous layer was then basified to pH 9-10 by adding a few drops of 10 N NaOH, then extracted with dichlormethane (5 X 200 mL), dried over anhydrous sodium sulfate and concentrated. Purification by flash column chromatrography using 5% MeOH in chloroform as eluent gave 3-acetylthioquinuclidine (10.5 g, 71%).

¹H (300 MHz, CDClₐ): δ 3.725-3.63 (m, 1H), 3.427 (dd, 10.23 Hz, 13.7 Hz), 2.9-2.75 (dd, 4H), 2.678 (dd, 5.7 Hz, 14.2 Hz, 1 H), 2.326 (S, 3H), 1.9-1.82 (m, 1 H), 1.81-1.675 (m, 3H), 1.53-1.4 (m, 1 H).

### 3-Mercaptoquinuclidine (14)

Acetylthioquinuclidine it treated with sodium methoxide in methanol. Upon completion of hydrolysis the so- vent is removed in vacuo to obtain 3-mercaptoquinclidine which is used in the next step without further purification.

### 2-(Quinuclidin-3-yl)thio-3-phenylpropionic acid (15)

To a stirred solution of 3-metcaptoquinuclidine in DMF at 0°C is added sodium hydride (1 equiv) and the mixture stirred for 0.5 hours. A solution of methyl-2-methanesulfonyloxy-3-phenylpropionate (1 equiv) in DMF or THF is added to the reaction mixture at 0°C and the resulting mixture stirred. After completion of reaction, methanol is added dropwise to quench the reaction. The volatiles are removed in vacuo and the residue is purified by flash chromatography to obtain the methyl esterwhich is sponified with aqueous sodium hydroxide (1 N, 1 equiv) in methanol to afford 2-(quinuclidin-3-yl)thio-3-phenylpropionic acid.

### 2-(Quinuclidin-3-yl)oxy-3-phenylpropionic acid (16)

To a slurry of potassium hydride (1 equiv) in THF at 0°C is added 3-quinuclidinol (1 equiv) and the mixture stirred for 0.25 hours. A THF solution of methyl-2-methanesulfonyloxy-3-phenylpropionate (1 equiv) is added to the reaction mixture and stirred until completion of reaction. The reaction is quenched by slow addition of methanol, the mixture is concentrated and the residue is purified by flash chromatography to afford methyl ester which is treated with aqueous sodium hydroxide (IN, NaOH) to produce the 2-(quinuclidin-3-yl)oxy-3-phenylpropionic acid.

### Methyl 2-Benzylacrylate (17)

Methyl 2-benzylacrylate is prepared by the method of J. Harley-Mason et al., Tetrahedron, 36,1063(1980).

### Methyl 2-(quinuclidin-3-yl)thiomethyl-3-phenylpropionate (18)

3-Acetylthioquinuclidine is hydrolyzed to 3-mercaptoquinuclidine by treating with sodium methoxide in methanol. To the sodium salt of 3-metcaptoquinuclidine in methanol at 0°C, is added methyl 2-benzylacrylate and the mixture stirred for a few hours. Upon completion of reaction, methanol is removed and the residue is subjected to flash column chromatography to give the title compound.

### 2-(quinuclidin-3-yl)sulfonylmethyl-3-phenylpropionic acid (19)

Methyl 2-(quincuclidin-3-yl)thiomethyl-3-phenylpropionate is treated with 2 equivalents of m-chloroperoxybenzoic acid in CH₂CI₂. The reaction mixture is filtered to remove m-chlorobenzoic acid and the filtrate is concentrated. The residue is purified by flash chromatography, and then subjected to the action of 6N HCI-HOAc (1:1) at 60°C for 24 hours, providing the title compound.

### SECTION B: PREPARATION OF MACROCYCLIC RENIN INHIBITORS OF FORMULA I where W = -NH-, Z = -OH, and Y = -OCO-:

Schemes 2, 3, 5 and 6 illustrate the preparation of macrocyclic renin inhibitors of Formula I in which W = -NH-, Z = -OH, and Y = -OCO-. Here Boc-Nor-ACHPA acetonide (3) is esterified with an optionally substituted carboxyl-protected hydroxyacid, itself prepared from lactone or epoxide precursors (see General Procedure for esterfication below). The carboxyl protecting group is then removed, and the resulting carboxylic acid is esterified with the side-chain hydroxyl of Cbz-serine t-butyl ester (or Cbz-allo-threonine t-butyl ester). After removal of the Boc and acetonide protecting groups from the Nor-ACHPA element, macrocylization is effected using one of the general procedures (Method A, B, or C) described below. An alternative route to macrocycles is illustrated in Scheme 4. Here coupling of Boc-Nor-ACHPA acetonide to the (optionally substituted) hydroxyacid (in which the carboxylic acid is protected as a benzyl ester) is carried out as before, but then the Boc and acetonide groups are removed from the Nor-ACHPA element and the resulting amino derivative is coupled with Boc-Serine or O-benzyl Boc-serine. Hydrogenolytic removal of the benzyl ester (and benzyl ether when O-benzyl Boc-serine is used) provides the macrocyclization precursor, which is cyclized using EDC and DMAP.

Subsequent to the macrocyclization, the Cbz or Boc protecting group is removed from the macrocycle and the resulting amino derivative is acylated with a carboxyl acid (as described in Methods D, E and F below), or with an acid chloride or a sulfonyl chloride using standard procedures. As will be obvious to those skilled in the art, functional groups within the (optional) substituent of the protected hydroxy acid used to esterify Nor-ACHPA acetonide as described above (the R¹⁶ substituent in Formula I) may require protection during the following steps of the synthesis. In these cases, protecting groups are chosen so as to be compatible with the Boc, Cbz, and t-butyl ester protecting groups used for other amine and carboxylic acid groups as described in the general synthetic route above. Examples are the t-butyldimethylsilyl group for alcohols, the trimethylsilylethyloxycar- bonyl group for amines and trimethylsilylethyl ester for carboxylic acids.

### General Procedure for Esterification Using EDC/DMAP.

A solution of the appropriate acid and alcohol (0.95-1.2 equiv) in dichloromethane (0.1-0.33 M) was cooled to 0°C and dimethylaminopyridine (DMAP, 0.05-0.1 equiv) and 1-(3-dimethylaminopropyl)3-ethylcarbodiimide hydrochloride (EDC, 1.5-3 equiv) were added. The mixture was stirred at 0°C for 2-16 hours, until the reaction was judged complete by TLC analysis. The solution was then diluted with ethyl acetate, washed sequentially with 1 N aqueous sodium bisulfate, water, saturated aqueous sodium bicarbonate and saturated aqueous sodium chloride, dried over anhydrous magnesium sulfate and concentrated. Purification by silica gel chromatography provided the desired ester in good yield.

### General Procedure for Macrocyclization. Method A:

The macrocycle precursor was deprotected with 1:1 1 dicholormethane/triflouroacetic acid at room temperature until the reaction was judged complete by TLC analysis (4-6 hours). The solution was concentrated and trace amounts of acid were removed azeotropically with tetrahydrofuran and toluene. The resultant oil was dried over P₂0₅/KOH under vacuum for several hours and then dissolved in tetrahydrofuran to form a 0.001 M solution. The solution was cooled to 0°C and treated with N-methyl morpholine (1.1 equiv), hydroxybenzotriazole (HOBt, 4.0 equiv), and 1-(3-dimethylaminopropyl)-3-ethylcarbodiimide hydrochloride (EDC, 4.0 equiv). The mixture was allowed to warm to room temperature and was stirred for a total of 5-6 days. Solvent was removed in vacuo. The residue was dissolved in ethyl acetate, washed sequentially with 1 N aqueous sodium bisulfate, water, saturated aqueous sodium bicarbonate and saturated aqueous sodium chloride, dried over anhydrous magnesium sulfate and concentrated. Purification by silica gel and/or Sephadex LH-20 gel chromatography provided the macrocycles.

### General Procedure for Macrocyclization. Method B:

Deprotection was carried out as above. The deprotected material was dissolved in dimethylformamide (DMF) to form a 0.002 M solution. The solution was coooled to 0°C and treated with diphenylphosphorylazide (2.0 equiv) and triethylamine (2.2 equiv). After the reaction mixture was stirred at 0°C for several hours, 7.5°C for 3 days, and room temperature for 16 hours, the DM F was removed in vacuo. Isolation and purification were performed as described for Macrocyclization Method A.

### General Procedure for Macrocyclization. Method C:

Deprotection with TFA in dichloromethane was carried out as described above. A solution of the deprotected material in THF (0.38 mmol in 5 mL, 0.076 M) was added via a syringe pump over a period of 20 hours to a refluxing solution of EDC (2 equiv), N,N-dimethylaminopyridine (DMAP, 3 equiv) and DMAP.HCI (2 equiv) in chloroform (25 mL). After addition, the reaction mixture was added to 500 mL of ethyl acetate and washed with saturated aqueous solution of sodium bicarbonate, sodium chloride, dried over anhydrous magnesium sulfate and concentrated. Purification by flash column chromatography or MPLC on silica gel afforded the macrocycles in yields higher than those by Macrocyclization Methods A and B.

### General Procedure for Deprotection and Acylation of Macrocycles. Method D.

A solution of macrocycle in the indicated solvent was stirred with 10% Pd/C under 1 atm of hydrogen for several hours until the deprotection was judged complete by TLC analysis. The mixture was filtered through celite and concentrated. The resultant oil was dissolved in dichloromethane (0.05-0.2 M) unless otherwise indicated, cooled to 0°C, and treated with the appropriate acid (1.1-3 equiv), hydroxybenzotriazole (HOBt, 2.0 equiv), and 1-(3-dimethylaminopropyl)-3-ethylcarbodiimide hydrochloride (EDC, 2.0 equiv). The solution was stirred overnight with gradual warming to room temperature and then diluted with ethyl acetate, washed sequentially with 1 N aqueous sodium bisulfate, saturated aqueous sodium bicarbonate and saturated aqueous sodium chloride, dried over anhydrous magnesium sulfate and concentrated. Purification by silica gel and/or Sephadex LH-20 gel chromatography provided the acylated macrocycles.

### General Procedure for Deprotection and Acylation of Macrocycles. Method E.

A solution of macrocycle in 4:1 trifluoroacetic acid/methyl sulfide was stirred at room temperature for 6-8 hours or overnight. The solution was concentrated and trace amounts of acid were removed azeotropically with methanol and toluene. The resultant oil was dried over P₂0₅/KOH under vacuum for several hours and then suspended in dichloromethane or the indicated solvent. Upon addition of triethylamine (1.1 equiv), the oil dissolved. The solution was cooled to 0°C and treated with the appropriate acid, HOBt, and EDC. Isolation and purification were performed as described for Deprotection and Acylation Method A.

### General Procedure for the Deprotection and Acylation of Macrocycles. Method F.

A solution of macrocycle in 1:1 trifluoroacetic acid/dichloromethane was stirred at room temperature for 0.5-1 hours. The solution was concentrated and trace amounts of acid were removed azeotropically with tetrahydrofuran and toluene. The resultant oil was dried over P₂0₅/KOH under vacuum for several hours. It was then dissolved in dichloromethane (0.05-0.2 M), cooled to 0°C, and treated with the appropriate acid, N-methyl morpholine, HOBt, and EDC. The solution was stirred overnight with gradual warming to room temperature. The mixture was applied directly to a silica gel column or an aqueous workup was performed as follows. The solution was diluted with ethyl acetate, washed sequentially with saturated aqueous sodium bicarbonate and saturated aqueous sodium chloride, dried over anhydrous magnesium sulfate and concentrated. Purification by silica gel and/or Sephadex LH-20 gel chromatography provided the acylated macrocycles.

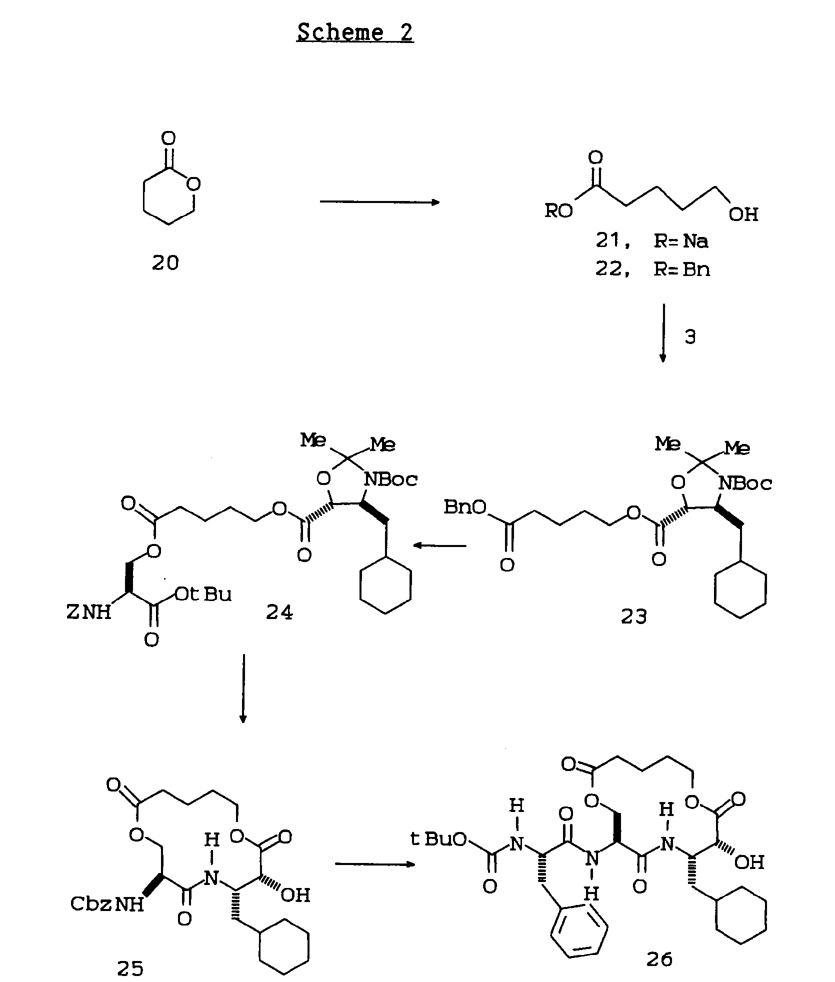

### Sodium 5-Hydroxypentanoate (21)

A suspension of 800 mg (8.0 mmol) of d-valerolactone in 8 mL (8.0 mmol, 1.0 equiv) of 1 N aqueous sodium hydroxide was heated at 65 °C overnight. The clear solution was cooled and concentrated. Toluene was added and the resultant slurry was concentrated to give a white solid: IR (nujol mull) 1550 cm-¹.

### Benzyl 5-Hydroxypentanoate (22)

To a suspension of 569 mg (4.06 mmol) of sodium 5-hydroxypentanoate (21) in 3 mL of acetone was added 1.39 g (0.97 mL, 8.11 mmol, 2.0 equiv) of benzyl bromide and 65 mg (0.203 mmol, 0.05 equiv) of tetrabutylammonium bromide. The mixture was heated at 45 °C for 24 h, cooled, and concentrated. The residue was dissolved in 200 mL of ethyl acetate, washed with 50-mL portions of 1 N aqueous sodium bisulfate, saturated aqueous sodium bicarbonate and saturated aqueous sodium chloride, dried over anhydrous magnesium sulfate and concentrated to give 1.49 g of a pale yellow oil. Purification by MPLC (Lobar C-column, 45% ethyl acetate/hexane) gave 641 mg (76%) of the title compound as an oil: ¹H NMR (300 MHz, CDCI₃) δ 7.38-7.26 (m, 5 H), 5.12 (s, 2 H), 3.64 (t, 2 H, J = 6.3 Hz), 2.41 (t, 2 H, J = 7.2 Hz), 1.80-1.71 (m, 2 H), 1.64-1.54 (m, 3 H).

### Benzyl ester 23

Boc-NorACHPA acetonide 3 (302 mg, 0.884 mmol, 1.0 equiv) was coupled with 208 mg (0.998 mmol, 1.1 equiv) of benzyl 5-hydroxypentanoate (22) using 254 mg (1.33 mmol, 1.5 equiv) of EDC and 11 mg (0.088 mmol, 0.1 equiv) of DMAP in 4 mL of dichloromethane for 4 hours according to the general procedure for EDC/DMAP esterification. Purification by MPLC (Lobar B-column, 15% ethyl acetate/hexane) gave 467 mg (99%) of the title compound as an oil:R_{f} 0.25 (15% ethyl acetate/hexane); ¹H NMR (300 MHz, CDCl₃) δ 7.37-7.26 (m, 5 H), 5.11 (s, 2 H), 4.32 (s, 1 H), 4.3-4.2 (br s, 1 H), 4.16 (br m, 2 H), 2.40 (br t, J = 7.0 Hz, 2 H), 1.90 (br d, J = 11.3 Hz, 1 H), 1.83-0.85 (m, 16 H), 1.61 (s, 3 H), 1.59 (s, 1.5 H), 1.56 (s, 1.5 H), 1.47 (s, 9 H); MS(FAB) 532 (M+1), 432.

Anal. calcd. for C₃₀H₄₅NO₇: C, 67.77; H, 8.53; N, 2.63. Found: C, 67.79; H, 8.78; N, 2.59.

### Acyl serine derivative 24

A solution of 351 mg (0.660 mmol) of benzyl ester 23 in ethyl acetate was stirred with 10% palladium on carbon under an atmosphere of hydrogen overnight. The mixture was then filtered through celite and concentrated. The resultant white crystalline solid was dissolved in 5 mL of dichloromethane, cooled to 0°C, and treated with 214 mg (0.726 mmol, 1.1 equiv) of N-carbobenzoxy-L-serine tert-butyl ester, 190 mg (0.990 mmol, 1.5 equiv) of EDC, and 8.0 mg (0.66 mmol, 0.1 equiv) of DMAP according to the general procedure for EDC/DMAP esterification. Purification by MPLC (Lobar B column, 25% ethyl acetate/hexane) provided 369 mg (78%) of the title compound as an oil: R_{f} 0.32 (25% ethyl acetate/hexane); ¹H NMR (300 MHz, CDCl₃) δ 7.37-7.24 (m, 5 H), 5.56-5.54 (d, J = 6.8 Hz, 1 H), 5.12 (s, 2 H), 4.53-4.44 (m, 2 H), 4.43-4.20 (m, 3 H), 4.20-4.08 (m, 2 H), 2.36-2.28 (m, 2 H), 1.88 (br d , J = 11.7 Hz, 1 H), 1.86-1.38 (m, 10H), 1.60 (s, 3 H), 1.59 (s, 3 H), 1.47 (s, 9 H), 1.45 (s, 9 H), 1.38-1.12 (m, 4 H), 1.07-0.85 (m, 2 H); MS(FAB) 719 (M+1), 619, 563.

Anal. calcd. for C₃₈H₅₈N₂O₁₁: C, 63.49; H, 8.13; N, 3.90. Found: C, 63.32; H, 8.37; N, 3.91.

### Macrocycle 25

Macrocyclization of 349 mg (0.485 mmol) of acyl serine derivative 24 was carried out according to the general procedure (Method A) described above. Purification by flash chromatography (20 x 150 mm silica gel, 50% ethyl acetate/hexane) provided 114 mg (46%) of the title compound: R_{f} 0.24 (50% ethyl acetate/hexane); ¹H NMR (300 MHz, CD₃OD) δ 7.36 (m, 5 H), 5.13 (d, J = 12.7 Hz, 1 H), 5.08 (d, J = 12.7 Hz, 1 H), 4.46-4.42 (m, 2 H), 4.35-4.27 (m, 2 H), 4.23 (d, J = 2.5 Hz, 1 H), 4.13-4.08 (overlapping d's, 2 H), 2.38-2.36 (br m, 2 H), 1.87-1.60 (m, 9 H), 1.47 (br t, J = 7.0 Hz, 2 H), 1.41-1.13 (m, 2 H), 1.20 (br t, J = 9.5 Hz, 2 H), 1.03-0.84 (m, 2 H); MS(FAB) 505 (M+1).

Anal. calcd. for C₂₆H₃₆N₂O₈: C, 61.89; H, 7.19; N, 5.55. Found: C, 61.78; H, 7.30; N, 5.53.

### Macrocycle 26

A solution of 58.3 mg (0.116 mmol) of 25 was deprotected in 1:1 methanol/ethyl acetate and treated with 61.3 mg (0.231 mmol, 2.0 equiv) of Boc-Phe, 35.4 mg (0.231 mmol, 2.0 equiv) of HOBt, and 44.3 mg (0.231 mmol, 2.0 equiv) of EDC as described in the general procedure (Method A). Purification by flash chromatography (20 x 150 mm silica gel, 70% ethyl acetate/hexane) followed by MPLC (Sephadex LH-20, MeOH) gave 33.3 mg (47%) of the title compound: R_{f} 0.16 (70% ethyl acetate/hexane); ¹H NMR (300 MHz, CD₃OD) d 7.30-7.18 (m, 5 H), 4.67 (dd, J = 3.9, 7.4 Hz, 1 H), 4.45-4.23 (m, 5 H), 4.15-4.04 (m, 2 H), 3.12 (dd, J = 4.8, 13.8 Hz, 1 H), 2.81 (dd, J = 9.8,13.8 Hz, 1 H), 2.46-2.32 (m, 2 H), 1.88 (m, 17 H), 1.36 (s, 9 H), 1.04-0.87 (m, 2 H); MS(FAB) 618 (M+1), 562, 518.

Anal. calcd. for C₃₂H₄₇N₃0₉: C, 62.22; H, 7.67; N, 6.80. Found: C, 61.93; H, 7.45; N, 6.70.

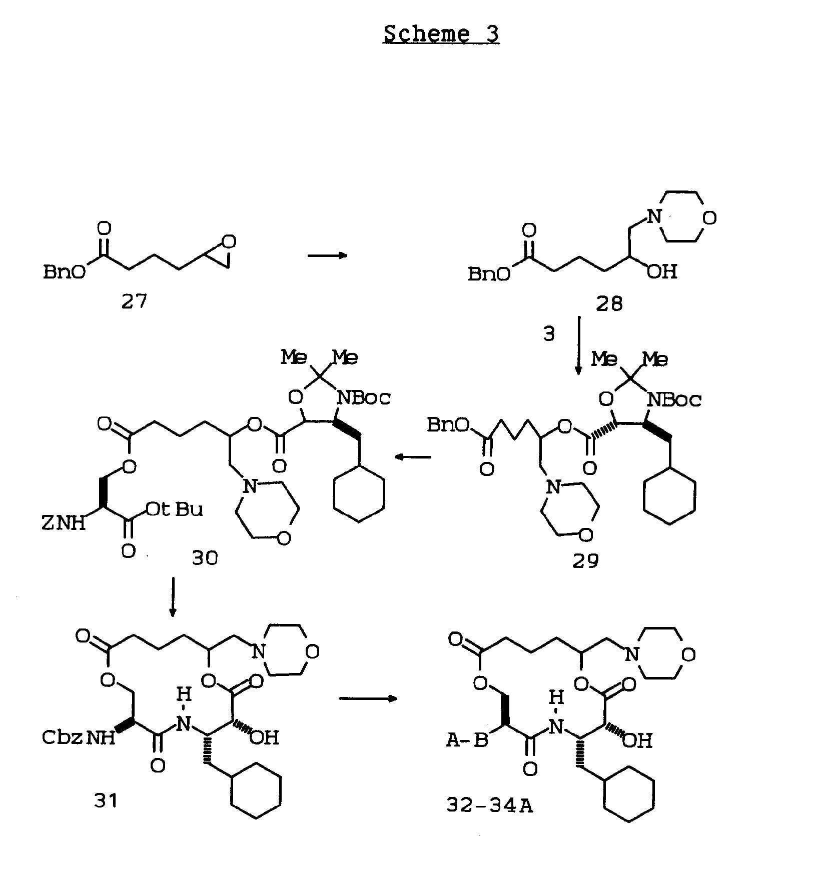

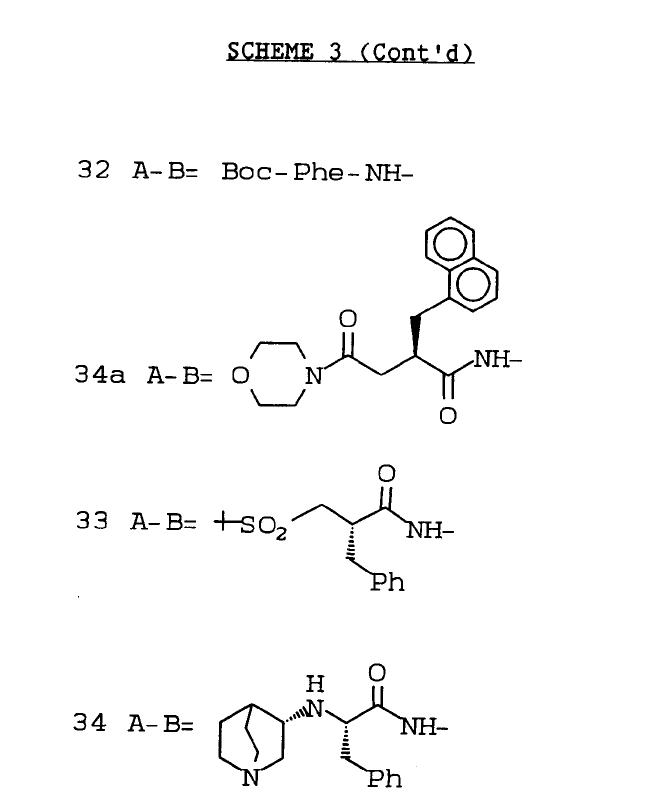

### Benzyl 5-hydroxy-6-(4'-morpholino)hexanote (28)

To a solution of 28.4 g (129 mmol) of epoxide 27 and 22.5 g (22.5 mL, 258 mmol, 2.0 equiv) of morpholine in 250 mL of ether was added 20 g of neutral alumina. The resultant suspension was stirred at room temperature for 6 days until TLC analysis indicated the starting epoxide was completely consumed. The mixture was filtered. The filtrate was diluted with 1 L of ethyl acetate, washed with three 500-mL portions of water, dried over anhydrous magnesium sulfate and concentrated to give 34.6 g (87%) of an oil: R_{f} 0.32 (5% methanol/ dichloromethane); ¹H NMR (300 MHz, CDCl₃) δ 7.35-7.26 (m, 5H), 5.10 (s, 2H), 3.75-3.62 (m, 5H), 3.41 (s, 1 H), 2.66-2.58 (m, 2H), 2.44-2.19 (m, 6H), 1.89-1.68 (m, 2H), 1.45-1.38 (m, 2H); MS(FAB) 308 (M+1).

### Benzyl ester 29

Boc-NorACHPA acetonide 3 (2.77g, 8.11 mmol, 1.1 equiv) was coupled with 2.26 g (7.37 mmol, 1.0 equiv) of alcohol 28 using 2.12 g (11.1 mmol, 1.5 equiv) of EDC and 45 mg (0.369 mmol;, 0.05 equiv) of DMAP in 14 mL of dichloromethane for 3 hours according to the general procedure for EDC/DMAp esterification with one modification: in the work-up, the acid wash was omitted. Purification by MPLC (2 Lobar B-columns, 30% ethyl acetate/hexane) gave 1.66 g (36%) of a slower eluting iosmer (R_{f} 0.21 (25% ethyl acetate/hexane) along with 1.65 g (36%) of the title compound as an oil: R_{f} 0.27 (25% ethyl acetate/ hexane); ¹H NMR (300 MHz, CDCl₃) 8 7.42-7.26 (m, 5H), 5.20-5.06 (m, 3H), 4.37 (s, 1 H), 4.5-4.2 (br m, 1 H), 3.65-3.50 (br m, 4H), 2.57 (br m, 2H), 2.52-2.71 (m, 6H, 1.91 (br d, J = 10.9 Hz, 1 H), 1.86-0.85 (m, 16H), 1.69 (s, 6H), 1.47 (s, 9H); MS (FAB) 631 (M+1).

Anal. calcd, for C₃₅H₅₄N₂O₈: C, 66,64; H. 8.63; N, 4.44. Found: C, 66.64; H, 8.79; N, 4.38.

### Acyl serine derivative 30

A solution of 1655 mg (2.62 mmol) of benzyl ester 29 in tetrahydrofuran was stirred with 10% palladium on carbon under an atmosphere of hydrogen overnight. The mixture was then filtered through Celite and concentrated. The resultant oil was dissolved in 25 mL of dichloromethane, cooled to 0°C, and treated with 782 mg (2.65 mmol, 1.01 equiv) of N-carbobenzoxy-L-serine tert-butyl ester, 754 mg 3.93 mmol, 1.5 equiv) of EDC, and 16.0 mg (0.131 mmol, 0.05 equiv) of DMAP according to the general procedure for EDC/DMAP esterification with one modification: in the work-up, the acid wash was omitted. The crude product was filtered through LH-20 (30 X 2000 mm, methanol) and further purified by MPLC (2 Lobar B columns, 35% ethyl acetate/hexane) to provide 1783 mg (83%) of the title compound as an oil: ¹H NMR (300 MHz, CDCl₃) δ 7.37-7.26 (m, 5H), 5.55 (d, J = 8.2 Hz, 1 H), 5.28-5.12 (m, 3H), 4.51-4.28 (m, 5H), 3.64-3.58 (m, 4H), 2.57 (br m, 2H), 2.45-2.27 (m, 6H), 1.91 (br d, J = 11.8 Hz, 1 H), 1.68 (s, 6H), 1.47 (s, 9H), 1.80-0.85 (m, 16H); MS(FAB) 719 (M+1), 619, 563. Anal. calcd. for C₄₃H₆₇N₁₃O₁₂: C, 63.14; H, 8.26; N, 5.14. Found: C, 62.82; H, 8.08; N, 5.08.

### Macrocycle 31

Macrocyclization of 398 mg (0.486 mmol) of acyl serine derivative 30 was carried out according to the general procedure (Method A) described above with one modification: in the work-up, the acid wash was omited. The crude product was filtered through LH-20 (30 X 2000 mm, methanol) and further purified by flash chromatography (20 X 150 mm silica gel, 2.5% methanol/dichloromethane) to provide 104 mg (35%) of the title compound: R_{f} 0.33 (5% methanol/dichloromethane); ¹H NMR (300 MHz, CD₃OD) δ 7.35-7.27 (m, 5H), 5.18-5.11 (m, 1 H), 5.11 (s, 2H), 4.51 (ddd, J = 2, 5, 10Hz, 1 H), 4.42 (dd, J = 4.6, 9.4Hz, 1 H), 4.23 (d, J = 2.1 Hz 1 H), 4.23-4.11 (m, 2H), 3.70-3.60 (m, 4H), 2.73 (dd, J = 9.2, 13.1 Hz, 1 H), 2.60-2.51 (m, 2H), 2.44-2.33 (m, 5H), 1.88-0.80 (m, 17H); MS(FAB) 604 (M+1).

Anal. calcd. for C₃₁H₄₅N₃O₉: C, 61.68; H, 7.51; N, 6.96. Found: C, 61.63; H, 7.48; N, 7.04.

### Macrocycle 32

A solution of 23.3 mg (0.0386 mmol) of 31 was deprotected in tetrahydrofuran and treated with 20.5 mg (0.0772 mmol, 2.0 equiv) of Boc-Phe, 118 mg (0.0772 mmol, 2.0 equiv) of HOBt, and 14.8 mg (0.0772 mmol, 2.0 equiv) of EDC as described in the general procedure (Method D) with one modification: the aqueous workup was omitted. The reaction mixture was subjected directly to flash chromatography (20 X 150 mm silica gel, 40- mL portions of 1, 2, 3, 4, and 5% methanol/chloroform) to give 23.4 mg (84%) of the title compound: R_{f} 0.29 (5% methanol/dichloromethane); ¹H NMR (300 MHz, CDCl₃) δ 7.35-7.23 (m, 5H), 6.97 (br m, 1 H), 6.79 (br m, 1 H), 5.10 (br m, 1 H), 4.95 (br m, 1 H), 4.68 (br m, 1 H), 4.45-4.20 (br m, 3H), 4.29 (s, 1 H), 4.00 (dd, J = 6.8, 10.7Hz, 1 H), 3.65 (br s, 4H), 3.16-3.00 (br m, 2H), 2.68 (br dd, J = 10.7, 12.0Hz, 1 H), 2.61-2.24 (m, 7H), 2.04 (brs, 1H), 1.40 (s, 9H), 1.86-0.82 (m, 17H); MS(FAB) 717 (M+1).

Anal. calcd. for C₃₇H₅₆N₄O₁₀: C, 61.99; H, 7.87; N, 7.82. Found: C, 61.81; H, 8.00; N, 7.70.

### Macrocycle 33

A solution of 212 mg (0.351 mmol) of 31 was deprotected in 1:1 tetrahydrofuran/methanol and treated with 200 mg (0.702 mmol, 2.0 equiv) of (2R)-3-tert-butysulfonyl-2-phenylmethyl-propionic acid (prepared according to P. Buhlmayer et al., J. Med. Chem., 31, 1839-46 (1988)), 108 mg (0.702 mmol, 2.0 equiv) ofHOBt. and 135 mg (0.702 mmol, 2.0 equiv) of EDC as described in the general procedure (Method D) with one modification: the aqueous workup was omitted. The reaction mixture was subjected directly to flash chromatography (20 X 150 mm silica gel, 150-mL portions of 2.5 and 5% methanol/dichloromethane). Further purification by MPLC (Lobar B column, 2% 10:1 methanol/ammonium hydroxide in chloroform) gave 184 mg (71%) of the title compound: R_{f} 0.26 (5% methanol/dichloromethane); ¹H NMR (300 MHz, CD₃OD/CDCl₃) δ 7.31-7.19 (m, 5H), 5.16 (br m, 1 H), 4.59 (dd, J = 4.2, 9.7Hz, 1 H), 4.45 (dt, J = 1.7, 6.2Hz, 1 H), 3.66 (br s, 4H), 3.55 (dd, J = 10.2,13.3Hz, 1 H), 3.31-3.21 (m, 1H), 3.05 (dd, J = 6.8, 13.7Hz, 1 H), 2.92 (dd. J = 2.6, 13.4Hz, 1H), 2.80-2.72 (overlapping dd, 2H), 2.59-2.53 (m, 2H), 2.49-2.31 (m, 5H), 1.30 (s, 9H), 1.88-0.88 (m, 17H); MS(FAB) 736 (M+1).

Anal. calcd. for C₃₇H₅₇M₃O₁₀S: C, 60.39; H, 7.81; N, 5.71. Found. C, 60.42; H, 7.81; N, 5.56.

### Inhibitor 34

A solution of 350 mg (0.580 mmol) of 31 in 1:1 tetrahydrofuran/methanol was deprotected according to the general procedure (Method D) and treated with 0.15 mL (141 mg, 1.39 mmol, 2.4 equiv) of N-methylmorpholine, 222 mg (0.638 mmol, 1.1 equiv) of N-(quinuclidin-3-(S)-yl)phenylalanine, 93.2 mg (0.609 mmol, 1.05 equiv) of HOBt, and 180 mg (0.0870 mmol, 1.5 equiv) of DCC (not EDC). The solution was stirred overnight with gradual warming to room temperature and then concentrated. The residue was submitted directly to flash chromatography (20 X 150 mm silica gel, 125 mL of 117.5/7.5, 125 mL of 110/15 and 250 mL of 102.5/22.5 chloroform to 10:1 methanol/ammonium hydroxide) and purified further by MPLC (Lobar B column, 160/7.5 chloroform to 10:1 1 methanol/ammonium hydroxide) to give 255 mg (60%) of the title compound: R_{f} 0.63 (105/20 chloroform to 10:1 methanol/ammonium hydroxide); ¹H NMR (300 MHz, CD₃OD) δ 7.33-7.20 (m, 5H), 5.14 (m, 1H), 4.64 (dd, J = 4.2, 8.7Hz, 1 H), 4.49 (m, dt, J = 2, 7Hz, 1 H), 4.25 (d, J = 2.0Hz 1 H), 4.20 (dd, J = 4.3, 10.5Hz, 1 H), 4.07 (dd, J = 8.8, 10.5 Hz, 1 H), 3.66 (br 2, 4H), 3.34 (dd, J = 3.0, 5.3Hz, 1 H), 3.06 (dd, J = 5.1, 1.3Hz, 1 H), 2.89 (ddd, J = 2.5, 9.5, 13.4Hz, 1H), 2.81-2.52 (m, 10H), 2.44-2.26 (m, 5H), 2.14 (ddd, J = 2.0, 4.3, 13.3Hz, 1 H), 1.89-0.85 (m, 21 H); MS(FAB) 726 (M+1).

### Macrocycle 34a

A solution of 26.3 mg (0.0436 mmol) of inhibitor 31 was deprotected in 1:1 DMF/methanol and treated with 17.8 mg (0.0523 mmol, 1.2 equiv) of (2R)-3-[(morpholin)-4-yl)carbonyl]-2-(1-naphthylmethyl]-propionic acid (prepared according to K. lizuka et al., J. Med. Chem., 31, 704-706 (1988)), 13.3 mg (0.0871 mmol, 2.0 equiv) of HOBt, and 16.7 mg (0.0871 mmol, 2.0 equiv) of EDC as described in the general procedure (Method A) with one modification: the aqueous workup was omitted. The reaction mixture was subjected directly to flash chromatography (20 x 150 mm silica gel, 100-mL portions of 1.25%, 2.5%, 3.75% and 5% methanol/dichloromethane) to give 19.2 mg (57%) of the title compound: R_{f} 0.43 (5% methanol/dichloromethane); ¹H NMR (300 MHz, CD₃OD) δ 8.20 (d, J = 8.8 Hz, 1 H), 7.85 (d, J = 7.4 Hz, 1 H), 7.57-7.31 (m, 4 H), 5.16 (br m, 1 H), 4.59 (dd, J = 4.3, 9.8 Hz, 1 H), 4.45 (dt, J = 1.9, 7 Hz, 1 H), 4.24-4.19 (m, 2 H), 4.10 (t, J = 10.1 Hz, 1 H), 3.71-3.17 (m, 15 H), 2.87-2.70 (m, 2 H), 2.59-2.52 (m, 2 H), 2.45-2.32 (m, 6 H), 1.91-0.88 (m, 17 H); MS(FAB) 778 (M+1).

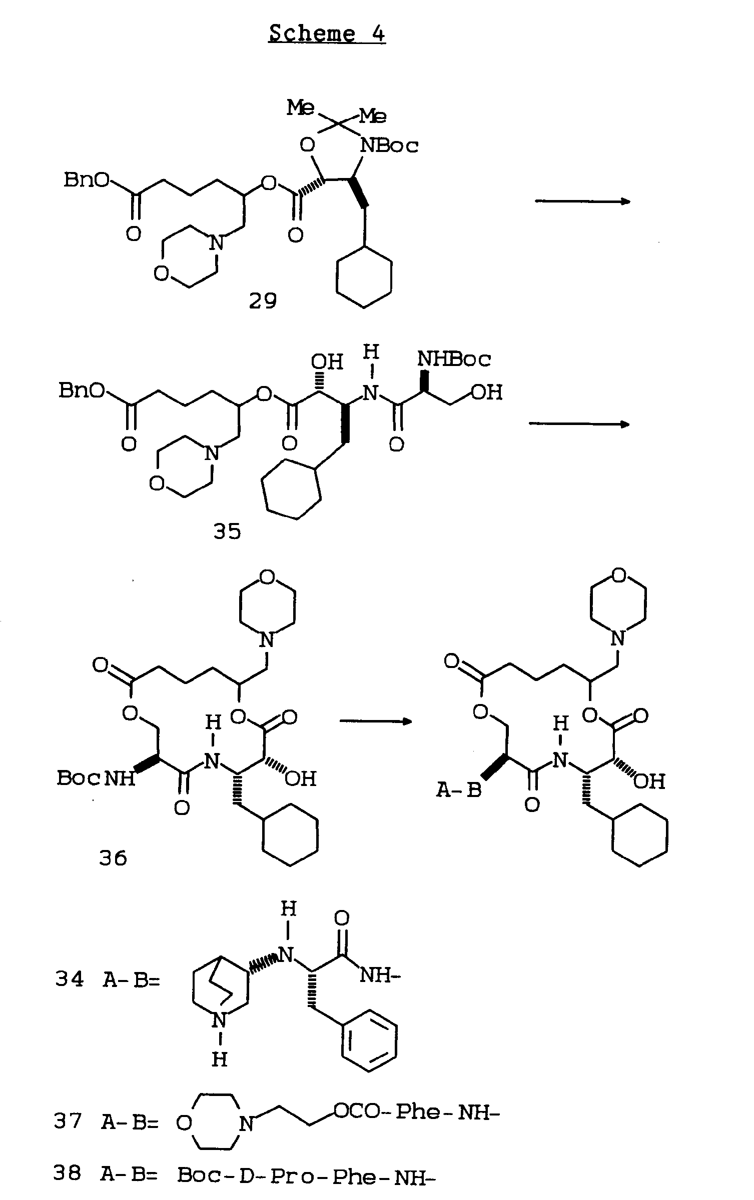

### Serine ester 35

A solution of 750 mg (1.19 mmol) of 29 in 10 mL of 1:1 trifluoroacetic acid/dichloromethane was stirred at room temperature for 1 hour. The solution was concentrated and trace amounts of acid were removed azeotropically with toluene. The resultant oil was dried over P₂O₅/KOH under vacuum for 1 hour and then dissolved in 10 mL of dichloromethane. The solution was cooled to 0°C and treated with 488 mg (2.378 mmol, 2 equiv) of Boc-Ser (alternatively, N-Boc-O-Benzyl-Ser can be used to provide higher yields in this reaction; the benzyl ether is subsequently removed along with the benzyl ester in the next step), 241 mg of triethylamine (0.33 mL, 2.38 mmol, 2 equiv), 364 mg of HOBt (2.38 mmol, 2 equiv), and 456 mg (2.38 mmol, 2 equiv) of EDC. The solution was stirred overnight with gradual warming to room temperature and then diluted with 200 mL of ethyl acetate, washed sequentially with 50-mL portions of saturated aqueous sodium bicarbonate and saturated aqueous sodium chloride, dried over anhydrous magnesium sulfate and concentrated. Purification by flash chromatography (20 X 150 mm silica gel, 2.5% methanol/dichloromethane) followed by MPLC (Lobar B column, 97:3 chloroform to 10:1 methanol/ammonium hydroxide) gave 494 mg (61 %) of the title compound: R_{f} 0.32 (5% methanol/dichloromethane); ¹H NMR (300 MHz, CD₃OD) δ 7.36-7.30 (m, 5 H), 5.06 (m, 1 H), 4.35 (dt, J = 2.9, 7.0 Hz, 1 H), 4.15 (d, J = 3.1 Hz, 1 H), 4.06 (br t, J = 5.2 Hz, 1 H), 3.78-3.61 (m, 6 H), 2.64-2.30 (m, 8 H), 1.46 (s, 9 H), 1.90-0.84 (m, 17 H); MS(FAB) 678 (M+1).

Anal. calcd. for C₃₅H₅₅N₃O₁₀·1/2H₂O: C, 61.21; H, 8.22; N, 6.12. Found: C, 61.56; H, 8.20; N, 6.07.

### Macrocycle 36

A solution of 247 mg (0.365 mmol) of serine ester 35 in ethyl acetate was stirred over 10% Pd/C under 1 atm of hydrogen overnight. The suspension was then filtered and concentrated. The resultant oil was dissolved in 5 mL of tetrahydrofuran and added dropwise over 19 hours to a refluxing solution of 140 mg (0.730 mmol, 2.0 equiv) of EDC, 49.0 mg (0.401 mmol, 1.1 equiv) of DMAP and 116 mg (0.730 mmol, 2.0 equiv) of DMAP hydrochloride in 25 mL of chloroform. After addition was complete, the solution was cooled, diluted with 200 mL of ethyl acetate, washed sequentially with 50-mL portions of saturated aqueous sodium bicarbonate and saturated aqueous sodium chloride, dried over anhydrous magnesium sulfate and concentrated. Purification by flash chromatography (20 X 150 mm silica gel, 2.5% methanol/dichloromethane) gave 159 mg (76%) of the title compound as a white solid: R_{f} 0.31 (5% methanol/dichloromethane); ¹H NMR (300 MHz, CD₃OD) δ 5.16 (m, 1 H), 4.51 (m, 1 H), 4.32 (dd, J = 3.3, 9.7 Hz, 1 H), 4.23 (d, J = 2.0 Hz, 1 H), 4.22-4.06 (m, 2 H), 3.70-3.60 (m, 4 H), 2.73 (dd, J = 9.0,13.1 Hz, 1 H), 2.60-2.55 (m, 2 H), 2.46-2.35 (m, 5 H), 1.91-0.86 (m, 17 H); MS(FAB) 570 (M+1), 514, 470.

Anal. calcd. for C₂₈H₄₇N₃O₉·1/2H₂O C, 58.11; H, 8.36; N, 7.26. Found: C, 58.20; H, 8.45; N, 7.16.

### Macrocycle 34

A 451 mg (0.791 mmol) sample of inhibitor 36 was deprotected and treated with 0.38 mL (352 mg, 3.48 mmol, 4.4 equiv) of N-methyl morpholine, 302 mg (0.870 mmol, 1.1 equiv) of N-quinuclidin-3(S)-yl-phenylalanine, 127 mg (0.831 mmol, 1.05 equiv) of HOBt, and 245 mg (1.19 mmol, 1.5 equiv) of DCC (not EDC) according to the general procedure (Method F). Work-up and purification were performed as before to provide 367 mg (64%) of the title compound.

### Macrocycle 37

A 286 mg (0.502 mmol) sample of inhibitor 36 was deprotected and treated with 405 mg (1.25 mmol, 2.5 equiv) of N-[(2-morpholin-4-yl)ethoxycarbonyl]phenylalanine, 102 mg (0.11 mL, 1.25 mmol, 2.5 equiv) of N-methyl morpholine, 241 mg (1.25 mmol, 2.5 equiv) of EDC, and 192 mg (1.25 mmol, 2.5 equiv) of HOBt according to the general procedure (Method F). The mixture was subjected directly to flash chromatography (20x150 mm silica gel, 80 mL of 5% methanol/dichloromethane and 100 mL of 10% and 20% 10:1 methanol/ammonium hydroxide in chloroform). Further purification by MPLC (Sephadex LH-20, MeOH and then Lobar B silica gel column, 2% 10:1 methanol/ammonium hydroxide in chloroform) provided 270 mg (70%) of the title compound as a white solid: ¹H NMR (300 MHz, CD₃OD) δ 7.33-7.19 (m, 5 H), 5.17 (br m, 1 H), 4.60 (dd, J = 4.5, 8.7 Hz, 1 H), 4.50 (t, J = 7 Hz, 1 H), 4.40 (dd, J = 5.0, 9.0 Hz, 1 H), 4.24-4.09 (m, 5 H), 3.68 (br m, 8 H), 3.11 (dd, J = 5.0, 14 Hz, 1 H), 2.86 (dd, J = 9.1, 14 Hz, 1 H), 2.76 (dd, J = 9.4, 13 Hz, 1 H), 2.64-2.32 (m, 13 H), 1.90-0.86 (m, 17 H); MS(FAB) 774 (M+1).

Anal. calcd. for C₃₉H₅₉N₅O₁₁ 1/2H₂0: C, 59.83; H, 7.72; N, 8.94. Found: C, 59.76; H, 7.46; N, 8.90.

### Macrocycle 38

A 237 mg (0.417 mmol) sample of inhibitor 36 was deprotected and treated with 226 mg (0.625 mmol, 1.5 equiv) of Boc-D-Pro-Phe, 92.7 mg (0.101 mL, 0.926 mmol, 2.2 equiv) of N-methyl morpholine, 160 mg (0.833 mmol, 2.0 equiv) of EDC, and 128 mg (0.833 mmol, 2.0 equiv) of HOBt according to the general procedure (Method F). Following an aqueous workup, the compound was purified by flash chromatography (20x150 mm silica gel, 1.25% to 5% methanol/dichloromethane) to provide 261 mg (77%) of the title compound: R_{f} 0.27 (5% methanol/dichloromethane); ¹H NMR (300 MHz, CD₃OD) δ 7.28-7.16 (m, 5 H), 5.17 (br m, 1 H), 4.78 (br dd, 1/2 H), 4.68 (br m, 1/2 H), 4.61 (br m, 1 H), 4.51 (br m, 1 H), 4.25 (br s, 2 H), 4.11-4.07 (overlapping d's, 2 H), 3.67 (br m, 4 H), 3.44-3.34 (m, 2 H), 3.22 (br dd, 1/2 H), 3.10 (br dd, 1/2 H), 2.88 (br m, 1 H), 2.75 (dd, J = 9.1, 13 Hz, 1 H), 2.59-2.54 (m, 2 H), 2.45-2.32 (m, 5 H), 2.04-0.87 (m, 30 H); MS(FAB) 814 (M+1), 714.

Anal. calcd. for C₄₂H₆₃N₅0₁, 1/5H₂0: C, 61.70; H, 7.82; N, 8.57. Found: C, 61.53; H, 8.04; N, 8.35.

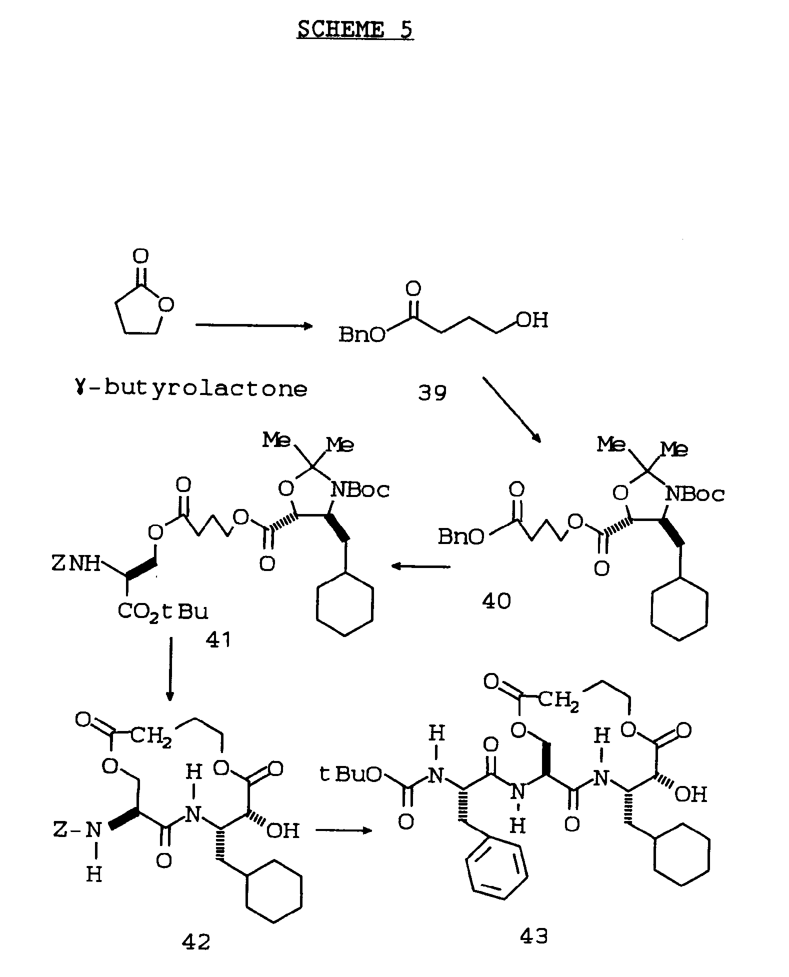

### Benzyl 4-Hydroxybutyrate 39

A mixture of 5.10 g (59.2 mmol) of gamma-butyrolactone and 2.37 g (59.2 mmol) of sodium hydroxide in 59 mL of water was heated at 70°C overnight. The clear solution was then cooled and concentrated. The resultant white solid was suspended in toluene and concentrated to remove trace amounts of water. This solid was suspended in 60 mL of acetone along with 955 mg (2.96 mmol, 0.05 equiv) of tetrabutylammonium bromide and 12.2 g (8.46 mL, 71.1 mmol, 1.2 equiv) of benzyl bromide and heated at reflux for 24 hours. The reaction mixture was cooled and concentrated. The residue was partitioned between 750 mL of ethyl acetate and 250 mL of 1 N aqueous sodium bisulfate. The organic phase was washed with 250-mL portions of saturated aqueous sodium bicarbonate and saturated aqueous sodium chloride, dried over anhydrous magnesium sulfate, and concentrated. Purification by MPLC (Lobar C column, 40% ethyl acetate/hexane) provided 8.94 g (78%) of the title compound as a clear oil: R_{f} 0.21 (40% ethyl acetate/hexane); ¹H NMR (300 MHz, CDCl₃) δ 7.30-7.27 (m, 5 H), 5.16 (s, 2 H), 3.67 (t, 2 H), 2.49 (t, 2 H), 1.95-1.86 (m, 3 H).

### Benzyl ester 40

Boc-NorACHPA acetonide 3 (1370 mg, 4.01 mmol, 1.0 equiv) was coupled with 935 mg (4.82 mmol, 1.2 equiv) of benzyl 5-hydroxybutyrate 39 using 1150 mg (6.02 mmol, 1.5 equiv) of EDC and 49 mg (0.401 mmol, 0.1 equiv) of DMAP in 16 mL of dichloromethane for 4.5 hours according to the general procedure for EDC/DMAP esterification. Purification by MPLC (Lobar C column, 20% ethyl acetate/hexane) gave 1100 mg (96%) of the title compound as an oil: ¹H NMR (300 MHz, CDCl₃) δ 7.39-7.32 (m, 5 H), 5.12 (s, 2 H), 4.32-4.18 (m, 4 H), 2.46 (t, J = 7.4 Hz, 2 H), 2.02 (quint, J = 6.8 Hz, 2 H), 1.89 (br d, J = 13 Hz, 1 H), 1.83-0.94 (m, 12 H), 1.67 (s, 3 H), 1.63 (s, 3 H), 1.60 (s, 9 H); MS(FAB) 518 (M+1), 418.

Anal. calcd. for C₁₉H₄₃NO₇: C, 67.29; H, 8.37; N, 2.71. Found: C, 67.34; H, 8.56; N, 2.70.

### Acyl serine derivative 41

A solution of 303 mg (0.586 mmol) of benzyl ester 40 in ethyl acetate was stirred with 10% palladium on carbon under an atmosphere of hydrogen overnight. The mixture was then filtered through celite and concentrated. The resultant white crystalline solid was dissolved in 5 mL of dichloromethane, cooled to 0°C, and treated with 190 mg (0.644 mmol, 1.1 equiv) of N-carbobenzoxy-L-serine tert-butyl ester, 168 mg (0.879 mmol, 1.5 equiv) of EDC, and 7.2 mg (0.059 mmol, 0.1 equiv) of DMAP according to the general procedure for EDC/DMAP esterification. Purification by MPLC (Lobar B column, 20% ethyl acetate/hexane) provided 404 mg (98%) of the title compound as an oil: R_{f} 0.26 (20% ethyl acetate/hexane); ¹H NMR (300 MHz, CDCl₃) δ 7.37-7.27 (m, 5 H), 5.55 (br d, J = 7.3 Hz, 1 H), 5.12 (s, 2 H), 4.52-4.18 (m, 7 H), 2.38 (t, J = 7.1 Hz, 2 H), 2.03-0.95 (m, 21 H), 1.47 (s, 9 H), 1.45 (s, 9 H); MS(FAB) 705 (M+1), 605, 549.

Anal. calcd. for C₃₇H₅₆N₂O₁₁: C, 63.05; H, 8.01; N, 3.97. Found: C, 62.94; H, 8.28; N, 3.95.

### Macrocycle 42

Macrocyclization of 376 mg (0.533 mmol) of acyl serine derivative 41 was carried out according to the general procedure (Method A) described above. Purification by flash chromatography (20 x 150 mm silica gel, 60% ethyl acetate/hexane) provided 138 mg (53%) of the title compound: R_{f} 0.23 (60% ethyl acetate/hexane); ¹H NMR (300 MHz, CD₃OD) δ 7.43-7.27 (m, 5 H), 5.10 (s, 2 H), 4.47-4.01 (m, 7 H), 2.59 (ddd, J = 2.8, 9.8, 13 Hz, 1 H), 2.40 (ddd, J = 3.1, 7.9, 13 Hz, 1 H), 2.18-1.94 (m, 2 H), 1.83-0.83 (m, 13 H); MS(FAB) 491 (M+1), 447. Anal. calcd. for C₂₅H₃₄N₂O₈: C, 61.21; H, 6.99; N, 5.71. Found: C, 61.05; H, 6.93; N, 5.61.

### Macrocycle 43

A solution of 48.5 mg (0.0988 mmol) of 42 was deprotected in 1:1 methanol/ethyl acetate and treated with 52.5 mg (0.198 mmol, 2.0 equiv) of BocPhe, 30.3 mg (0.198 mmol, 2.0 equiv) of HOBt, and 37.9 mg (0.198 mmol, 2.0 equiv) of EDC as described in the general procedure (Method A). Purification by flash chromatography (20 x 150 mm silica gel, 70% ethyl acetate/hexane) gave 43.4 mg (73%) of the title compound as a white solid: R_{f} 0.48 (75% ethyl acetate/hexane); ¹H NMR (300 MHz, CD₃OD) δ 7.27-7.18 (m, 5 H), 4.65 (dd, J = 3.4, 8.0 Hz, 1 H), 4.42-4.22 (m, 4 H), 4.15 (d, J = 2.5 Hz, 1 H), 4.14-4.00 (m, 2 H), 3.13 (dd, J = 5.1, 13.5 Hz, 1 H), 2.83 (dd, J = 9.5, 13.5 Hz, 1 H), 2.59 (ddd, J = 3.0, 10, 15 Hz, 1 H), 2.41 (ddd, J = 3.0, 8.2, 15 Hz, 1 H), 2.18-1.96 (m, 2 H), 1.83-0.85 (m, 13 H), 1.36 (s, 9 H); MS(FAB) 604 (M+1), 548, 504, 357.

Anal. calcd. for C₃₁H₄₅N₃O₉: C, 61.68; H, 7.51; N, 6.96. Found: C, 61.67; H, 7.67; N, 6.83.

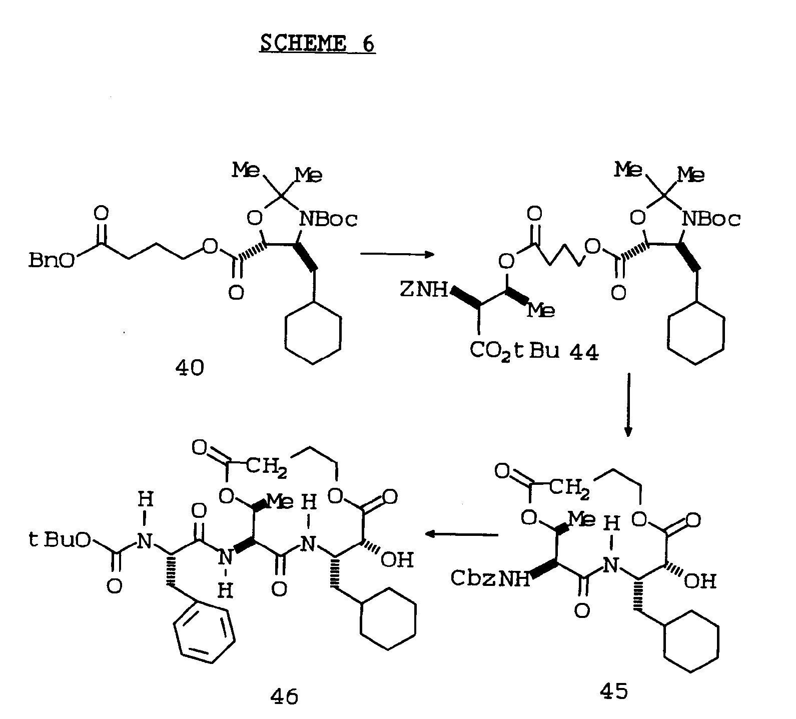

### Acyl allo-threonine derivative 44

A solution of 395 mg (0.762 mmol) of benzyl ester 40 in ethyl acetate was stirred with 10% palladium on carbon under an atmosphere of hydrogen for 24 h. The mixture was then filtered through celite and concentrated. The resultant white crystalline solid was dissolved in 5 mL of dichloromethane, cooled to 0°C, and treated with 235 mg (0.762 mmol, 1.0 equiv) of N-carbobenzoxy-L-allo-threonine tert-butyl ester, 438 mg (2.29 mmol, 3.0 equiv) of EDC, and 4.7 mg (0.038 mmol, 0.05 equiv) of DMAP according to the general procedure for EDC/DMAP esterification. Purification by MPLC (2 Lobar B columns, 15% ethyl acetate/hexane) provided 454 mg (83%) of the title compound as an oil: R_{f} 0.30 (20% ethyl acetate/hexane); ¹H NMR (300 MHz, CDCl₃) δ 7.36-7.26 (m, 5 H), 5.53 (br d, J = 8.3 Hz, 1 H), 5.20 (m, 1 H), 5.10 (s, 2 H), 4.58 (dd, J = 3.0, 8.1 Hz, 1 H), 4.33-4.11 (m, 4 H), 2.50 (br t, J = 7.2 Hz, 2 H), 1.99-0.90 (m, 21 H), 1.48 (s, 9 H), 1.47 (s, 9 H), 1.23 (d, J = 6.7 Hz, 3 H); MS(FAB) 719 (M+1), 619, 563.

Anal. calcd. for C₃₈H₅₈N₂O₁₁: C, 63.49; H, 8.13; N, 3.90. Found: C, 63.31; H, 8.17; N, 4.15.

### Macrocycle 45

Macrocyclization of 428 mg (0.595 mmol) of acyl allo-threonine derivative 44 was carried out according to the general procedure (Method A) described above. purification by flash chromatography (20 x 150 mm silica gel, 1.25% and 2.5% methanol/dichloromethane) followed by MPLC (Sephadex LH-20, MeOH) gave 109 mg (36%) of the title compound: R_{f} 0.50 (5% methanol/dichloromethane); ¹H NMR (300 MHz, CD₃OD) δ 7.40-7.20 (m, 5 H), 5.08 (s, 2 H), 5.00 (m, 1 H), 4.25-4.00 (m, 5 H), 2.65 (ddd, J = 3.5, 11, 15 Hz, 1 H), 2.37 (ddd, J = 4.3, 5.9, 15 Hz, 1 H), 2.12-1.96 (m, 2 H), 1.71-0.78 (m, 13 H), 1.24 (d, J = 6.2 Hz, 3 H); MS(FAB) 505 (M+1). Anal. calcd. for C₂₆H₃₆N₂O₈: C, 61.89; H, 7.19; N, 5.55. Found: C, 62.09; H, 7.21; N, 5.59.

### Macrocycle 46

A solution of 31.0 mg (0.0614 mmol) of 45 was deprotected in 1:1 methanol/tetrahydrofuran and treated with 32.6 mg (0.123 mmol, 2.0 equiv) of Boc-Phe, 18.8 mg (0.123 mmol, 2.0 equiv) of HOBt, and 23.6 mg (0.123 mmol, 2.0 equiv) of EDC as described in the general procedure (Method A). Purification by flash chromatography (20 x 150 mm silica gel, 2.5% methanol/dichloromethane) followed by MPLC (Sephadex LH-20, MeOH) gave 26.7 mg (70%) of the title compound as a white solid: R_{f} 0.13 (2.5% methanol/dichloromethane); ¹H NMR (300 MHz, CD₃OD/CDCl₃) δ 7.31-7.18 (m, 5 H), 5.06 (m, 1 H), 4.53 (d, J = 10.2 Hz, 1 H), 4.32 (dd, J = 5.0, 9.1 Hz, 1 H), 4.26-4.05 (m, 4 H), 3.10 (dd, J = 4.9, 14 Hz, 1 H), 2.81 (dd, J = 10, 14 Hz, 1 H), 2.65 (ddd, J = 3.4, 11, 15 Hz, 1 H), 2.38 (dq, J = 3.7, 15 Hz, 1 H), 2.17-1.94 (m, 2 H), 1.76-0.81 (m, 13 H), 1.35 (s, 9 H), 1.21 (d, J = 6.3 Hz, 3 H); MS(FAB) 618 (M+1), 562, 518, 371.

Anal. calcd. for C₃₂H₄₇N₃0₉: C, 62.22; H, 7.67; N, 6.80. Found: C, 62.02; H, 7.74; N, 6.70.

### SECTION C: PREPARATION OF MACROCYCLIC RENIN INHIBITORS OF FORMULA I where W = -NH-, Z = -OH, and Y = -CH₂CH(OH)-:

Scheme 7 illustrates the preparation of macrocyclic renin inhibitors of Formula I in which W = -NH-, Z = -OH, and Y = -CH₂CH(OH)-. As shown in the scheme, after the macrocyclization, the Boc protecting group may be removed from the ketal-containing macrocycle and the resulting amino derivative may be acylated with Boc- Phe affording 56. Hydrolysis of the ketal and reduction of the resulting ketone 57 provides diol 58. Other carboxylic acids, acid chlorides or sulfonyl chlorides may be substituted for Boc-Phe in this scheme to prepare other diol-containing inhibitors.

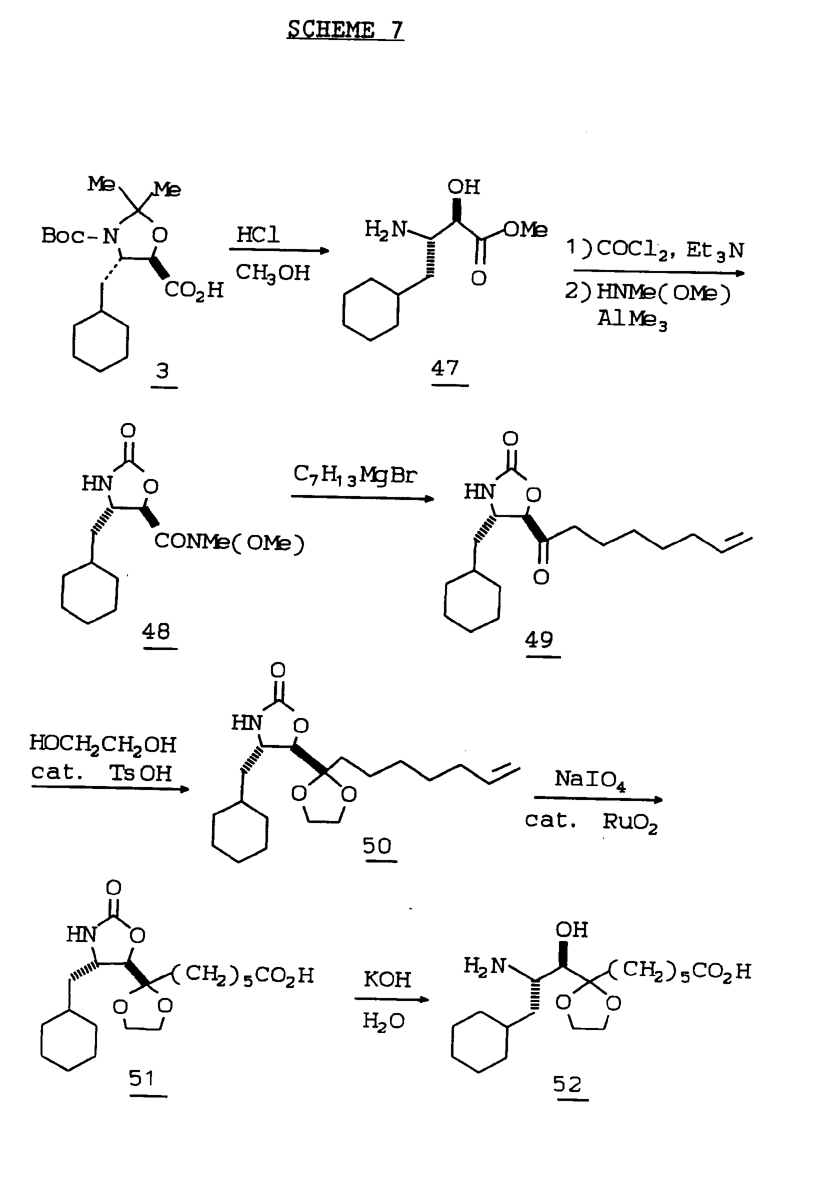

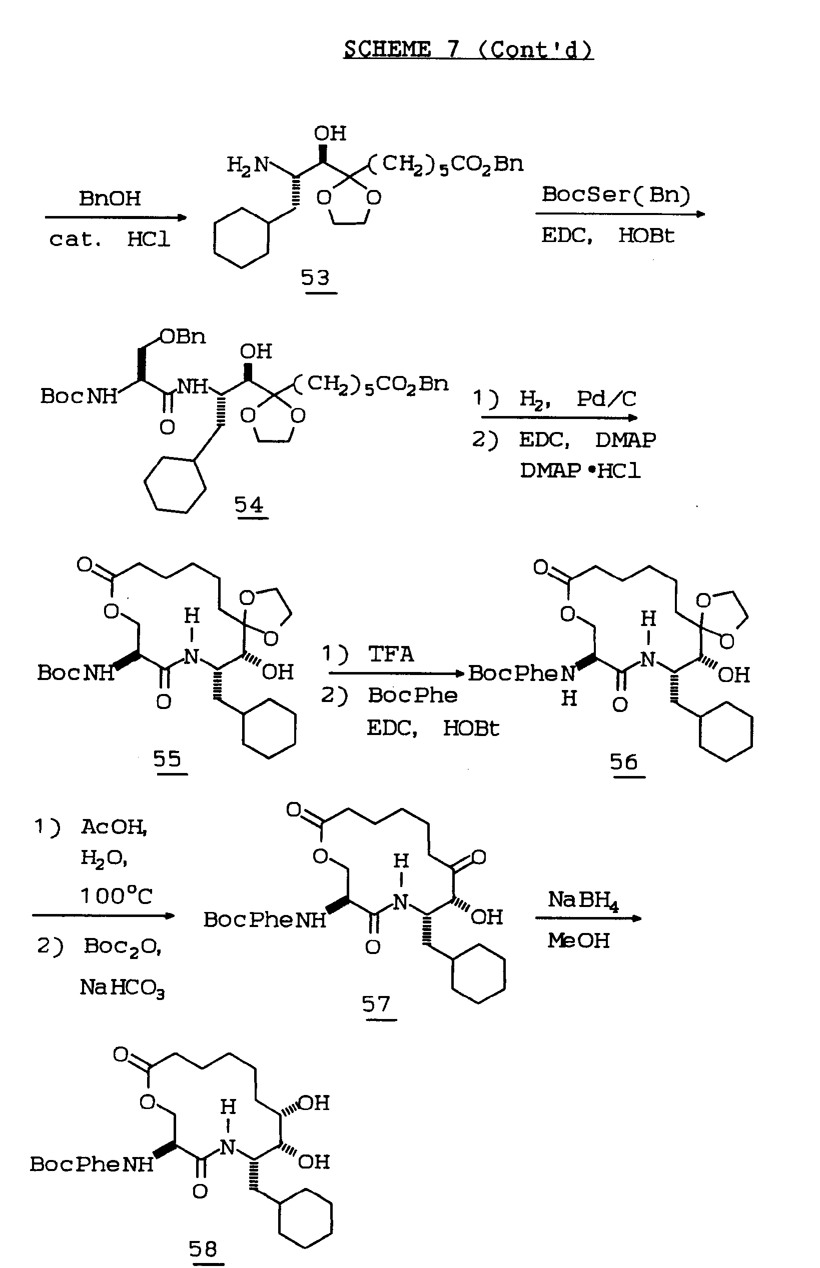

### SECTION D: PREPARATION OF MACROCYCLIC RENIN INHIBITORS OF FORMULA I where W = -NCH₃-, Z = -OH, and Y = -OCO-:

Scheme 8 illustrates the preparation of macrocyclic renin inhibitors of Formula I in which W = -NCH₃-, Z = -OH, and Y = -OCO-. After the macrocyclization shown in the scheme, the Boc protecting group may be removed from 63, and the resulting amino derivative may be acylated with carboxylic acids, acid chlorides or sulfonyl chlorides as described in Method F above.

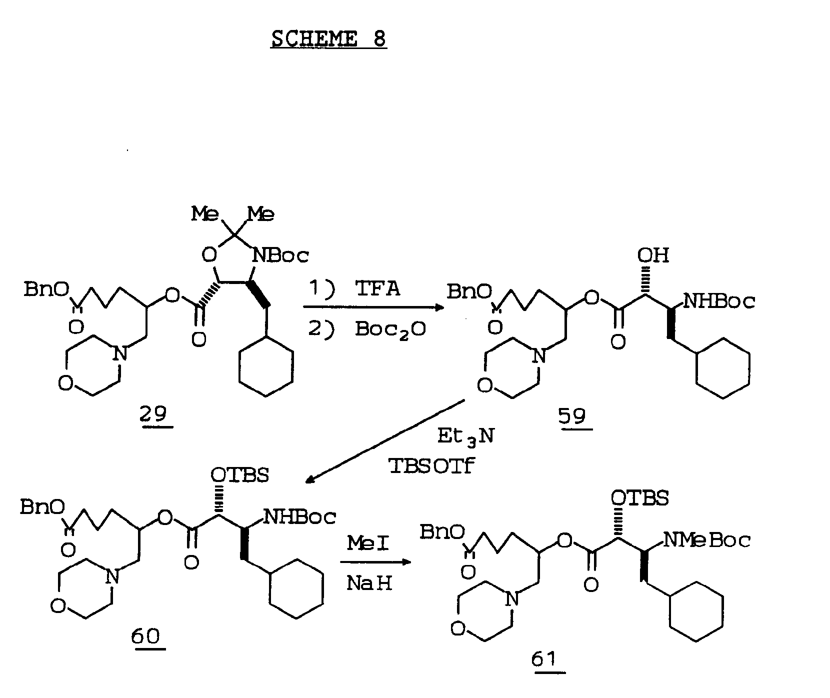

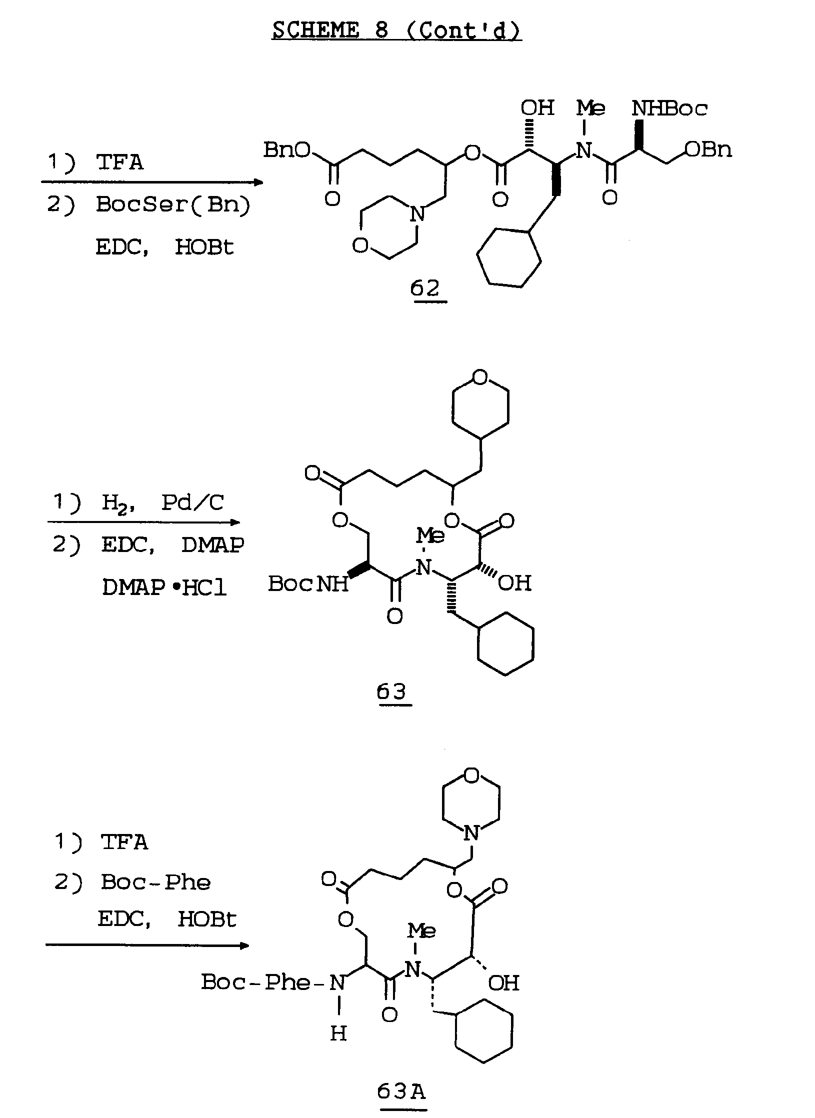

### SECTION E: PREPARATION OF MACROCYCLIC RENIN INHIBITORS OF FORMULA I where W = -NH-, Z = -OCOR²², and Y = -OCO-:

Schemes 9 and 10 illustrate the preparation of macrocyclic renin inhibitors of Formula I in which W = -NH-, Z = -OCOR²², and Y = -OCO-. As shown in Scheme 9, Macrocycle 31 was treated with acetic anhydride and DMAP, yielding the acetate analog 71. The Cbz protecting group was removed from the macrocycle and the resulting amino derivative was coupled with Na-(quinuclidin-3-yl)Phe (5S), affording macrocycle 72. The amino derivative prepared from 71 may likewise be acylated with carboxylic acids, acid chlorides or sulfonyl chlorides as described in Methods D and E above.

In a similar fashion, other macrocycles described herein may be acylated with carboxylic acid anhydrides or acid chlorides to provide similar ester analogs. In cases where functional groups are present in the R²² element, it may be necessary for these to be protected with protecting groups during the acylation step. The protecting groups are chosen so as to be stable to the conditions used to remove the Cbz protecting group (see above), and are then removed after the coupling step which follows.

In cases where the hydroxyl group of a macrocycle is to be esterified with the carboxyl acid moiety of an amino acid, Method G below is used, as illustrated in Scheme 10.

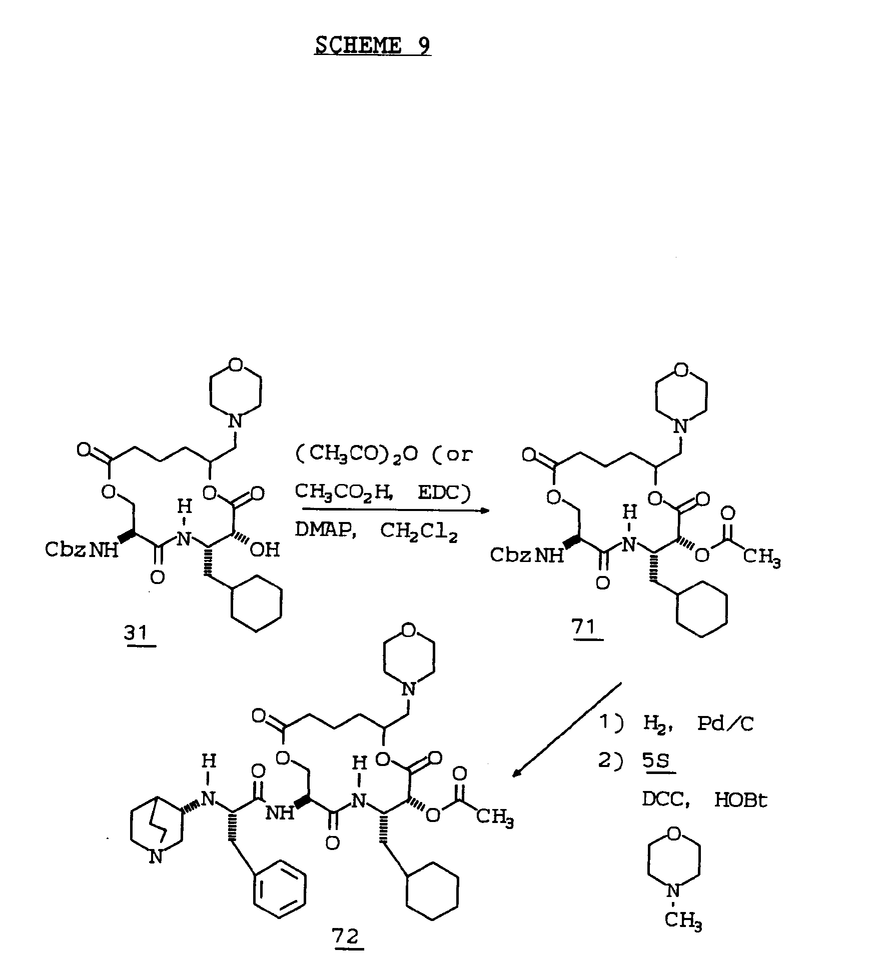

### Macrocycle 71

To a 0°C solution of 145 mg (0.242 mmol) of inhibitor 31 in 2 mL of dichloromethane was added 29.6 mg (0.027 mL, 0.290 mmol, 1.2 equiv) of acetic anhydride and 35.4 mg (0.290 mmol, 1.2 equiv) of dimethylaminopyridine. After the reaction mixture was stirred for 0.5 hours, it was subjected directly to flash chromatography (20x150 mm silica gel, 40-mL portions of 1, 2, 3, 4, and 5% methanol/chloroform) to provide 62 mg (40%) of the title compound: R_{f} 0.50 (5% methanol/dichloromethane); ¹H NMR (300 MHz, CD₃OD) δ 7.32-7.26 (m, 5 H), 5.18 (d, J = 1.9 Hz, 1 H), 5.09 (br s, 3 H), 4.71 (m, 1 H), 4.44 (t, J = 6.9 Hz, 1 H), 4.18 (br d, 2 H), 3.64 (br t, J = 4.2 Hz, 4 H), 2.54-2.23 (m, 8 H), 2.18 (s, 3 H), 1.80-0.83 (m, 17 H); MS(FAB) 646 (M+1).

Anal. calcd. for C₃₃H₄₇N₃0₁₀ 1/2H₂0: C, 60.54; H, 7.39; N, 6.42. Found: C, 60.24; H, 7.45; N, 6.25.

### Macrocycle 72

A 56.7 mg (0.0878 mmol) sample of inhibitor71 was deprotected according to the general procedure (Method A) and treated with 0.023 mL (21.3 mg, 0.211 mmol, 2.4 equiv) of N-methyl morpholine, 36.6 mg (0.105 mmol, 1.2 equiv) of N-(3-quinuclidinyl)-phenylalanine, 16.1 mg (0.105 mmol, 1.2 equiv) of HOBt, and 27.2 mg (0.132 mmol, 1.5 equiv) of DCC (not EDC). The solution was stirred overnight with gradual warming to room temperature. The reaction mixture was submitted directly to flash chromatography (20 x 150 mm silica gel, 100 mL of 5%, 10%, and 15% 10:1 methanol/ammonium hydroxide in chloroform) and purified further by MPLC (Lobar A column, 140/7.5 chloroform to 10:1 methanol/ammonium hydroxide) to give 20.4 mg (40%) of the title compound: ¹H NMR (300 MHz, CD₃OD) δ 7.33-7.20 (m, 5 H), 5.24 (d, J = 1.9 Hz, 1 H), 5.09 (br m, 1 H), 4.71-4.65 (m, 2 H), 4.21-4.10 (m, 2 H), 3.64 (br m, 4 H), 3.36 (m, 1 H), 3.06 (dd, J = 5.0, 13.5 Hz, 1 H), 2.93-2.32 (m, 16 H), 2.19 (s, 3 H), 2.19-2.10 (m, 1 H), 1.94-0.89 (m, 21 H); MS(FAB) 768 (M+1).

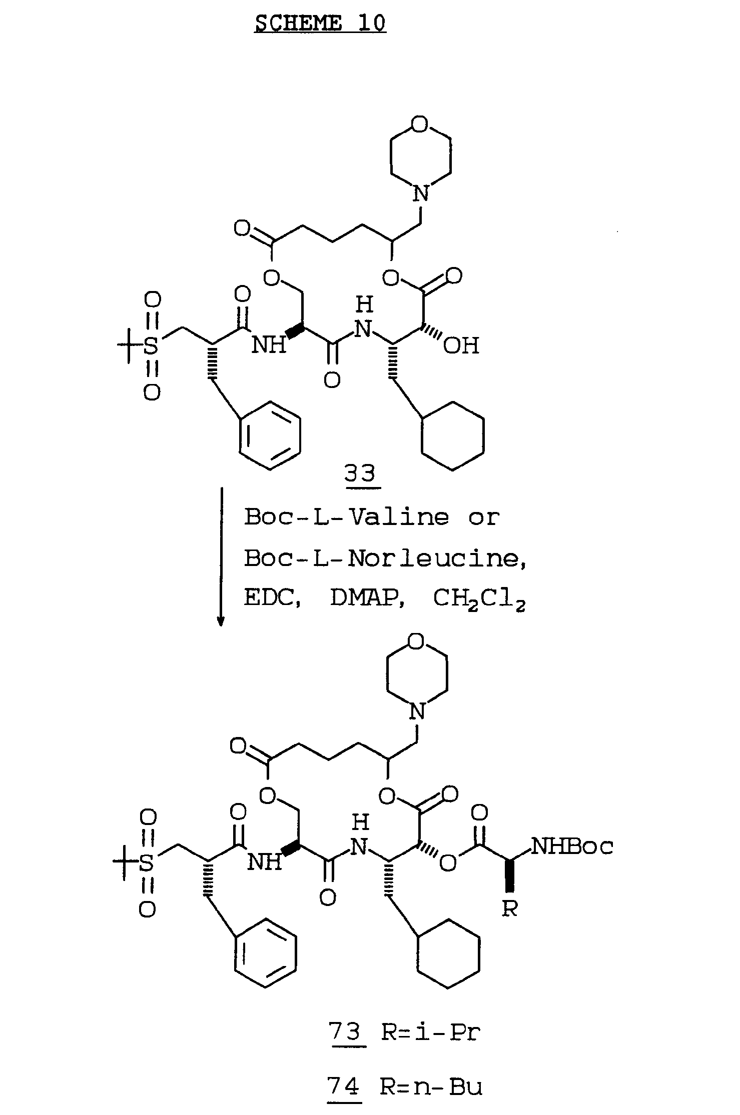

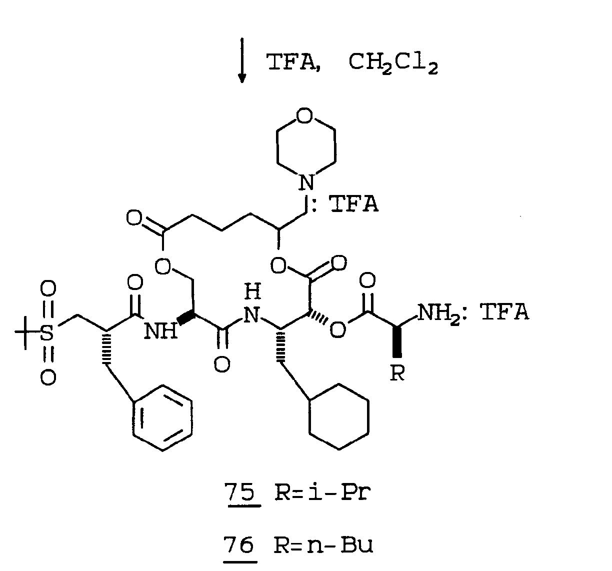

### GENERAL PROCEDURE FOR THE PREPARATION OF ESTERS OF MACROCYCLE 33: METHOD G

To a stirred dichloromethane solution of 33 the N-Boc derivative of the appropriate amino acid (Boc-AA), EDC (2 equiv) and DMAP (0.25 equiv) were added and the mixture stirred overnight. The reaction mixture was diluted with a mixture of ethyl acetate, dichloromethane and ether (3: 1: 1) and washed with saturated aqueous solution of sodium bicarbonate, and brine. The organic phase was dried over anhydrous magnesium sulfate, then filtered and concentrated to an oil. Purification of the crude oil by flash column chromatography (gradient methanol (2-5%) in dichloromethane) afforded Boc-AA ester 73 and 74. The Boc derivatives were then treated with TFA in dichloromethane to give, after purification, the macrocycles 75 and 76 as their TFA salts.

### Macrocycle 73:

¹H NMR (300 MHz, CDCl₃/CD₃OD) 8: 8.25 (d, 1H, NH), 7.37-7.175 (m, 5H), 6.45 (d, 1H, NH), 6.025 (d, 1 H, NH), 5.275 (d), 5.25-5.1 (m), 4.86-4.73 (m), 4.73-4.5 (m), 4.325-4.075 (m), 3.73-3.6 (m), 2.6-3.47 (m), 3.38-3.2 (m), 3.13-3.02 (m), 3.0-2.73 (m), 2.575-2.28 (m), 1.85-1.55 (m), 1.48 (s, 9H), 1.45 (S, 9H), 1.43-1.08(m), 1.3(d, 6H), 1.08-0.84(m); MS(FAB) 935 (M+1).

### Macrocycle 74:

¹H NMR (300MHz, CDCl₃) 8: 7.5 (d, 1H, NH), 7.42-7.13(m, 5H), 6.52 (d, 1H, NH), 6.33 (d, 1H, NH), 5.3-5.1 (m), 4.75-4.63 (m), 4.6-4.5 (m), 4.5-4.22 (m), 4.17-4.03 (m), 3.66 (br s), 3.5 (dd), 3.23-3.125 (m), 3.125-3.02 (m), 2.98-2.83 (m), 2.74-2.57 (m), 2.55-2.18 (m), 1.98-1.58 (m), 1.47 (S, 9H), 1.365 (s, 9H), 1.45-1.06 (m), 1.05-0.8 (m); MS(FAB) 949 (M +1).

### Macrocycle 75:

¹H NMR (300 MHz, CD₃OD) 8: 7.4-7.2 (m, 5H), 5.5(d), 5.48-5.38 *m), 4.73-4.63 *m), 4.34 (m), 4.2 (m), 4.0-3.83 *m, 3.7-3.5 *m), 3.48-3.2 (m), 3.15-2.82 *m), 2.82-2.66 *m), 2.53-2.2 (m), 1.9-1.55 (m), 1.48-1.15 (m), 1.48-1.15 (m), 1.32 (d, 6H), 1.3(S, 9H), 1.15-0.85 (m); MS(FAB) 835 (M +1).

### Macrocycle 76:

¹H NMR (300 MHz, CD₃OD) 8: 7.4-7.1 (m), 5H), 5.49-5.325 (m, 2H), 5.0-4.6 (m), 4.4-4.3 (m, 1 H), 4.3-4.1 (m, 1H), 4.0-3.8 (m, 3H), 3.7-3.45 (m, 2H), 3.366-3.05 (m, 4H), 2.925 (dd, 1H), 2.275 (dd, 1H), 2.53-2.25 (m), 2.177-2.03 (m, 1H), 1.9-1.55 (m), 1.55-.07 (m), 1.32 (s, 9H), 1.06-0.8 (m); MS (FAB) 849 (M+1).

### SECTION F: PREPARATION OF MACROCYCLIC RENIN INHIBITORS OF FORMULA I where W = -NH-, Z = -OH, Y = -OCO-, and A-B is a substitued lactam:

Scheme 11 illustrates the preparation of macrocyclic renin inhibitors of Formula I in which W = -NH-, Z = -OH, Y = -OCO-, and A-B is a substituted lactam moiety. The amino analog 36A resulting from removal of the Boc protecting group from macrocycle 36 is reductively alkylated with aldehyde 78 (prepared by hydrolysis of acetal 77), affording 79. Treatment of 79 with LiOOH, and cyclization of the resulting amino acid intermediate then affords the lactam product 80. The preparation of macrocycles 81, 82 and 83 is then carried out as shown. The amino derivative 81 may likewise be acylated with other carboxylic acids, acid chlorides or sulfonyl chlorides as described in Methods D and E above.

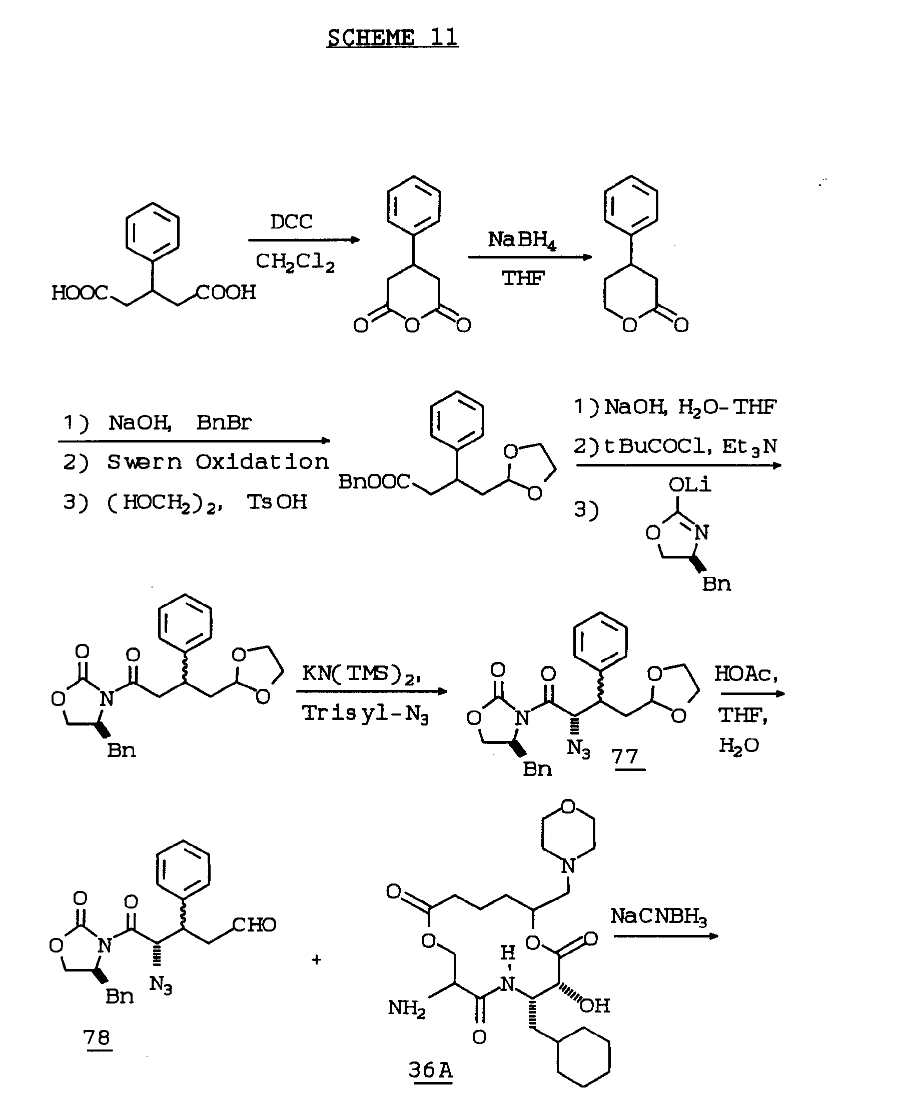

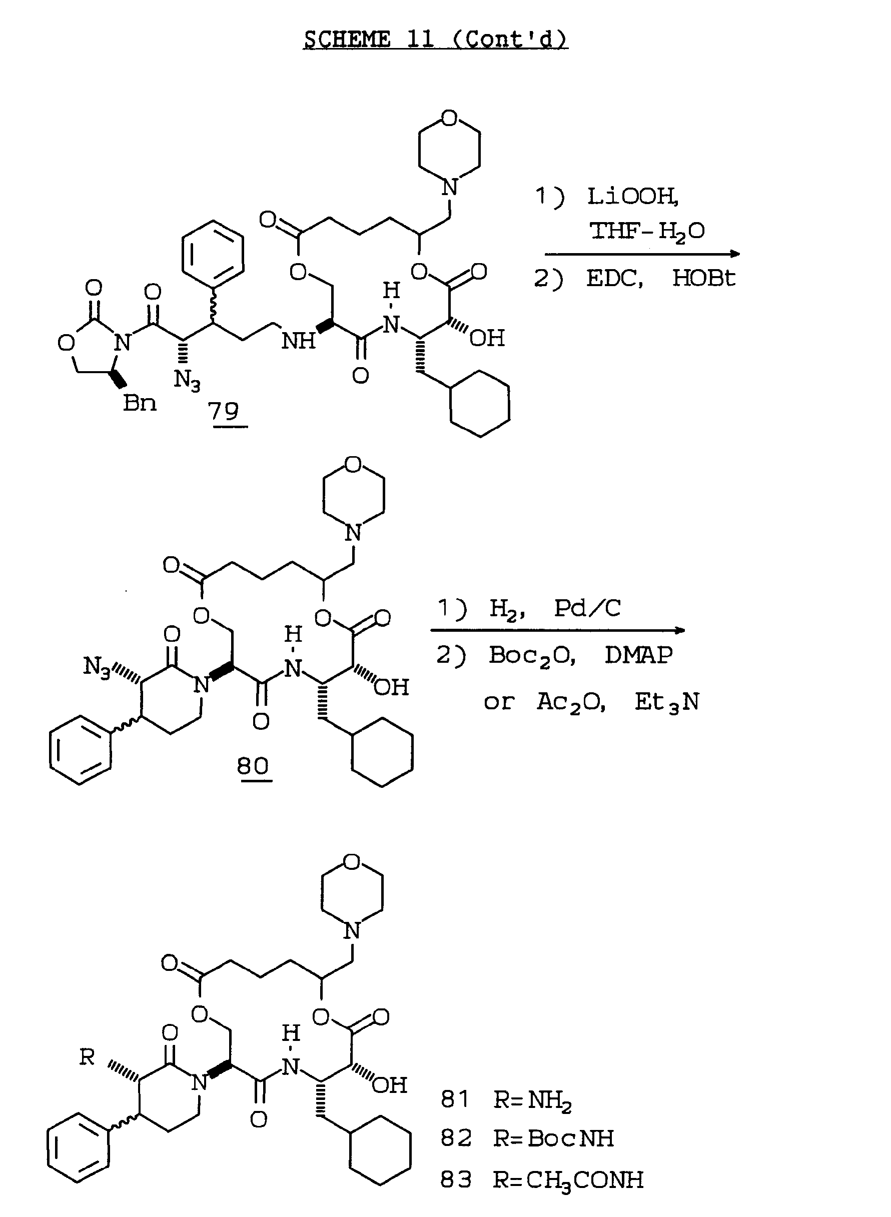

### SECTION G: PREPARATION OF MACROCYCLIC RENIN INHIBITORS OF FORMULA I where W = -NH-, Z = -OH, Y = -OCO-, and A-B is a fused lactam:

Scheme 12 illustrates the preparation of macrocyclic renin inhibitors of Formula I in which W = -NH-, Z = -OH, Y = -OCO-, and A-B is a fused lactam. Indole 86 is prepared as shown and used to reductively alkylate macrocycle 36A (prepared from macrocycle 36 by treatment with TFA). The resulting macrocycle 87 is treated as shown in Scheme 12 to afford macrocycle 88.

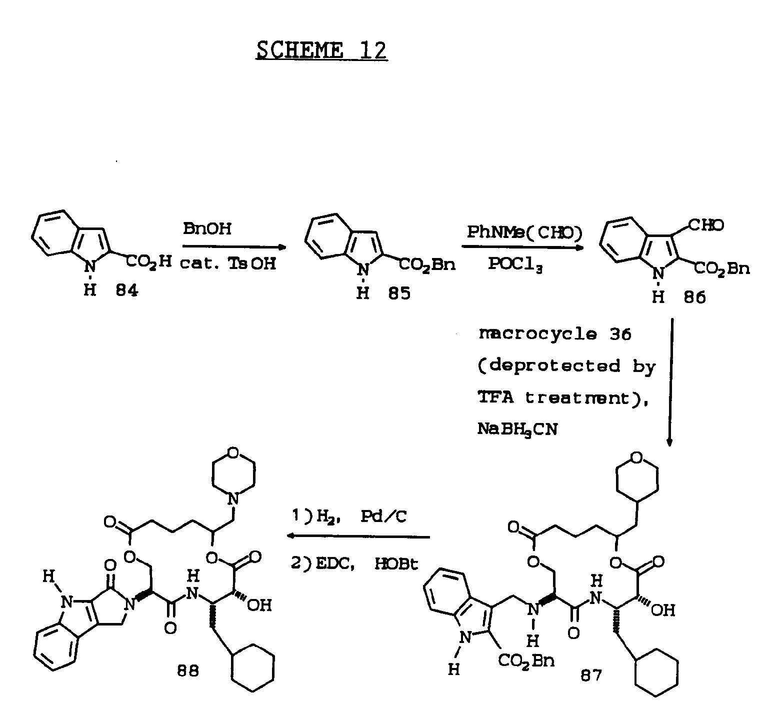

### Benzyl Ester 85.

A solution of 888 mg (5.51 mmol) of indole 84, 655 mg (0.63 mL, 6.06 mmol, 1.1 equiv) of benzyl alcohol, and 52 mg (0.275 mmol, 0.05 equiv) of tosic acid in 11 mL of toluene was heated at reflux overnight with removal of water through a Dean-Stark trap. An additional 655 mg (0.63 mL, 6.06 mmol, 1.1 equiv) of benzyl alcohol was added and the solution was heated at reflux for an additional 24 hours. It was then cooled, diluted with 300 mL of ethyl acetate, and washed with 75-mL portions of saturated aqueous sodium bicarbonate and saturated aqueous sodium chloride. The organic phase was dried over anhydrous magnesium sulfate and concentrated. Purification by flash chromatography (40x150 mm silica gel, 15% ethyl acetate/hexane) provided 882 mg (64%) of the title compound: ¹H NMR (300 MHz, CD₃OD) δ 7.79-7.04 (m, 10 H, 5.37 (s,2H).

### Aldehyde 86.

A solution of 198 mg (0.787 mmol) of benzyl ester 85, 160 mg (0.15 mL, 1.18 mmol, 1.5 equiv) of N-me- thylformanalide, and 181 mg (0.11 mL, 1.18 mmol, 1.5 equiv) of phosphorus oxychloride in 0.5 mL of orthodi- chlorobenzene was heated at 100°Cfor6 h. It was then cooled, diluted with 250 mL of ethyl acetate, and washed with 50-mL portions of saturated aqueous sodium bicarbonate and saturated aqueous sodium chloride. The organic phase was dried over anhydrous magnesium sulfate and concentrated. Purification by flash chromatography (35x150 mm silica gel, 15% ethyl acetate/hexane) gave 62 mg (28%) of the title compound: ¹H NMR (300 MHz,CDCl₃) δ 10.75 (s,1H), 9.41 (br s,1H), 8.47 (d, J=7.8Hz, 1H), 7.53-7.20 (m,8H), 5.48 (s,2H); MS(FAB)280(M+1).

### Macrocycle 87.

Macrocycle 36 (46 mg, 0.0811 mmol) was deprotected by treatment with 2 mL of 1:1 TFA/dichloromethane for 1 hour according to the general procedure (Method F) and the resulting macrocycle 36A was then dissolved in 0.4 mL of anhydrous methanol. To this solution was added 23 mg (0.0811 mmol, 1.0 equiv) of indole 86 and ground 4 A sieves. A solution of 5.1 mg (0.0811 mmol, 1.0 equiv) of sodium cyanoborohydride in 0.20 mL of THF was added dropwise over 7 hours. The resultant mixture was stirred at room temperature for 2 days. It was then diluted with 200 mL of ethyl acetate, and washed with 50-mL portions of saturated aqueous sodium bicarbonate and saturated aqueous sodium chloride. The organic phase was dried over anhydrous magnesium sulfate and concentrated. Purification by flash chromatography (20x150 mm silica gel, 150 mL of 1.25,2.5,3.75% methanol/dichloromethane) gave 30 mg (51%) of the title compound: ¹H NMR (300 MHz, CD₃OD) 87.76-7.08 (m,9H), 5.43 (s,2H), 5.16 (m, 1H), 4.50 (m,1H), 4.32-4.21 (m,3H), 3.90 (t, J=1 OHz, 1 H), 3.65 (m,4H), 3.51 (dd, J=4.2, 9.5Hz, 1H), 2.71 (dd, J=9.1, 13.1Hz, 1H), 2.58-2.30 (m,7H), 1.89-0.80 (m,17H); MS(FAB)733(M+1),470.

### Macrocycle 88.

A solution of 26 mg (0.0351 mmol) of macrocycle 87 in 1 mL of THF was treated with 50 mg of 10% Pd/C under an atmosphere of hydrogen for 3 hours. The mixture was then filtered and concentrated. The residue was dissolved in 1 mL of dichloromethane and cooled to 0°C. To this solution was added 10.7 mg (0.701 mmol, 2.0 equiv) of HOBt and 13.4 mg (0.701 mmol, 2.0 equiv) of EDC. The solution was stirred overnight with gradual warming to room temperature. It was then diluted with 200 mL of ethyl acetate, and washed with 50-mL portions of saturated aqueous sodium bicarbonate and saturated aqueous sodium chloride. The organic phase was dried over anhydrous magnesium sulfate and concentrated. Purification by flash chromatography (20x150 mm silica gel, 100 mL of 1.25,2.5,3.75,5% methanol/dichloromethane) gave 9.7 mg (44%) of the title compound: ¹H NMR (300 MHz,CD₃OD) δ 7.65 (d, J=7.9Hz, 1 H), 7.50 (d, J=8.3Hz, 1 H), 7.29 (dt, J=1.2, 8.3Hz, 1 H), 7.14 (dd, J=6.3, 7.8Hz, 1 H), 5.28 (m,1H), 5.11 (dd, J=4.0, 11.0Hz, 1 H), 4.77 (t, J=10.9Hz, 1 H), 4.65-4.52 (m,3H), 4.25-4.19 (m,2H), 3.67 (m,4H), 2.76 (dd, J=9.2, 13.3Hz, 1H), 2.60-2.28 (m,7H), 1.85-0.54, m, 17H); MS(FAB)625(M+1).

### SECTION H: PREPARATION OF MACROCYCLIC RENIN INHIBITORS OF FORMULA I where W = -NH-, Z = -OH, Y = -OCO-, and R¹⁶incorporates a quaternary ammonium group:

Scheme 13 illustrates the preparation of macrocyclic renin inhibitors of Formula I in which W = -NH-, Z = -OH, Y = -OCO-, and the R¹⁶ substituent incorporates a quaternary ammonium group. The macrocycle containing a tertiary amino group in the R¹⁶ substituent is treated with an alkyl halide such as methyl iodide. Ion exchange chromatography may then be used convert the resulting product to the chloride salt. In some cases it is useful to protect the hydroxyl group of the macrocycle as a silyl ether prior to the quaternization step. This protecting group is then removed after the quaternization step.

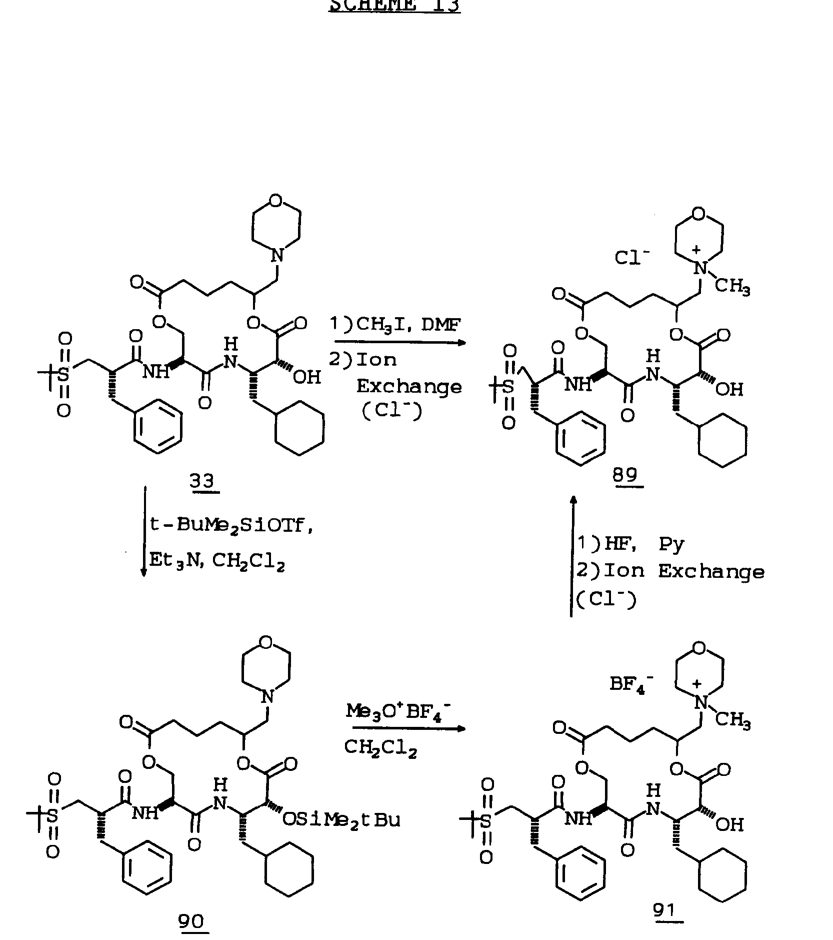

### Macrocyclic Inhibitor 89

Macrocycle 33 is treated with excess of iodomethane in DMF. Purification followed by ion exchange of the iodide with the chloride ion affords the quarternized inhibitor 89.

### Macrocycle 90

To a dichloromethane solution of 33 is added triethylamine and t-butyldimethylsilyl trifluoromethanesulfonate (t-BuMe₂SiOTf) and the mixture stirred for a few hours. Upon completion of silylation, the reaction mixture is poured into a mixture of ethyl acetate-dichloromethane-ether (3:1:1) and washed with water and saturated aqueous solution of sodium chloride. Purification of the crude material by flash column chromatography provides the silylated macrocycle 90.

### Macrocycle 91

Macrocyclic t-butyldimethylsilyl ether 85 is treated with trimethyloxonium tetrafluoroborate in dichloromethane to give, after purification, the quarternized tetrafluoroborate salt 91.

### Macrocyclic Inhibitor 89

t-Butyldimethylsilyl group of the macrocycle 91 is removed by treating it with hydrofluoric acid in pyridine and the resulting tetrafluoroborate salt is then ion exchanged with chloride to give the quarternized inhibitor 89.

### SECTION I: PREPARATION OF MACROCYCLIC RENIN INHIBITORS OF FORMULA I where W=-NH-,Z=-OH,Y=-OCO-, and R¹¹=methyl:

Scheme 14 illustrates the preparation of macrocyclic renin inhibitors of the Formula I where W = -NH-, Z = -OH, Y = -OCO-, and R¹¹ = methyl. As shown in the scheme, intermediate 29 is coupled with an N-Me serine derivative to provide 92. Deprotection and cyclization gives macrocycle 93. The Boc protecting group of 93 is removed, and the resulting amino acid derivative is acylated with a carboxylic acid (as described in Method F) to give inhibitors such as 94, or with an acid chloride or a sulfonyl chloride using standard procedures. Macrocyclic inhibitors with R¹¹ = alkyl other than methyl are available from the appropriate N-alkyl serine derivative.

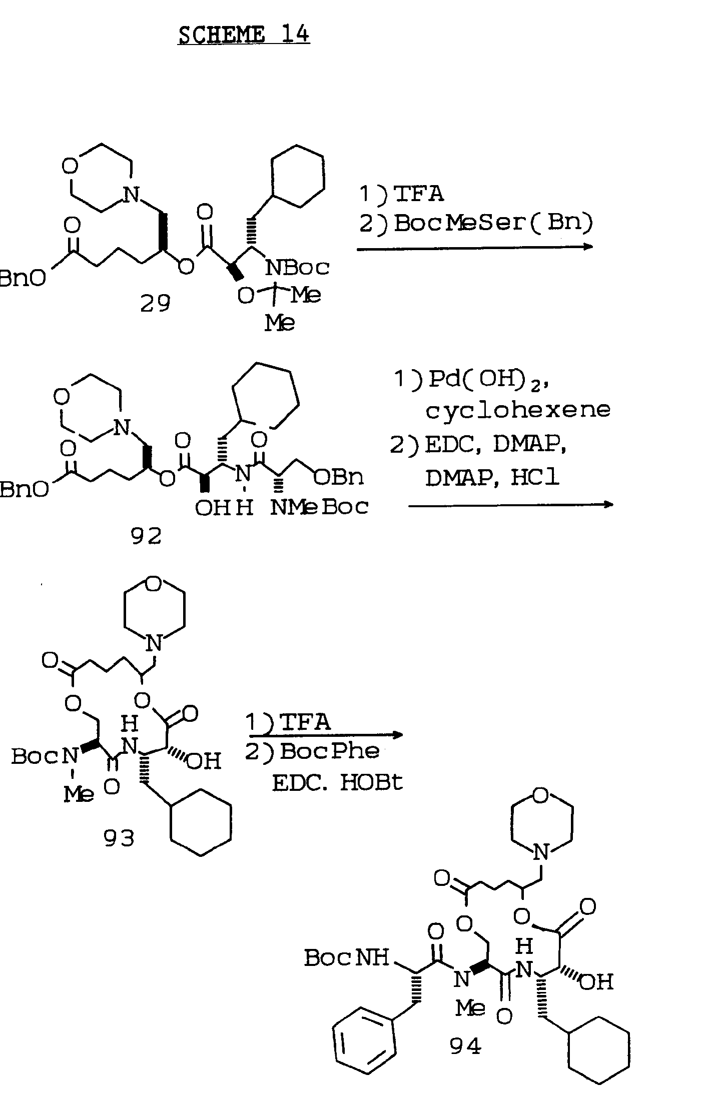

### N-Me Serine Ester 92.

A solution of 1060 mg (1.68 mmol) of benzyl ester 29 in 20 mL of 1:1 trifluoroacetic acid/dichloromethane was stirred at room temperature for 5 hours. The solution was concentrated and trace amounts of acid were removed azeotropically with toluene. The resultant oil was dried over P₂O₅/KOH under vacuum overnight. Meanwhile, 825 mg (2.02 mmol, 1.2 equiv) of N-Me-N-Boc-O-BenzyISer cyclohexylamine salt was dissolved in 300 mL of dichloromethane, washed with 1 N aqueous sodium bisulfate solution, dried over anhydrous magnesium sulfate, and concentrated. This compound was added to a solution of deprotected ester in 10 mL of dichloromethane. After cooling the mixture to 0°C, 374 mg (0.41 mL, 3.70 mmol, 2.2 equiv) of N-methylmorpholine, 515 mg (3.36 mmol, 2.0 equiv) of HOBt, and 645 mg (3.36 mmol, 2.0 equiv) of EDC were added. The solution was stirred overnight with gradual warming to room temperature and then diluted with 500 mL of ethyl acetate, washed sequentially with saturated aqueous sodium bicarbonate and saturated aqueous sodium chloride, dried over anhydrous magnesium sulfate and concentrated. Purification by MPLC (Lobar B column, 40% ethyl acetate/hexane) gave 330 mg (25%) of the title compound: ¹H NMR (300 MHz,CD₃OD) δ 7.36-7.25 (m,10H), 5.11 (s,2H), 5.02 (m,1H), 4.82 -4.65 (m, 1 H), 4.58 (d, J = 11.7 Hz, 1 H), 4.50 (d, J = 11.7 Hz, 1 H), 4.36 (m, 1 H), 4.15 (d, J = 2.8 Hz, 1 H), 3.83 (dd, J = 5.8, 10.4 Hz, 1 H), 3.74 (dd, J = 8.4, 10.4 Hz, 1 H), 3.62 (br t, J = 4.5 Hz, 4 H), 2.84 (s, 3 H), 2.44-2.31 (m, 8 H), 1.87-0.88 (m, 17 H), 1.47 (s, 9 H); MS(FAB) 782 (M+1), 682. Anal. calcd. for C₄₃H₆₃N₃O₁₀·1/4H₂O: C, 65.66; H, 8.14; N, 5.34. Found: C, 65.35; H, 8.18; N, 5.29.

### Macrocycle 93.

A solution of 301 mg (0.385 mmol) of serine ester 92 in 4 mL of 2:1 ethanol/cyclohexene was stirred over 20% Pd(OH)₂/C at reflux temperature for 6 hours. TLC indicated benzyl ether remained so the mixture was cooled, filtered, concentrated, and resubjected to the reaction conditions for 5 hours. The suspension was then filtered and concentrated. The resultant oil was dissolved in 5 mL of tetrahydrofuran and added dropwise over 19 hours to a refluxing solution of 148 mg (0.770 mmol, 2.0 equiv) of EDC, 41.7 mg (0.424 mmol, 1.1 equiv) of DMAP and 122 mg (0.770 mmol, 2.0 equiv) of DMAP hydrochloride in 25 mL of chloroform. After addition was complete, the solution was cooled, diluted with 300 mL of ethyl acetate, washed sequentially with 100-mL portions of saturated aqueous sodium bicarbonate and saturated aqueous sodium chloride, dried over anhydrous magnesium sulfate and concentrated. Purification by flash chromatography (20 x 150 mm silica gel, 1.25, 2.5, and 3.75% methanol/dichloromethane) followed by MPLC (30 mm x 2 m LH-20, methanol) gave 145 mg (65%) of the title compound as a white solid: ¹H NMR (300 MHz, CD₃OD) δ 5.23 (m, 1 H), 4.77 (m, 1 H), 4.61-4.51 (m, 2 H), 4.21 (s, 1 H), 4.07 (m, 1 H), 3.72-3.62 (m, 4 H), 2.85 (s, 3 H), 2.74 (dd, J = 9.3, 13.1 Hz, 1 H), 2.59-2.24 (m, 7 H), 1.89-0.80 (m, 17 H), 1.50 (s, 9 H); MS(FAB) 584 (M+1), 484.

Anal. calcd. for C₂₉H₄₉N₃0₉: C, 59.67; H, 8.46; N, 7.20. Found: C, 59.50; H, 8.50; N, 7.11.

### Macrocycle 94.

A 20.3 mg (0.0348 mmol) sample of macrocycle 93 was deprotected and treated with 27.7 mg (0.104 mmol, 3 equiv) of BocPhe, 7.74 mg (0.0084 mL, 0.0765 mmol, 2.2 equiv) of N-methyl morpholine, 20.0 mg (0.104 mmol, 3 equiv) of EDC, and 16.0 mg (0.104 mmol, 3 equiv) of HOBt according to the general procedure (Method F). After an aqueous work-up, the reaction mixture was purified by flash chromatography (20x150 mm silica gel, 100 mL of 1.75, 2.5, 3.75% methanol/dichloromethane). Further purification by flash chromatography (20x150 mm silica gel, 30 mL of ethyl acetate then methanol) provided 4.5 mg (18%) of the title compound as a white solid: ¹H NMR (300 MHz, CD₃OD, 3:1 mixture of two conformers) d 7.37-7.19 (m, 5 H), 5.25-5.10 (m, 1 H), 4.75 (m, 1 H), 4.23 (d,. J = 1.6 Hz, 1/4 H), 4.14 (d,. J = 1.6 Hz, 3/4 H), 4.12 (t, J = 10.5 Hz, 1 H), 3.67 (m, 4 H), 3.03 (s, 3/4 H), 3.06-2.98 (m, 1 H), 2.86-2.70 (overlapping dd, 1 H), 2.63 (s, 9/4 H), 2.62-2.21 (m, 7 H), 1.90-0.80 (m, 17 H), 1.48 (s, 27/4 H), 1.36 (s, 9/4 H); MS(FAB) 731 (M+1).

### SECTION J: PREPARATION OF MACROCYCLIC RENIN INHIBITORS OF FORMULA I where W = -0-, Z = -OH, and Y = -OCO-:

Scheme 15 illustrates the preparation of macrocylic renin inhibitors of Formula I in which W = -0-, Z = -OH, and Y = -OCO-. After removal of the Boc protecting group from macrocycle 101, the resulting amino analog may be acylated with Boc-Phe, giving macrocycle 102, or with other carboxylic acids of sulfonyl chlorides to provide similar analogs.

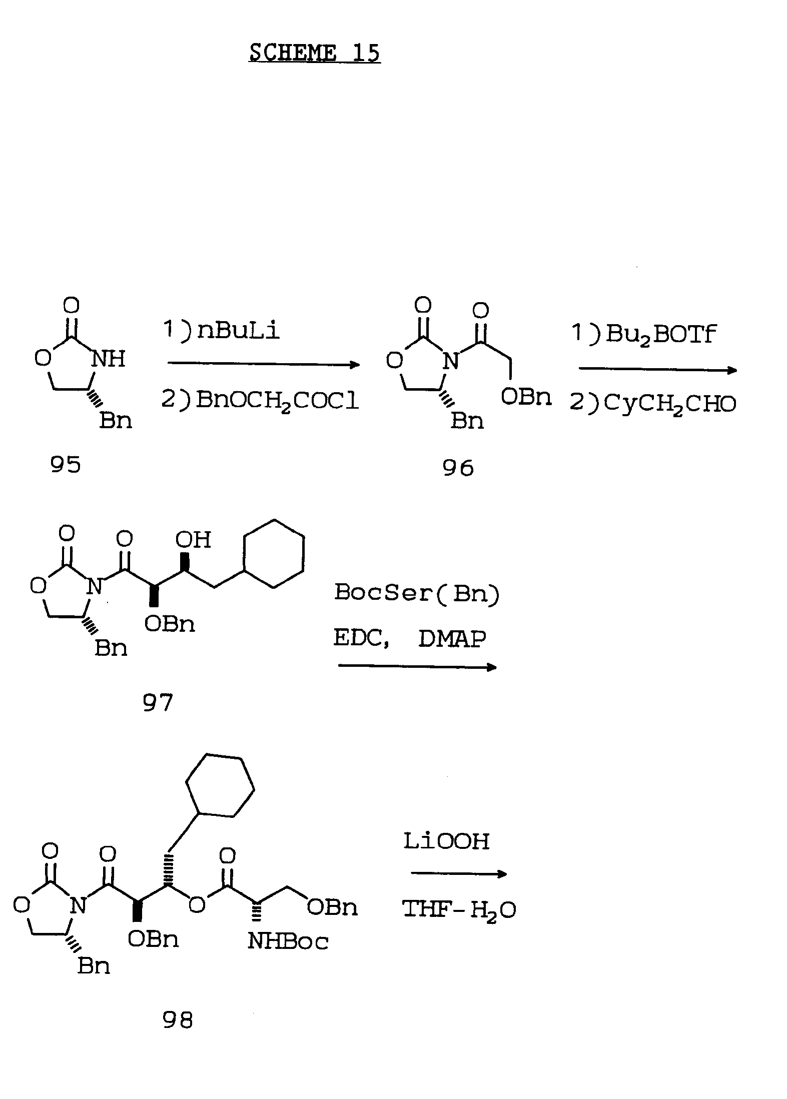

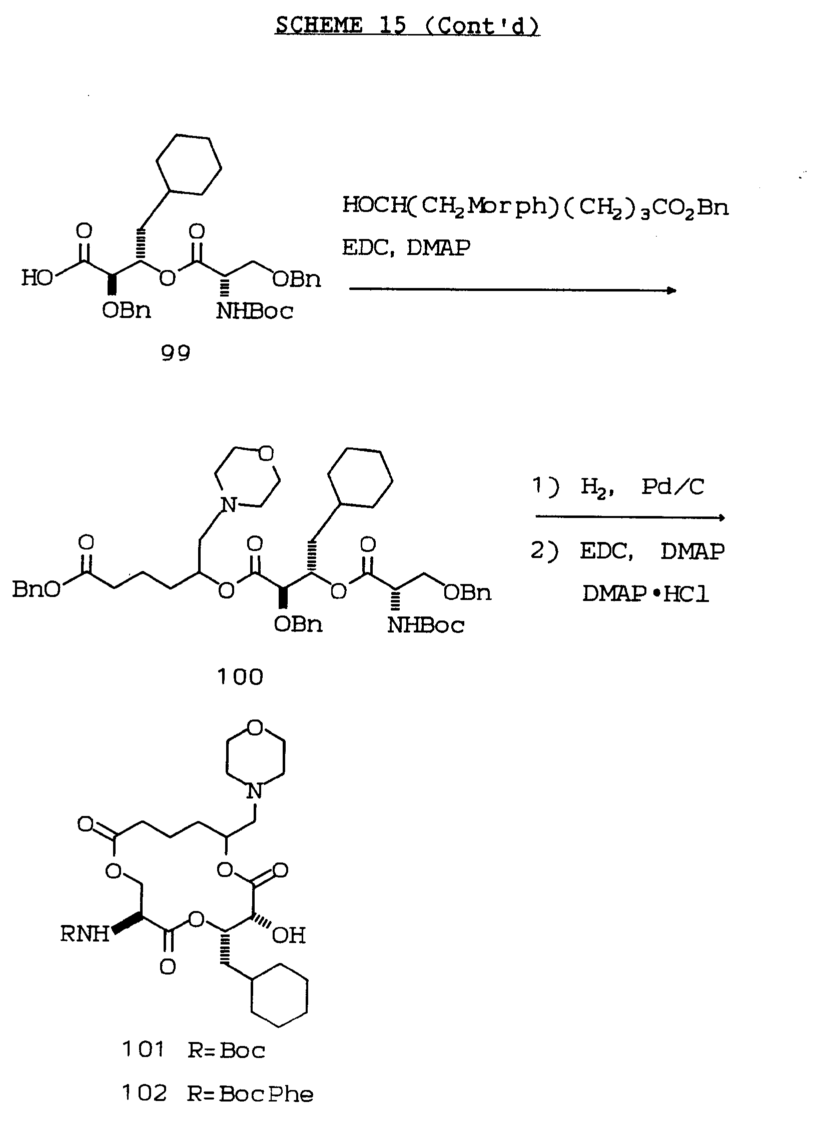

### Acyl Oxazolidinone 96.

To a solution of oxazolidoinone 95 in 180 mL of THF at -78 °C was added 20.1 mL (50.3 mmol, 2.5 M in hexane) of n-butyllithium. Afterfive min, 10.2 g (98.8.73 mL, 55.3 mmol) of benzyloxyacetyl chloride was added. The solution was stirred for 15 min at-78 °C and 15 min at room temperature, and then quenched by the addition of saturatd aqueous ammonium hydroxide solution. Volatiles were removed and the aqueous residue was extracted with three 200-mL portions of dichloromethane. The combined aqueous extracts were washed with saturated aqueous sodium bicarbonate solution, dried over anhydrous magnesium sulfate, and concentrated to give an oil. A portion of this oil was purified by MPLC (2 Lobar B silica gel columns in series, 35% ethyl acetate/hexane) to give 3.01 g of an oil whch crystallized. The remainder of the material was recrystallized from hot ethanol (two crops) to give an additional 10.04 g (total yield 80%) of the title compound: R_{f} 0.24 (30% ethyl acetate/hexane); ¹H NMR (300 MHz, CDCl₃) δ 7.44-7.20 (m, 10 H), 4.78-4.65 (m, 5 H), 4.32-4.22 (m, 2 H), 3.34 (dd, J = 3.3, 13.5 Hz, 1 H), 2.82 (dd, J = 9.5, 13.5 Hz, 1 H); MS(FAB) 326 (M+1).

Anal. calcd. for C₁₉H₁₉NO₄: C, 70.14; H, 5.89; N, 4.30. Found: C, 70.25; H, 6.02; N, 4.24.

### Aldol Adduct 97.

To solution of 2.20 g (6.78 mmol) of acyl imide 96 in 20 mL of dichloromethane at -78 °C was added 2.04 g (1.87 mL, 7.45 mmol, 1.1 equiv) of di-n-butylboryl triflate and 0.82 g (1.13 mL, 8.13 mmol, 1.2 equiv) of triethylamine. The reaction mixture was stirred at -78 °C for 1.5 h, then 1.03 g (8.13 mmol, 1.2 equiv) of cyclohexy- lacetaldehyde in 5 mL of dichloromethane was added via cannula. After the solution was stirred for 4 h at -78 °C, it was poured into 500 mL of ethyl acetate and washed with 250-mL portions of 1 N aqueous sodium bisulfate solution and saturated aqueous sodium bicarbonate solution, dried over magnesium sulfate, and concentrated. Purification by MPLC (2 Lobar B silica gel columns in series, 35% ethyl acetate/hexane) gave 2.21 g (72%) of the title compound as a white solid: R_{f}O.32 (40% ethyl acetate/hexane); ¹H NMR (300 MHz, CDCl₃)δ 7.45-7.20 (M, 10 H), 5.10 (d, J = 2.2 Hz, 1 H), 4.75 (d, J = 11.4 Hz, 1 H), 4.69 (m, 1 H), 4.52 (d, J = 11.4 Hz, 1 H), 4.29-4.18 (m , 2 H), 4.02 (br d, J = 8.2 Hz, 1 H), 3.30 (dd, J = 3.3, 13.4 Hz, 1 H), 2.76 (dd, J = 9.5, 13.4 Hz, 1 H), 2.15 (br s, 1 H), 1.83-0.84 (m, 13 H); MS(FAB) 474 (M+23), 452 (M±1).

Anal. calcd. for C₂₇H₃₃NO₅·1/4H₂O: C, 71.11; H, 740; N, 307. Found: C, 71.25; H,7.43; N, 2.85.

### Ester 98.

A 2.21 g (4.88 mmol) sample of aldol adduct 97 and 2.89 g (9.77 mmol, 1 equiv) of N-Boc serine O-benzyl ether were coupled according to the general procedure of EDC/DMAP esterification. Purification by MPLC (2 Lobar B silica gel columns in series, 20% ethyl acetate/hexane) afforded 3.42 g (96%) of the title compound as a white foam: R_{f}0.64 (40% ethyl acetate/hexane); ¹H NMR (300 MHz, CDCl₃)δ 7.41-7.06 (M, 15 h), 5.50 (ddd, J = 1.8, 5.7, 7.0 Hz, 1 H), 5.35 (d, J= 8.3 Hz, 1 H), 5.19 (d, J = 1.8 Hz, 1H), 4.79 (d, J = 11.8 Hz, 1H), 4.65 (d, J = 12.0 Hz, 1 H), 4.52 (d, J = 12.0 Hz, 1 H), 4.46-4.30 (m, 2 H), 4.41 (d, J = 11.8 Hz, 1 H), 4.04 (t, J = 8.7 Hz, 1 H), 3.95 (dd, J= 2.0, 8.7 Hz, 1 H), 3.90 (dd, J = 3.0, 9.6 Hz, 1 H), 3.73 (dd, J = 3.8, 9.6 Hz, 1 H), 2.98 (dd, J = 2.8, 13.4 Hz, 1 H), 2.73 (dd, J = 9.0, 13.4 Hz, 1 H), 1.78-0.81 (m, 13 H), 1.43 (s, 9 H); MS(FAB) 729 (M+1), 629.

Anal. calcd. for C₄₂H₅₂N₂0₉: C, 69.21; H, 7.19; N, 3.84. Found: C, 69.08; H, 7.27; N, 3.55.

### Acid 99.

To a solution of 3.42 g (4.69 mmol) of ester 98 in 94 mL of 3:1 THF/water at 0°C was added 1.88 mL (18.8 mmol, 4 equiv) of 30% aqueous hydrogen peroxide followed by 394 mg (9.38 mmol, 2 equiv) of lithium hydroxide monohydrate. The reaction mixture was stirred for 40 min and then quenched by the addition of a solution of 2.60 g (20.6 mmol, 4.4 equiv) of sodium sulfite in 20 mL of water. The mixture was poured into a separatory funnel containing 1 L of ethyl acetate and 250 mL of 1 N aqueous sodium bisulfate solution. The layers were separated. The aqueous phase was washed with 500 mL of ethyl acetate. The combined organic layers were dried over anhydrous magnesium sulfate and concentrated to give a clear oil. Purification by MPLC(0.03 x 2 m Sephadex LH-20, methanol) provided 2.13 g (80%) of the title compound as a white foam: 1 H NMR (300 MHz, CDCl₃) δ 7.39-7.21 (m, 10 H), 5.80 (br s, 1 H), 5.43 (d, J = 8.8 Hz, 1 H), 5.37 (m, 1 H), 4.74 (d, J = 11.7 Hz, 1 H), 4.57-4.39 (m, 4 H), 3.96-3.93 (m, 2 H), 3.68 (dd, J = 3.5, 9.6 Hz, 1 H), 1.65-0.81 (m, 13 H), 1.43 (s, 9H); MS(FAB) 592 (M+23), 470.

### Benzyl ester 100.

A 1.92 g (3.37 mmol) sample of acid 99 and 1.04 g (3.37 mmol, 1 equiv) of benzyl 5-hydroxy-6-(4'-mor- pholino)hexanoate were coupled according to the general procedure of EDC/DMAP esterification. The reaction was allowed to proceed in the refrigerator overnight and the acid wash in the work-up was omitted. Purifiation by flash chromatography (30 x 150 mm silica gel, 35% ethyl acetate/hexane) followed by MPLC (Lobar B siica gel column, 40% ethyl acetate/hexane) gave 1.59 g (55%) of the title compound as a mixture of diastereomers: R_{f} 0.30 (35% ethyl acetate/hexane); ¹H NMR (2:1 mixture of diastereomers, 300 MHZ, CDCl₃) δ 7.39-7.21 (m, 15 H), 5.54 (d, J = 9.8 Hz, 1/3 H), 5.41-5.34 (m, 1 2/3 H), 5.12 (s, 2 H), 5.06 (m, 1 H), 4.84 (d, J = 12.1 Hz, 2/3 H), 4.78 (d, J = 12.1 Hz, 1/3 H), 4.57-4.39 (m, 4 H), 3.95 (d, J = 4.2 Hz, 1/3 H), 3.91 (d, J = 3.4 Hz, 2/3 H), 3.85 (m, 1 H), 3.72-0.55(m, 5 H), 2.57-2.23 (m, 8 H), 1.76-0.76 (m, 17 H), 1.26 (s, 9 H); MS(FAB) 859 (M+1), 803.

Anal. calcd. forC4gH66N206: C, 68.51; H, 7.74; N, 3.26. Found: C, 68.64; H,7.71; N, 3.42.

### Macrocycle 101.

A suspension of 1573 mg (1.831 mmol) of benzyl ester 100 and 1.88 g of 20% palladium hydroxide on carbon in 18 mL of 2:1 ethanol/cyclohexene was heated at reflux for 12 h. The suspension was then filtered and concentrated. The resultant oil was dissolved in 18 mL of tetrahydrofuran and added dropwise over 21 h to a refluxing solution of 702 mg (3.66 mmol, 2.0 equiv) of EDC, 246 mg (2.01 mmol, 1.1. equiv) of DMAP and 581 mg (3.66 mmol, 2.0 equiv) of DMAP hydrochloride in 100 mL of ethanol free chloroform. After addition was complete, the solution was cooled, diluted with 500 mL of ethyl acetate, washed sequentially with 250-mL portions of saturated aqueous sodium bicarbonate and saturated aqueous sodium chloride, dried over anhydrous magnesium sulfate and concentrated. Purification by flash chromatography (30 x 150 mm silica gel, 2.5% methanol/dichloromethane) followed by MPLC (Lobar B silica gel column, 80:20:5 ethyl acetate/hexane/isopropanol) gave 80 mg of a faster eluting diastereomer (R_{f}0.29 (75% ethyl acetate/hexane)) and 73 mg (7%) of the title compound: R_{f}0.21 (75% ethyl acetate/hexane)); ¹H NMR (300 MHz, CD₃OD) δ 5.48 (br t, J = 6.4 Hz, 1 H), 5.11 (br m, 1 H), 4.47-4.20(m, 4H), 3.66 (br s, 4 H), 2.71 (d, J = 8.8, 13.2 Hz, 1 H), 2.67-2.22 (m, 7 H), 1.89-0.89 (m, 17 H), 1.45 (s, 9 H); MS(FAB)571 (M+1), 515.

Macrocycle 102.

A solution of 28 mg (0.0492 mmol) of macrocycle 101 in 1:1 trifluoroacetic acid/dichloromethane was stirred at room temperature for 0.5 h. The solution was concentrated and trace amounts of acid were removed azeo- tropicallywith tetrahydrofuran and toluene. The resultant oil was dried over P₂O₅/KOH under vacuum for several hours. It was then dissolved in 1 mL of dichloromethane and treated with 0.011 mL(18 mg, 0.0985 mmol, 2 equiv) of N-methyl morpholine, 26 mg (0.0985 mmol, 2 equiv) of Bocphe, 15 mg (0.0985 mmol, 2 equiv) of HOBt, and 19 mg (0.0985 mmol, 2 equiv) of EDC. The solution was stirred overnight with gradual warming to room temperature. It was then subjected directly to flash chromatography (20 x 150 mm silica gel, 2.5% methanol/dichloromethane). Further purification by MPLC (LobarA silica gel column, 99:1 chloroform to 10:1 methanol/ammonium hydroxide) provided 22 mg (62%) of the title compound as a clear glass: R_{f}0.30 (5% methanol/dichloromethane); 1H NMR (300 MHz, CD₃OD) δ 7.35-7.18 (m, 5 H), 5.52 (br t, J = 7.7 Hz, 1 H), 5.10 (m, 1 H), 4.74 (dd, J = 3.2, 4.8 Hz, 1 H), 4.42-4.27 (m, 4 H), 3.72-3.60 (m, 4 H), 3.12 (dd, J = 4.8, 13.7 Hz, 1 H), 2.81 (dd, J = 9.6, 13.7 Hz, 1 H), 2.70(dd, J = 9.0, 13.3 Hz, 1 H), 2.60-2.20 (m, 7 H), 1.89-0.93 (m, 17 H), 1.36 (s, 9 H); MS(FAB) 718 (M+1), 662, 618.

Anal. calcd. for C₃₇H₅₅N₃O₁₁·1/2H₂O: C, 61.14; H, 7.77; N, 5.78. Found: C, 61.49; H, 7.84; N, 5.58.

### SECTION K: PREPARATION OF MACROCYCLIC RENIN INHIBITORS OF FORMULA I where W =-NH-, Z = -OH, Y = -OCO-, and A-B is a hydroxyethylene isostere:

Scheme 16 illustrates the preparation of macrocyclic renin inhibitors of the Formula I where W = -NH-, Z = -OH, Y = -OCO-, and A-B is hydroxyethylene isostere. As shown in the scheme, Phe-Ser hydroxyethylene isostere derivative 109 is prepared by asymmetric alkylation of imide 107 followed by hydrolysis. Imide 107 in turn is prepared from phenylalaninal 103 as shown in the scheme. Coupling of 109 to alcohol 28 followed by deprotection and macrocyclization give macrocycle 111. This compound is subsequeritly treated with acid and then acylated with Boc₂0 to give inhibitors such as 112, or with an acid chloride or a sulfonyl chloride using standard procedures. Alternatively, compound 111 is treated with acid and then reductively alkylated with aldehydes or ketones such as 3-quinuclidinone using standard procedures.

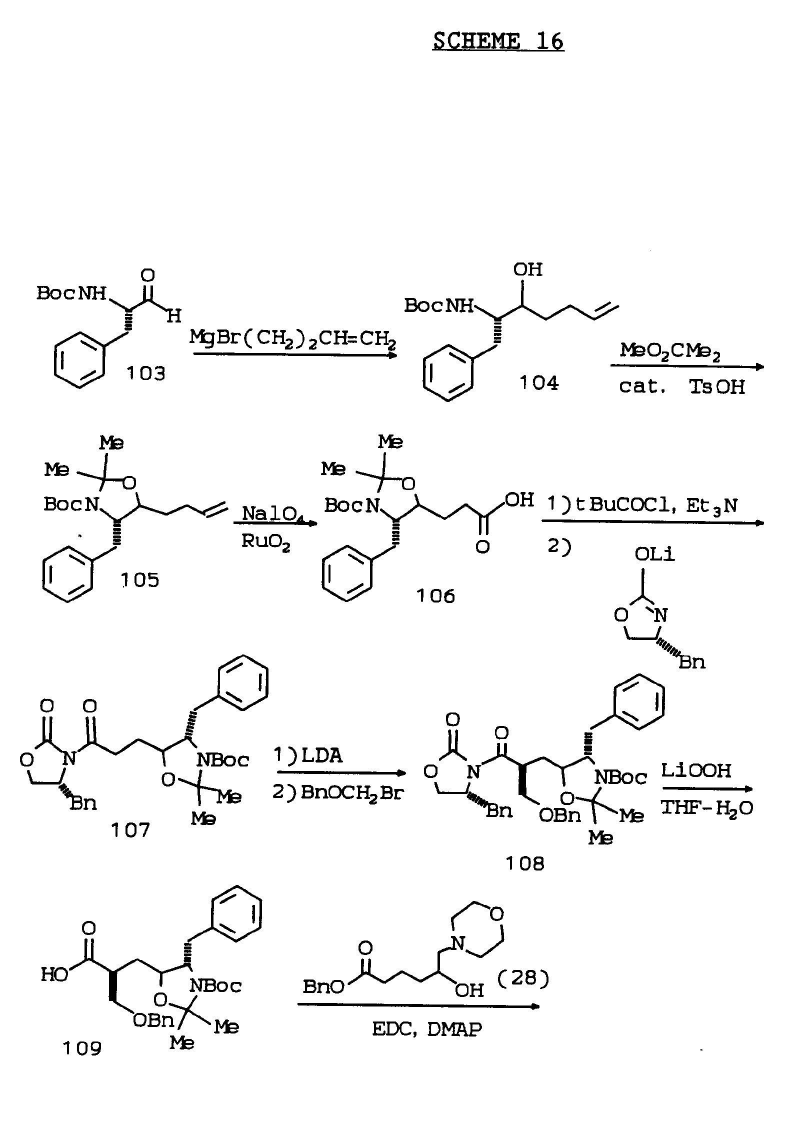

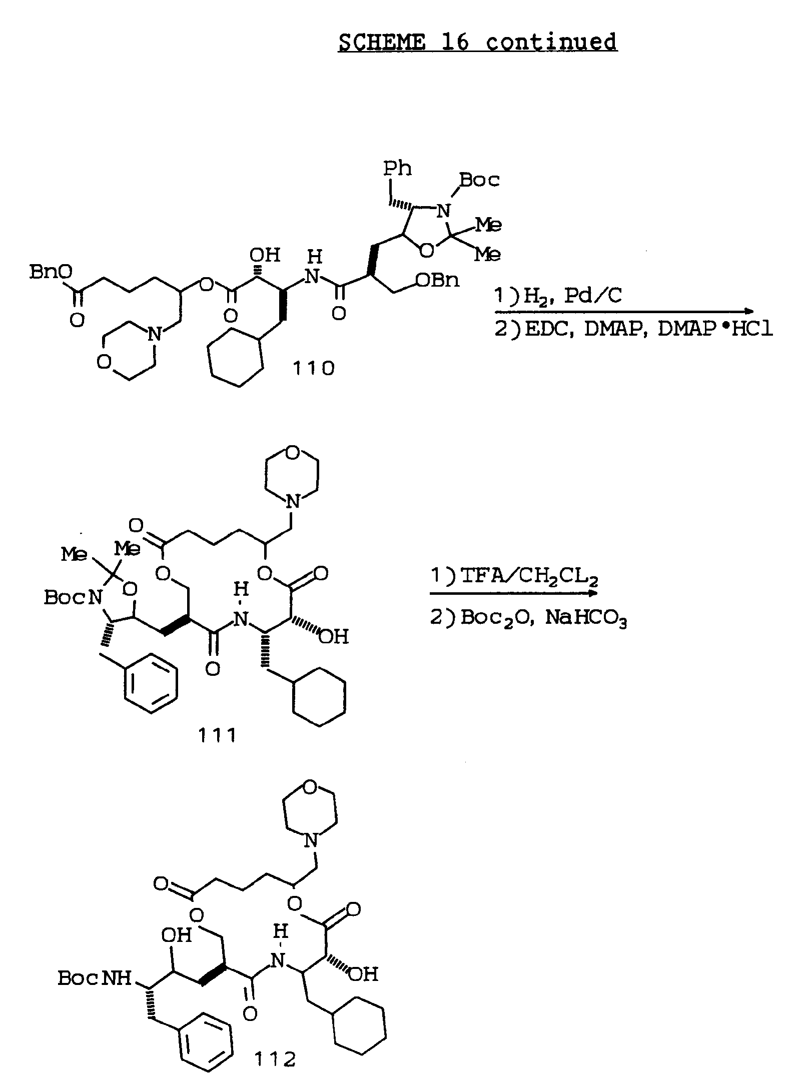

### SECTION L: PREPARATION OF MACROCYCLIC RENIN INHIBITORS OF FORMULA I where W = -0-, Z = -OP0₃H₂ and Y = -OCO-.

Macrocyclic renin inhibitors of Formula I where W = -O-, Z = -OPO₃H₂ and Y = -OCO- may be prepared by standard methods of phosphorylation starting from, for example, macrocycle 102. One method for phosphorylation is treatment of the macrocycle with dibenzylphosphorochloridate and diisopropylethylamine (or pyridine) to afford a dibenzylphosphate ester, followed by removal of the benzyl esters by treatment with Pd/C and H₂. An alternative method which may be used to prepare phosphate derivatives of some macrocycles is treatment of the macrocycle with tetrabenzyl pyrophosphate, followed by deprotection by hydrogenolysis or by treatment with trimethylsilyl bromide (P. M. Chouinard et al, J. Org. Chem., 51, 75-78 (1986)).

### SECTION M: PREPARATION OF MACROCYCLIC RENIN INHIBITORS OF FORMULA I, where W = -NH-, Z = -OH, Y = -OCO-, and A-B is an N-carboxyalkyl derivative

Scheme 17 illustrates the preparation of macrocyclic renin inhibitors of the formula I, where W = -NH-, Z = -OH, Y = -OCO-, and A-B is an N-carboxyalkyl derivative. As shown in Scheme 17, the Boc group of macrocycle 36 is removed and the resultant amine is reductively alkylated with a 2-ketoester to give compounds such as ester 113. Hydrogenolysis of the benzyl esterfollowed by coupling with amines using standard coupling conditions yields amides such as macrocycle 114.

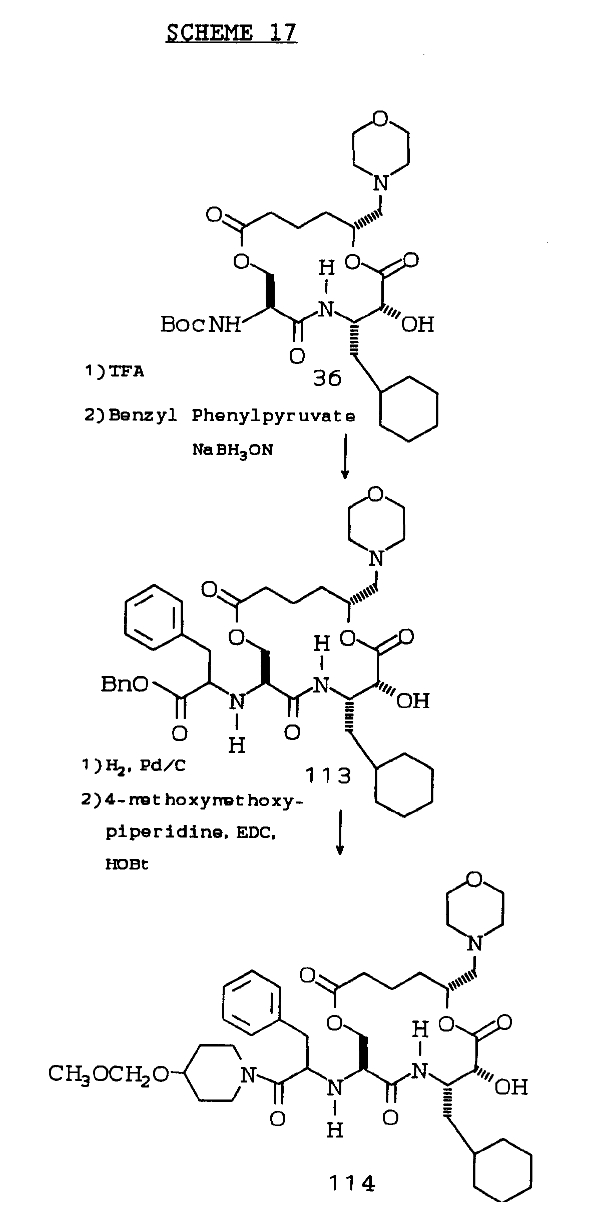

### SECTION N: PREPARATION OF MACROCYCLIC RENIN INHIBITORS OF FORMULA I, where W = -NH-, Z = -OH, Y = -OCO-, and A-B is a carboxyalkoxy derivative

Scheme 18 and 19 illustrate the preparation of macrocyclic renin inhibitors of the formula I, where W = -NH-, Z = -OH, Y = -OCO-, and A-B is a carboxyalkoxy derivative. As shown in Scheme 18, compound 29 is treated with acid and the resultant amine is coupled with acid amide 119 (prepared as shown from serine derivative 115) to provide cyclization precursor 120. Removal of the benzyl groups from compound 120 followed by cyclization gives macrocycle 122. Alternatively, as shown in Scheme 19, compound 29 is treated with acid and the resultant amine is coupled with acid ester 123 (prepared as shown from bromide 116) to provide cyclization precursor 124. Deprotection and cyclization provides macrocycle 125. The tert-butyl blocking group of macrocycle 125 is removed and the resultant acid is coupled to amines or alcohols using standard coupling conditions to provide macrocycles such as 122.

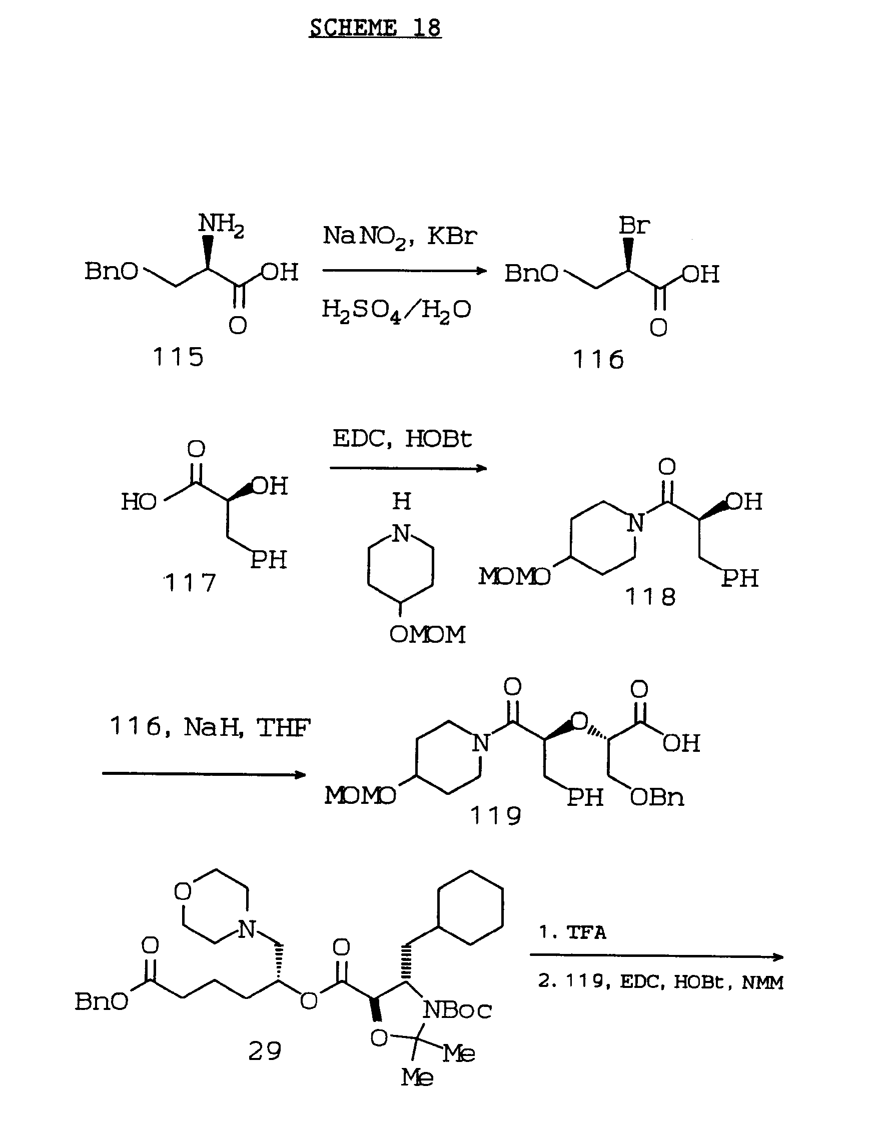

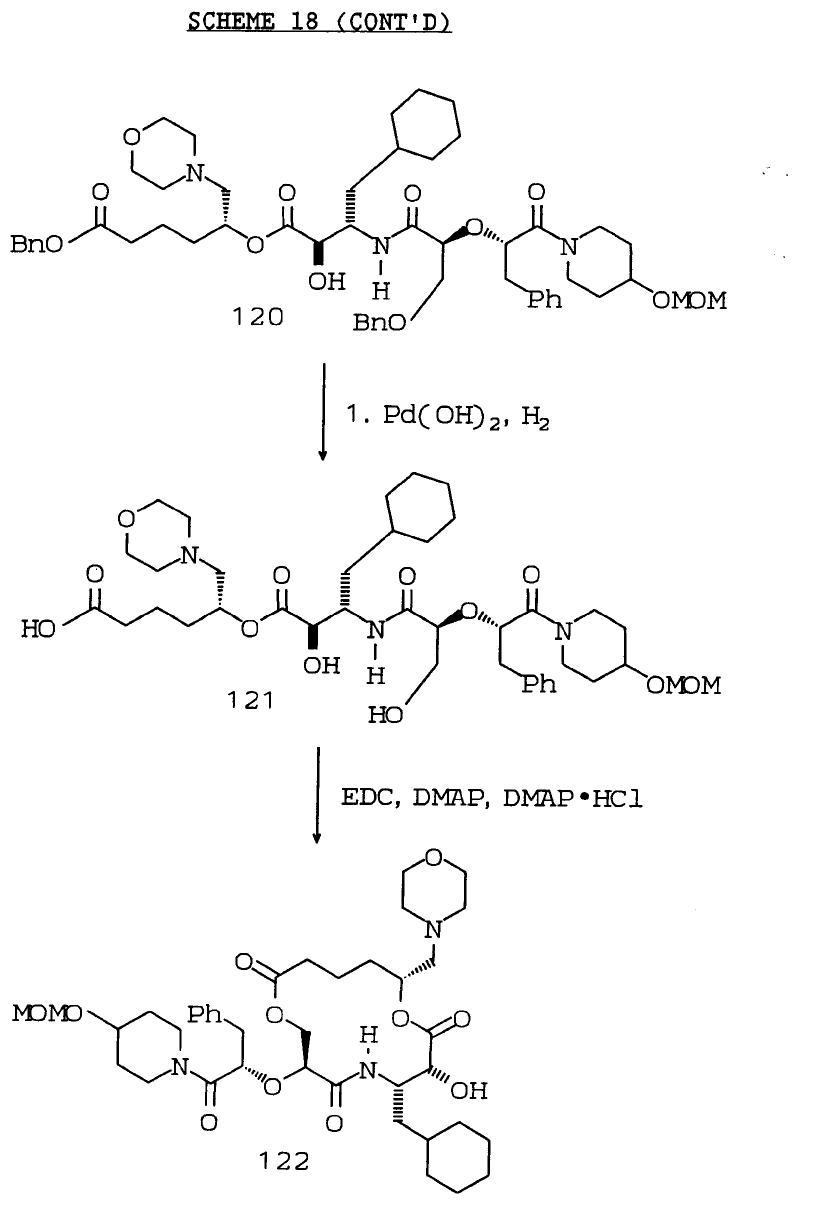

### Compound 118

A solution of 754 mg (4.53 mmol) of L-3-phenyllactic acid in 1:1 DMF/dichloromethane was cooled to 0°C and 658 mg (4.53 mmol) of 4-(methoxy-methoxy)piperdine was added followed by 1.83 g (13.59 mmol) of HOBt and 1.29 g (6.8 mmol) of EDC. The reaction mixture was stirred for 18 hours at room temperature, diluted with 200 ml of ethyl acetate and washed with saturated sodium bicarbonate. The organic layer was dried over magnesium sulfate and concentrated. Flash chromatography (30 x 150 mm silica gel, hexane/ethyl acetate 1:1) gave 915 mg (67%) of the title compound.

### Compound 116

A solution of 2.0 g (10 mmol) of D-O-benzyl-serine in 40 mL of 2.5 N H₂S0₄ was cooled to 0°C and 4.28 g (36 mmol) of KBr was added followed by 1.03 g (15 mmol) of NaN0₂. The solution was stirred for 1 hour at 0°C and then for 1 hour at room temperature. The reaction mixture was then extracted with ethyl acetate (3 x 1 vol). The combined organic phases were dried over magnesium sulfate and concentrated to produce 1.91 g (74%) of the title compound. The crude product was azeotroped from benzene and used in the next step without purification.

### Compound 119

A solution of 804 mg (2.74 mmol) of 118 in 2 ml of THF was added to a suspension 181 mg (6.03 mmol) of NaH in 3 ml of THF at 0°C. The reaction mixture was stirred for 45 minutes at room temperature and then a solution of 777 mg (3.0 mmol) of 116 in 3 ml of THF was added. The suspension was stirred at room temperature for 23 hours and then quenched with H₂0. The aqueous layer was extracted with ethyl acetate to remove unreacted 118. The aqueous layer was then made acidic with 1N sodium bisulfate and extracted with chloroform (4 x 1 vol). The combined organic phases were dried over magnesium sulfate and concentrated. Purification by MPLC (LH20 column, methanol) gave 386 mg (30%) of the title compound.

MS (FAB) 472 M+1.

### Compound 120

A solution of 315 mg (0.5 mmol) of 29 in 1:1 trifluoroacetic acid/dichloromethane was stirred for 1 hour. The solution was concentrated and azeotroped with toluene and tetrahydrofuran. The residue was dissolved in dichloromethane and cooled to 0°C. A solution of 235 mg (0.5 mmol) of 119 in dichloromethane was added followed by 101 mg (0.75 mmol) HOBt, 0.082 mL (0.75 mmol) NMM, and finally 143 mg (0.75 mmol) of EDC. The reaction mixture was stirred for 3 hours at 0°C and then for 12 hours at room temperature. The solution was then diluted with 50 mL of ethyl acetate and washed with saturated sodium bicarbonate followed by brine. The combined organic phases were dried over magnesium sulfate and concentrated. Flash chromatography (30 x 150 mm silica gel, hexane/acetone 2:1) gave 269 mg (56%) of the title compound.

MS (FAB) 944 M+1, 882, 854, 675.

### Compound 122

A solution of 57 mg (0.057 mmol) of 120 in tetrahydrofuran was stirred over Pd(OH₂) under 1 atm of H₂ for 48 hours. The suspension was then filtered and concentrated. The resulting oil was dissolved in 4 mL of tetrahydrofuran and added dropwise over 18 hours to a refluxing solution of 21 mg (0.108 mmol) of EDC, 20 mg (0.162 mmol) of DMAP, and 17 mg (0.108 mmol) of DMAP hydrochloride in 25 mL of chloroform. The reaction mixture was cooled and poured into 100 mL of ethyl acetate and washed with saturated sodium bicarbonate followed by brine. The organic layer was dried over magnesium sulfate and concentrated. Flash chromatography (20 x 150 mm silica gel, hexane/acetone 2.5:1) provided 13.7 mg (34%) of the title compound.

MS (FAB) 746 M+1, 730, 573.

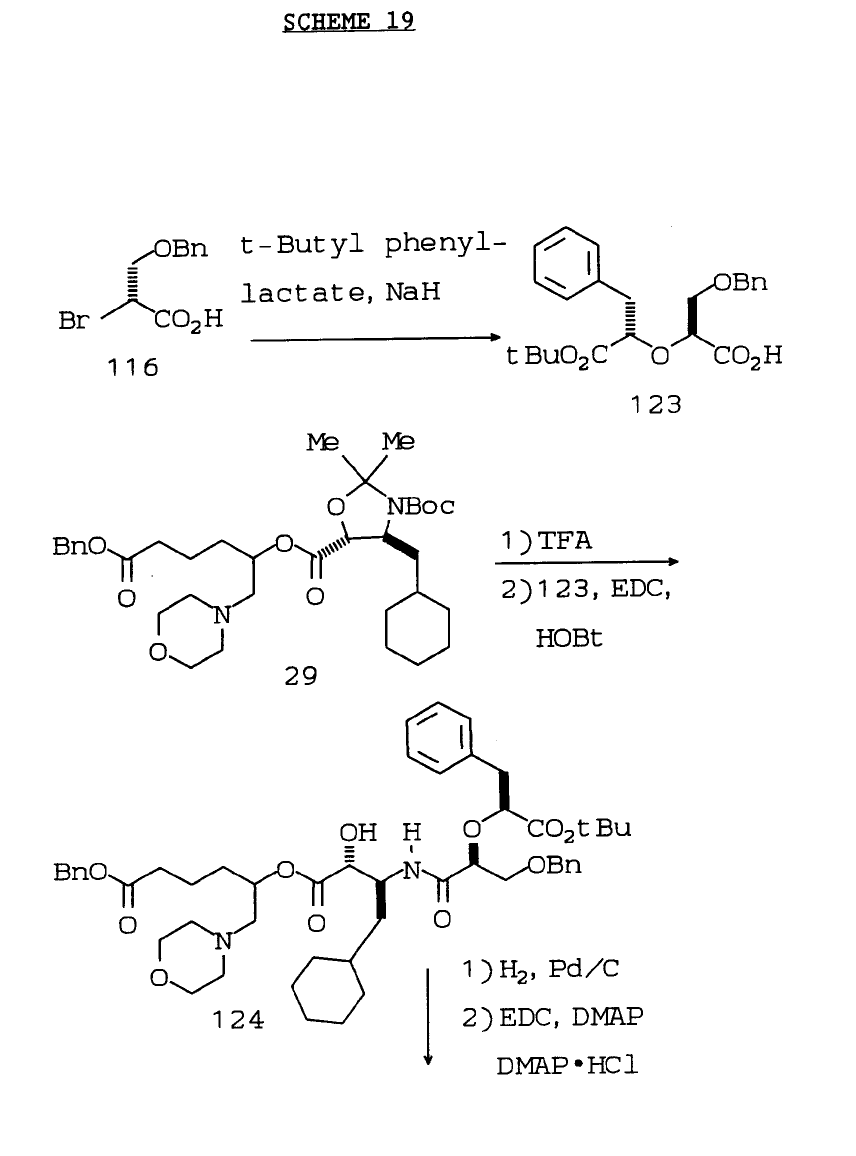

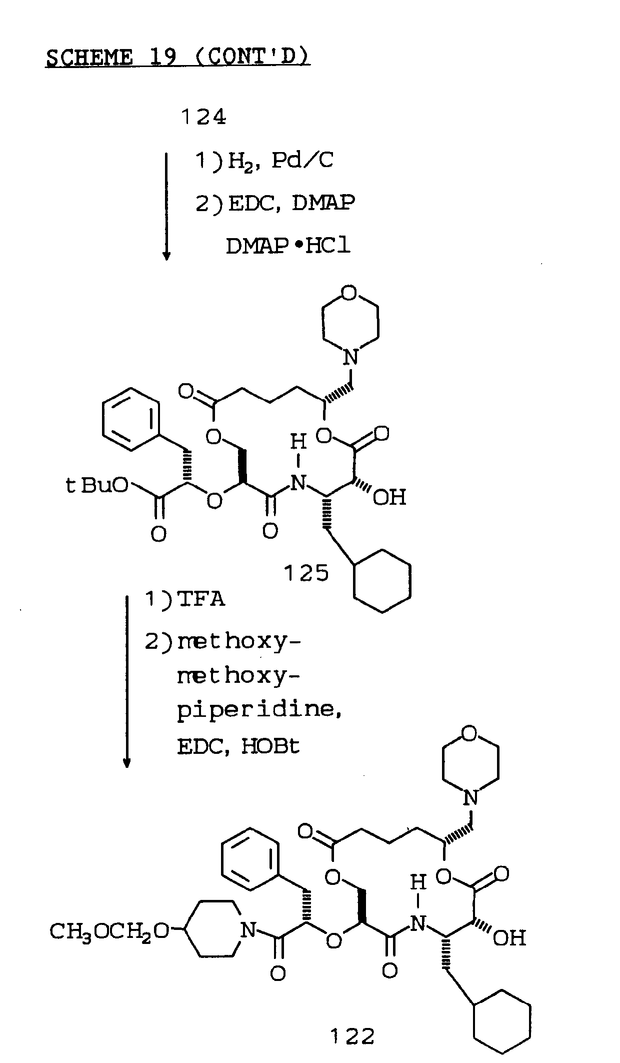

### SECTION O: PREPARATION OF MACROCYCLIC RENIN INHIBITORS OF FORMULA I, where W = -NH-, Z = -OH, Y = -OCO-, and A-B = RCO₂-

Scheme 20 illustrates the preparation of macrocyclic renin inhibitors of the formula I, where W = -NH-, Z = -OH, Y = -OCO-, and A-B is RCO₂-. As shown in Scheme 20, compound 29 is treated with acid and the resultant amine is coupled with acid 128 (prepared as shown from serine derivative 126) to provide cyclization precursor 129. Removal of the benzyl groups from compound 129 followed by cyclization gives macrocycle 130. The methoxymethyl blocking group of macrocycle 130 is removed and the resultant alcohol is coupled to carboxylic acids, acid chlorides, or sulfonyl chlorides using standard coupling procedures to yield macrocycles such as 131.

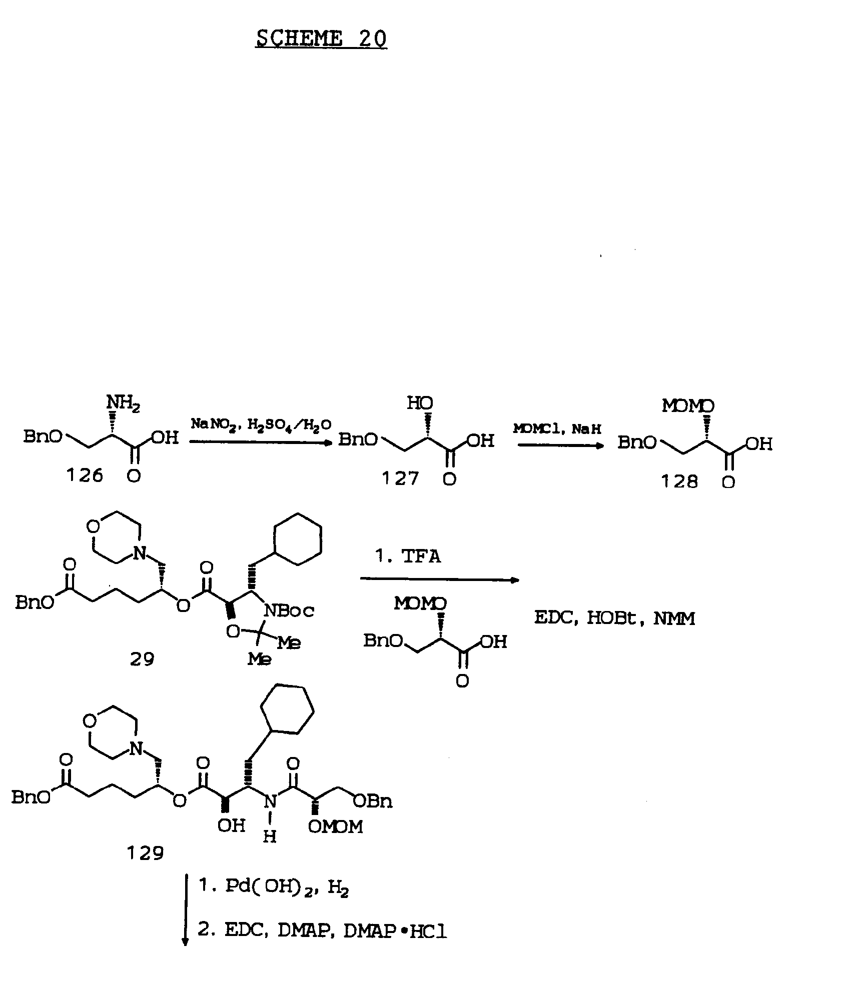

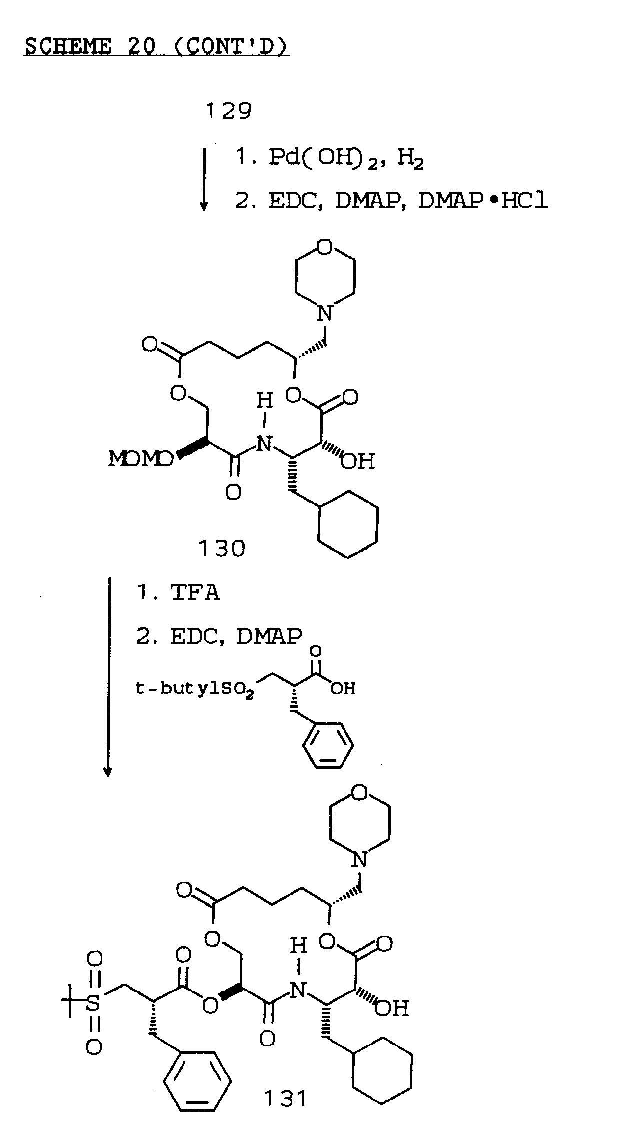

### Compound 127

A solution of 5.3 g (27 mmol) of O-benzyl-serine in 150 mL of H₂0 and 2.5 mL of H₂S0₄ was cooled to 0°C and 2.34 g (34 mmol) of sodium nitrite was added in three portions. The solution was stirred for 16 hours at 0°C and then extracted with ethyl acetate (3 x 1 vol). The combined organic layers were dried over magnesium sulfate and concentrated to provide 2.9 g (52%) of the title compound. The compound was azeotroped from benzene and used without purification in the next reaction.

### Compound 128

A solution of 1.34 g (6.8 mmol) of 127 in 20 mL of tetrahydrofuran was added to a 0°C suspension of 450 mg (15.0 mmol) of NaH in tetrahydrofuran. The reaction mixture was stirred for 10 minutes at 0°C and then for 25 minutes at room temperature. The suspension was then cooled to -78°C and 0.607 mL (8.1 mmol) of MOMCI was added. The C0₂ bath was allowed to melt and the reaction mixture was stirred for 12 hours at room temperature. The reaction was quenched with 5% aqueous potassium carbonate and the aqueous layers were extracted with ethyl acetate. The aqueous layers were made acidic with IN HCI and extracted with ethyl acetate (3 x 1 vol). The combined organic layers were dried over magnesium sulfate and concentrated. Purification by MPLC (LH20 column, methanol) gave 573 mg (34%) of the title compound.

### Compound 129

A solution of 398 mg (0.63 mmol) of 29 in 1:1 trifluoroacetic acid/dichloromethane was stirred for 1 hour. The solution was concentrated and azeotroped with toluene and tetrahydrofuran. The residue was dissolved in dichloromethane and cooled to 0°C. A solution of 211.6 mg (0.882 mmol) of 128 in dichloromethane was added followed by 119 mg (0.882 mmol) HOBt, 0.097 mL (0.882 mmol) NMM, and finally 169 mg (0.882 mmol) of EDC. The reaction mixture was stirred for 3 hours at 0°C and then for 14 hours at room temperature. The solution was then diluted with 50 mL of ethyl acetate and washed with saturated sodium bicarbonate followed by brine. The combined organic phases were dried over magnesium sulfate and concentrated. Flash chromatography (30 x 150 mm silica gel, hexane/acetone 2:1) gave 188 mg (42%) of the title compound.

MS (FAB) 804 M+1, 290, 198, 181.

### Compound 130

A solution of 172 mg (0.241 mmol) of 129 in tetrahydrofuran was stirred over Pd(OH₂) under 1 atm of H₂ for 17 hours. The suspension was then filtered and concentrated. The resulting oil was dissolved in 5 mL of tetrahydrofuran and added dropwise over 18 hours to a refluxing solution of 73 mg (0.381 mmol) of EDC, 70 mg (0.570 mmol) of DMAP, and 60 mg (0.381 mmol) of DMAP hydrochloride in 25 mL of chloroform. The reaction mixture was cooled and poured into 100 mL of ethyl acetate and washed with saturated sodium bicarbonate followed by brine. The organic layer was dried over magnesium sulfate and concentrated. Flash chromatography (20 x 150 mm silica gel, hexane/acetone 2:1) provided 48.1 mg (49%) of the title compound. MS (FAB) 515 M+1.

### Compound 131

A solution of 28.8 mg (0.054 mmol) of 130 in 1:1 trifluoroacetic acid/dichloromethane was stirred for 1 hour. The solution was concentrated and azeotroped with toluene and tetrahydrofuran. The residue was diluted with ethyl acetate and then 0.1 N KOH was added until pH 12. The aqueous layers were extracted with ethyl acetate (3 x 1 vol). The organic layers were dried over magnesium sulfate and concentrated. Flash chromatography (10 x 150 mm silica gel, hexane/acetone 1:1) provided 19.8 mg (78%) of the deprotected macrocycle. A solution of 11.3 mg (0.024 mmol) of the deprotected macrocycle and 7.1 mg (0.025 mmol) of (2R)-3-tert-butylsulfonyl-2-phenylmethyl-propionic acid in dichloromethane was cooled to 0°C. A 6.8 mg (0.036 mmol) sample of EDC and 2.0 mg (0.015 mmol) of DMAP were added. The reaction was allowed to warm to room temperature. After 12 hours, the solution was poured into 50 mL of ethyl acetate and washed sequentially with saturated sodium bicarbonate and saturated sodium chloride. The organic phases were dried over magnesium sulfate and concentrated. Flash chromatography (10 x 150 mm silica gel, hexane/acetone 2:1) provided 7.1 mg of slightly impure product. A second flash column (10 x 150 mm silica gel, hexane/acetone 3:1) gave 3.7 mg (21%) of the pure title compound.

MS (FAB) 737, 525, 416.

### SECTION P: PREPARATION OF MACROCYCLIC RENIN INHIBITORS OF FORMULA I, where D = -CH₂0-, W = -NH-, Z = -OH-, and Y = -OCO-

Scheme 21 illustrates the preparation of macrocyclic renin inhibitors of the formula I, where D = -CH₂0-, W = -NH-, Z = -OH-, and Y = -OCO-. As shown in Scheme 21, compound 134 (prepared as shown from bromoacetic acid) is coupled to 3 to provide compound 135. Compound 135 is teated with acid and the resultant amine is coupled with N-Boc O-benzylserine to yield the cyclization precurser 136. Removal of the benzyl groups of 136 followed by cyclization gives macrocycle 137. The Boc group is removed and the resultant amine is coupled to carboxylic acids, acid chlorides or sulfonyl chorides using standard coupling procedures to yield macrocycles such as 138.

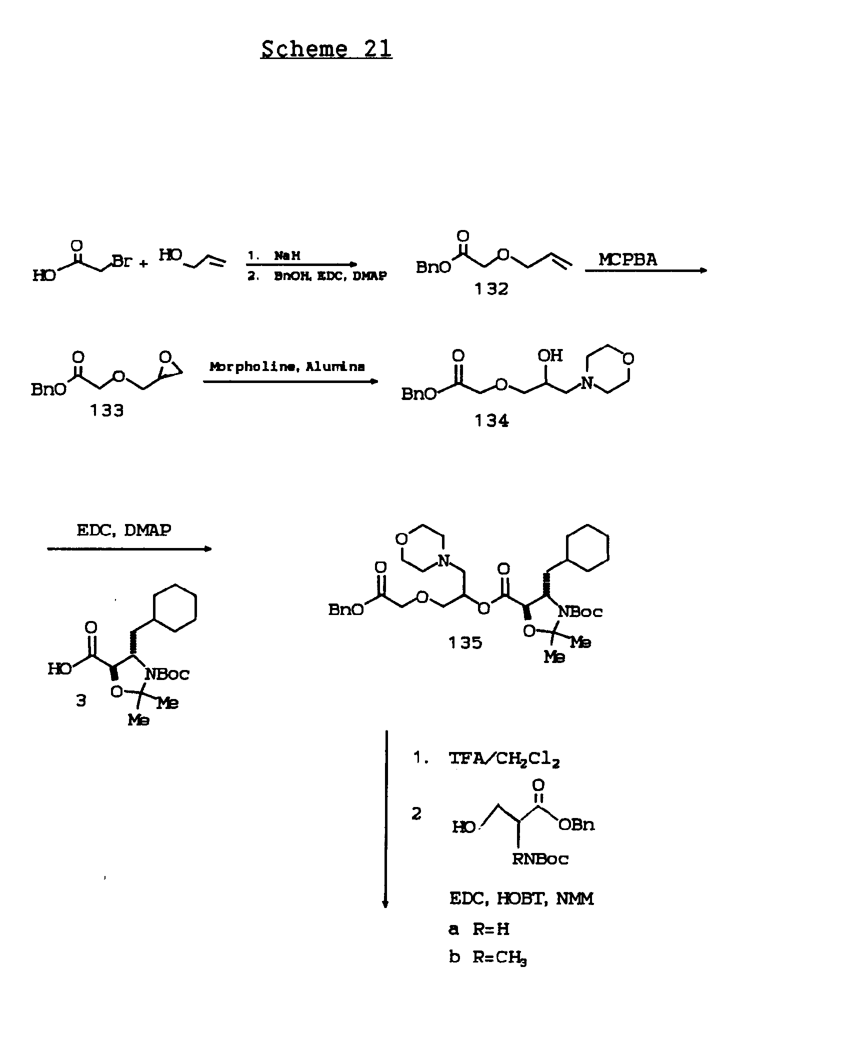

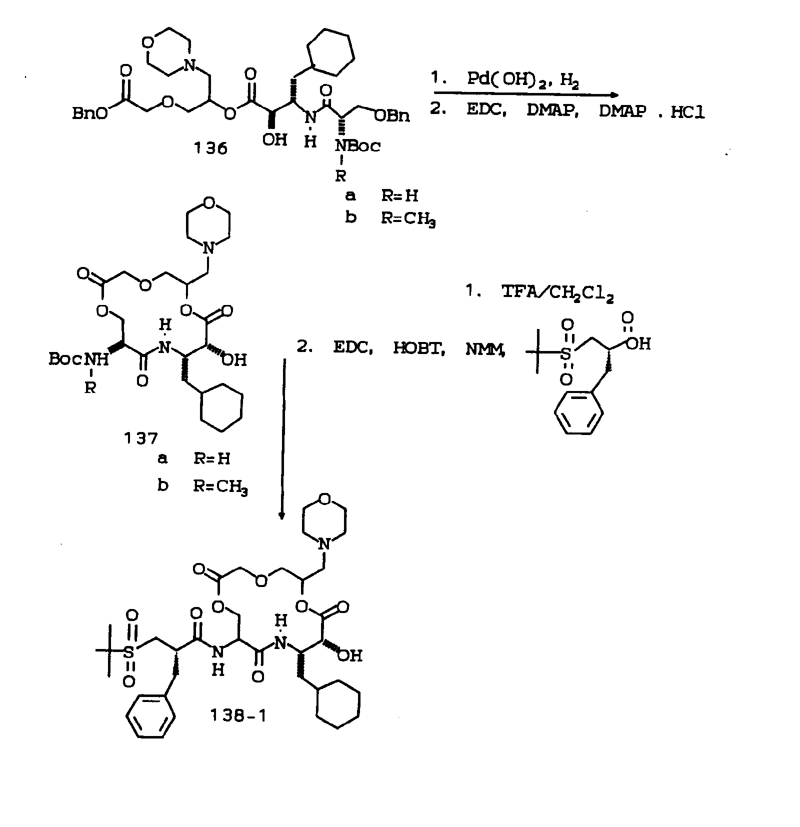

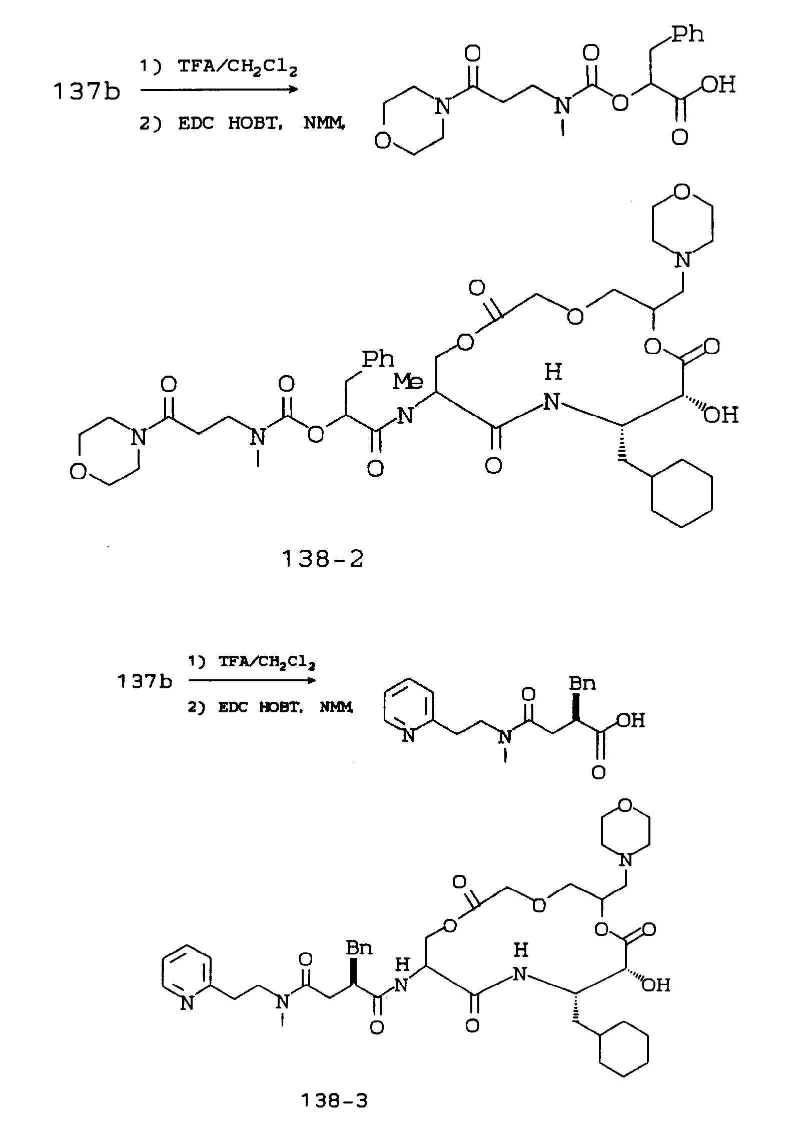

### Compound 132

A suspension of NaH (7.2g, 241 mol) in 230 ml of tetrahydrofuran was cooled to 0°C and allyl alcohol (7.2 ml, 0.115 mol) was added. After 20 minutes bromoacetic acid (16.0 g, 115 mol) was added in five portions. The reaction mixture was heated to 80° for 5 hours and then cooled to room temperature. The pH was adjusted to 1 with 1N aqueous HCI and the aqueous layer was extracted with ethyl acetate (3x1 vol). The combined organic layers were dried over magnesium sulfate and concentrated to yield 11.6 g of crude product. The crude product was dissolved in dichloromethane (100mL) and cooled to 0°C. Benzyl alcohol (15.5 g, 0.15mol) was added followed by EDC (28.65 g, 0.15 mol). The reaction mixture was stirred for 5 hours at room temperature and then poured into ethyl acetate (500 mL). The organic layer was washed with saturated aqueous sodium bicarbonate followed by saturated sodium chloride and dried over magnesium sulfate and concentrated. Flash chomatography (silica gel, 70x150 mm, hexane/ether 2:1) provided the title compound 132 (12.8 g, 62%).

### Compound 133

To a solution of 132 (5.5 g, 26 mmol) in dichloromethane (80ml) at 0°C was added MCPBA (6.88 g, 40 mmol). The ice bath was allowed to melt and the reaction mixture was stirred at room temperature over night. The benzoic acid was filtered off and the reaction mixture was diluted with dichloromethane (100 ml) and washed with .5N aqueous NaOH and saturated aqueous sodium chloride. The organic layer was dried over magnesium sulfate and concentrated. Flash chromatography (silica gel, 50x150 mm, hexane/ether 1:1) provided the title compound (2.7 g, 47%).

### Compound 134

Alumina (11 g) was added to 133 (2.5 g, 11.3 mmol) and morptioline (1.28 ml, 13.56 mmol) in ether (20ml) and the reaction mixture was stirred for 48 hours at room temperature. Concentration followed by flash chromatography (silica gel 30x150 mm, hexane/acetone 2:1) provided the title compound 134 (2.06 g, 59%). MS (FAB) 310 (M+1).

### Compound 135

To a solution of 134 (1.05 g, 3.44 mmol) in dichloromethane was added 3 (1.47 g, 4.30 mmol) and DMAP (0.105 g, 0.86 mmol). The solution was cooled to 0°C and EDC (0.985 g, 5.16 mmol) was added. The reaction mixture was stirred for 1.5 hours at 0 °C. The solution was then diluted with ethyl acetate and washed with saturated aqueous sodium bicarbonate followed by brine. The combined organic phases were dried over magnesium sulfate and concentrated. The 1:1 mixture of diastereomers was separated by flash chromatography (50x150 mm silica gel, hexane/ethyl acetate 3:1) to give a high RF compound, the title compound (1.08 g, > 50%) and a low RF compound (1.13 g, > 50%)

### Compound 136

A solution of 135, (1.06 g, 1.69 mmol) in 1:1 trifluroacetic acid/dichloromethane was stirred for 1 hour. The solution was concentrated and azeotroped with toluene and tetrahydrofuran. The residue was dissolved in dichloromethane and cooled to 0°C. N-Boc-Serine-O-benzyl ether (750 mg, 2.53 mmol), HOBT (341 mg, 2.53 mmol), NMM (0.278 ml, 2.53 mmol), and finally EDC (483 mg, 2.53 mmol) were added. The ice bath was allowed to melt and the reaction mixture was stirred for 15 hours at room temperature. The solution was then diluted with ethyl acetate and washed with saturated aqueous sodium bicarbonate followed by brine. The combined organic phases were dried over magnesium sulfate and concentrated. Flash chromatography (50 x 150 mm silica gel, hexane / acetone 3:1) gave the title compound (963 mg, 74%). MS (FAB) 770 (M+1).

### Compound 137

A solution of 136 (940 mg, 1.2 mmol) in tetrahydrofuran was stirred over Pd(OH)₂ under 1 atm of H₂ for 48 hours. The suspension was then filtered and concentrated. The resulting white foam (682 mg) was dissolved in 8 mL of tetrahydrofuran and added dropwise over 18 hours to a refluxing solution of EDC (420 mg, 2.2 mmol), DMAP (402 mg, 3.3 mmol), and DMAP hydrochloride (341 mg, 2.2 mmol) in 50 mL of chloroform. The reaction mixture was cooled and poured into bicarbonate followed by brine. The organic layer was dried over magnesium sulfate and concentrated. Flash chromatography (30 x 150 mm silica gel, hexane/acetone 2:1) provided the title compound (371 mg, 59%). MS (FAB) 572 (M+1), 516.

### Compound 138

A solution of 137 (318 mg, 0.557 mmol) in 1:1 trifluroacetic acid/dichloromethane was stirred for 1 hour. The solution was concentrated and azeotroped with toluene and tetrahydrofuran. The residue was dissolved in dichloromethane (5.0 ml) and cooled to 0°. R-3-t-butylsulfonyl-2-phenylmethylpropionic acid (205 mg, 0.724 mmol), HOBT (113 mg, 0.835 mmol), NMM (0.092 ml, 0.835 mmol) and finally EDC (160 mg, 0.835 mmol) were added. The reaction mixture was allowed to warm to room temperature. After 16 hours the solution was poured into ethyl acetate and washed sequentially with saturated sodium bicarbonate and saturated aqueous sodium chloride. The organic phases were dried over sodium sulfate and concentrated. Flash chromatography (30 x 150 mm silica gel, hexane/acetone 3:1) provided the title compound (161.7 mg, 39%). MS (FAB) 738 (M+1).

### SECTION Q: PREPARATION OF MACROCYCLIC RENIN INHIBITORS OF FORMULA I where W = -NH-, Z = -OH, and Y = -CHOH-

Scheme 22 illustrates the preparation of macrocyclic renin inhibitors of the formula I where W = -NH-, Z = -OH, and Y=-CHOH-. As shown in Scheme 22, epoxide 141 is opened with the enolate of amide 140 (prepared as shown form lactone 139) to give compound 142. Following protecting group manipulation, the amide is reduced to provide compound 144. The benzyl ether is converted to benzyl ester 145 as shown. Compound 145 is then deprotected and coupled to a serine derivative to provide cyclization precursor 146. Removal of the benzyl groups from compound 146 followed by cyclization gives macrocycle 147. The Boc blocking group of macrocycle 147 is removed and the resultant amine is coupled to carboxylic acids, acid chlorides, or sulfonyl chlorides using standard coupling procedures to yield macrocycles such as 148.

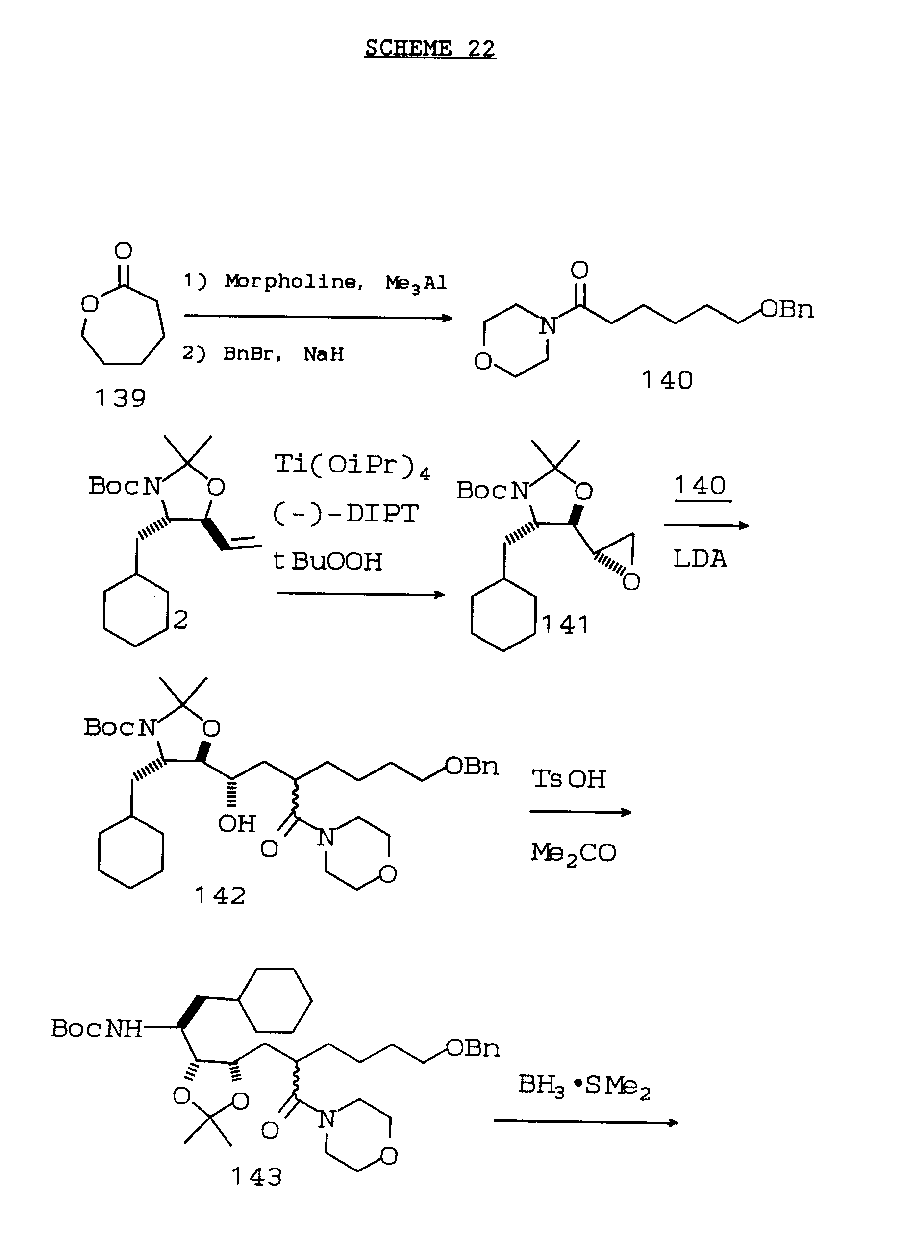

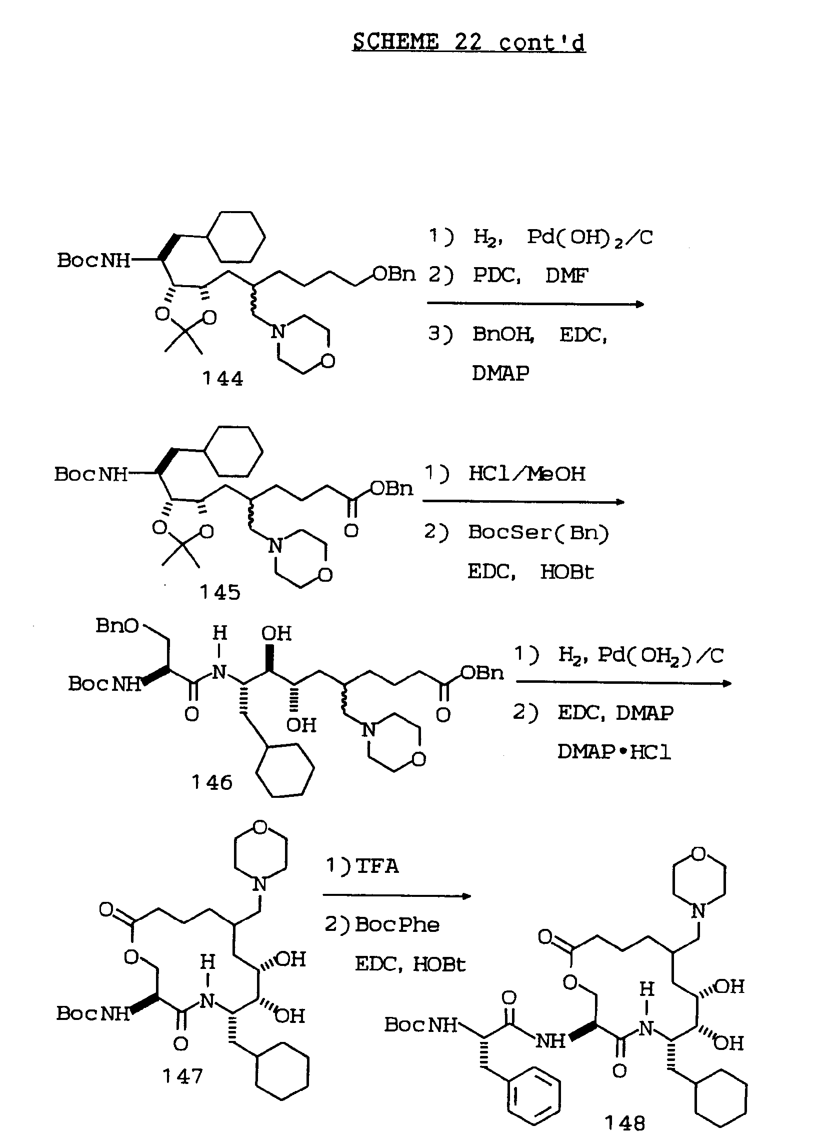

## Claims

1. A compound of the formula: 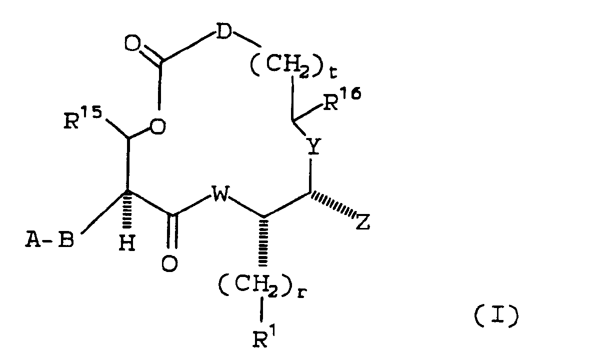
wherein:
A is hydrogen,
Het,
where Het is a saturated or unsaturated 5 to 7-membered monocyclic or 7 to 10-membered bicyclic ring which contains at least one and up to two nitrogen atoms (optionally quaternized or in the N-oxide form),
where Het may optionally be benzofused,
where Het may optionally contain one additional ring atom chosen from among the list consisting of O or S, in sulfide, sulfoxide or sulfone form,
where Het may optionally be substituted with one or two Het substituents independently selected from the group consisting of -OH, C₁-C₄-alkyl, -CF₃, -CN, C₁-C₄-alkoxy, C₁-C₄-alkoxy-C₁-C₄-alkoxy, halo, -NH₂, mono- or di-(C₁-C₄-alkyl)amino, -CO₂H, -CO₂-C₁-C₄-alkyl, -CONR^{2a}R^{2b}, -SO₃H, C₁-C₄-alkyl-CO-, aryl, (where aryl is unsubstituted or mono-, di-, or trisubstituted phenyl or naphthyl wherein the substitutent(s) is/are independently selected from the group consisting of C₁-C₈-alkyl, amino, phenyl-C₁-C₄-alkyl, mono- or di-C₁-C₄-alkyl amino, amino-C₁-C₄-alkyl, mono- or di-C₁-C₄-alkylamino-C₁-C₄-alkyl, guanidyl, guanidyl-C₁-C₄-alkyl, -OH, C₁-C₄-alkoxy, -CONR^{2a}R^{2b} -CO₂H, -C0₂-C₁-C₄-alkyl, -CF₃, halo, C₁-C₄-alkyl-CO-, C₁-C₄-alkyl-CONH-, tri-(C₁-C₄-alkyl)N⁺ X⁻, where X⁻ is a counterion selected from the group consisting of single negatively charged ions, such as chloride, bromide, nitrate, perchlorate, benzoate, maleate, benzenesulfonate, methanesulfonate, tartrate, hemitartrate, and acetate) and mono- or disubstituted C₁-C₄-alkyl, (where the substitutent(s) is/are independently selected from the group consisting of -CO₂H, -CO₂-C₁-C₅-alkyl, C₁-C₅-alkyl-CONH-, -OH, -SO₃H, C₁-C₄-alkyl-S0₂-, C₁-C₄-alkyl-SO-, -SO₂NHCO-C₁-C₄-alkyl, C₁-C₅-alkyl-OCONH- and aryl as defined above),
where if one or both N are quaternized in Het, then each nitrogen atom may be quaternized with a Het substitutent cited above selected from the group consisting of -C₁-C₄-alkyl, -CF₃, aryl, and mono- or disubstituted C₁-C₄-alkyl with the corresponding counterion being X⁻ as defined above,
where Het may have in the alternative to the above Het substituents, a Het substituent selected from the group consisting of -(CH₂)q- and -(CH₂)₂O(CH₂)₂- which forms a quaternary spirocyclic ring with the N atom wherein q is 3-to-6 and the counterion is X⁻ as defined above,
where Het may be substituted both with one Het substituent chosen from among those listed above and also with up to four Het substituents selected from the group consisting of C₁-C₂-alkyl substituents and Het-C₁-C₄-alkyl (where Het is as defined above without optional substitution and where the alkyl group is optionally substituted with one or two substituents independently selected from the group consisting of hydroxyl, -C0₂H, -C0₂-C₁-C₄-alkyl, -SO₃H, and aryl where aryl is as defined above),
aryl,
where aryl is defined above,
R²CO-,
where R² is unsubstituted or mono- or disubstituted C₁-C₄-alkyl where the substituent(s) is/are selected from the group consisting of C₁-C₄-alkyl, -SO₃H, aryl or aryl-CO- (where aryl is as defined above), Het or Het-CO- (where Het is as defined above), R^{2a}O-, R^{2a}OCO-, R²ₐR^{2b}N-, R²ₐR^{2b}NCO-, R²aR^{2b}NCONH-, R2a_{R}2bNS0₂-, (R^{2a}O)(R^{2b}O)PO-, R^{2c}S-, R^{2c}SO-, R^{2c}SO₂-, R^{2c}CONH-, R^{2c}OCONH-, and N(R¹⁷R¹⁸R¹⁹)⁺X⁻ (where R^{2a} and R^{2b} are independently hydrogen, C₁-C₄-alkyl, aryl as defined above, Het as defined above, R²_{c} is C₁-₄-alkyl, aryl as defined above or Het as defined above, R¹⁹ is C₁-C₄-alkyl, R¹⁷ and R¹⁸ are independently aryl as defined above, Het as defined above or C₁-C₄-alkyl optionally substituted with a substituent chosen from the group consisting of aryl as defined above, Het as defined above, -OH, -NH₂, -NH-C₁-C₄-alkyl, -N(C₁-C₄-alkyl)₂, -CO₂H, -C0₂-C₁-C₄-alkyl, -SO₃H, -CO-NH-S0₂-C₁-C₄-alkyl, and -CO-NH-SO₂-aryl, and X⁻ is as defined above), R²⁻ (where R² is defined above),
R²0CO- (where R² is as defined above),
R²SO₂- (where R² is as defined above),
Aryl-CO- (where aryl is as defined above),
Het-CO- (where Het is as defined above),
R2a_{R2bN}-_{CO}- (where R²a and R^{2b} are as defined above), 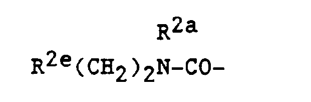
(where R²a is as defined above and R^{2e} is het-CO where HET is as defined above or Het SO₂-),
R²aR^{2b}N-S0₂- (where R²a and R^{2b} are as defined above), and
C₁-C₄-alkyl-(OCH₂CH₂)ₓOCO- (where x is 1 to 3);
B is -CH₂-CH[(CH₂)ᵣR³]CON(R")--N(A¹)CH[(CH₂)ᵣR³]CO-N(R¹¹)-,-O-CH[(CH₂)ᵣR³]CO-N(R¹¹)-,-N(A¹)CH[(CH₂)ᵣR³] -CO-O-, -O-CH[(CH₂)ᵣR³]CO-O- or -N(A¹)CH[(CH₂)ᵣR³]CH(OH)CH₂-,
where r is 0-to-2,
A¹ is hydrogen or C₁-C₄-alkyl,
R³ is hydrogen, C₁-C₄-alkyl,
C₃-C₇-cycloalkyl, aryl as defined above, Het as defined above or 4-(morpholin-4-yl)ethoxy-phenyl, and R¹¹ is hydrogen or C₁-C₄-alkyl;
A and B together may alternatively be:
G-CH₂CH[(CH₂)ᵣR³]-Q-N(R¹¹)-, G-CH₂CH[(CH₂)ᵣR³]-CO-O-, Het-S(O)ₘCH[(CH₂)ᵣR³]CON(R¹¹)-, (where r, R³, R¹¹ and Het are as defined above and Q is -CO- or -S0₂-), R^{2d}CON(R¹¹)-, R^{2d}OCON(R¹¹)-, R^{2d}CO-0-, R^{2d}SO₂N(R¹¹)-, (where R^{2d} is Het as defined above, aryl as defined above, or C₁-C₄-alkyl or C₂-C₄-alkenyl substituted with Het, Het-O-, aryl, or aryl-O-, each as defined above), 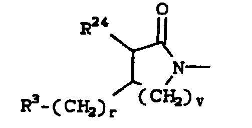
or 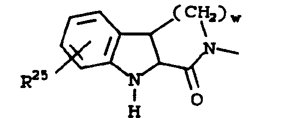
(where v is 1-to-3, w is 1 or 2, R²⁵ is C₁-C₄-alkyl, amino, mono- or di-C₁-C₄-alkylamino, -OH, C₁-C₄-alkoxy, -CO₂H, -C0₂-C₁-C₄-alkyl, -CONR^{2a}R^{2b}, -CF₃, halo, -NHCO-O-C₁-C₄-alkyl, -N(C₁-C₄-alkyl)CO-O-C₁-C₄-alkyl, -NHCO-C₁-C₄-alkyl or -N(C₁-C₄-alkyl)CO-C₁-C₄-alkyl, R³ and r are as defined above, R²⁴ is hydrogen, C₁-C₄-alkyl, or is A-N(H)- where A is independently selected from the definition of A as defined above);
G is
R²⁰⁻S(O)ₘ- (where m is 0-to-2 and R²⁰ is C₃-C₇-Cycloalkyl, aryl as defined above, Het as defined above or C₁-C₄-alkyl optionally substituted with one or two substituents chosen from the group consisting of C₁-C₄- alkoxy, -OH, -CO₂H, -C0₂-C₁-C₄-alkyl, -NH₂, -NH(C₁-C₄-alkyl), -N(C₁-C₄-alkyl)₂ and (C₁-C₄-alkyl)CO-O-), R¹⁷R¹⁸NS0₂- (where R¹⁷ and R¹⁸ are as defined above), 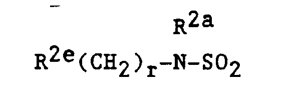
where r, R²a and R²e are as defined above) or 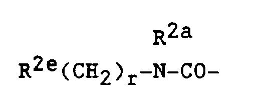
(where r, R²a, and R²e are as defined above; R²⁰CO- (where R²⁰ is as defined above), R²⁰OCO- (where R²⁰ is as defined above) or -CH(OH)CH₂Het (where Het is defined above);
A and B together may be -J-CH[(CH₂)ᵣ-R³]-K-;
K is
-CH₂-,
-CH(OH)-,
-CO-,
-NH-,
-O-,
-S-,
-SO-,
-SO₂-,
-NO-,
-P(O)O-;
J is
R²⁶⁻CO-(CH₂)_{d} (where d is 0 to 4, R²⁶ is -OH, -0-C₁-C₆-alkyl, -NR¹⁸R¹⁸, Het), R²⁷-SO₂ (where R²⁷ is -C₁-C₄-alkyl, aryl, Het), R²⁸, where R²⁸ is aryl, Het, C₁-C₄-alkyl optionally substituted with aryl, Het, -CO₂H, -C0₂-C₁-C₄-alkyl, -SO₂-C₁-C₄-alkyl, -SO₂Ar, -SO₂Het), R²⁸⁻NH-CO- where R²⁸ is as defined above; R¹ is
C₁-C₄-alkyl, aryl as defined above, unsubstituted, di-, or trisubstituted C₃-C₇-cycloalkyl, (where the substituents is/are selected from the group consisting of C₁-C₄-alkyl, trifluoromethyl, -OH, C₁-C₄-alkoxy, or halo) or a 5- or 6-membered ring saturated heterocycle containing one or two heteroatoms selected from the group consisting of N, O or S, optionally substituted with one or two substituents (where the substituents is/are selected from the group consisting of C₁-C₄-alkyl, C₁-C₄-alkoxy, halo, -NH₂ or -OH);
R¹⁵ is C₁-C₄-alkyl, aryl as defined above, imidazol-4-yl, thiazol-4-yl or thiazol-5-yl;
t is 1-to-4;
R¹⁶ is hydrogen or
C₁-C₄-alkyl optionally substituted with a substituent chosen from among the group consisting of C₁-C₄- alkyl, C₃-C₇-cycloalkyl, aryl as defined above, Het as defined above, -OH, -SO₃H, -CO₂H, CO₂-C₁-C₄-alkyl, -CO-Het, -NR¹⁷R¹⁸, -NHR¹⁸ -N(R¹⁷R¹⁸R¹⁹)⁺X⁻ (where X- R¹⁷ R¹⁸ and R¹⁹ as defined above), -S(O)ₘ-R²¹ (where m is as defined above and R²¹ is Het, aryl or C₁-C₄-alkyl the alkyl optionally substituted with a substituent chosen from among the group consisting of aryl, Het, -NH₂, -OH, -NH-C₁-C₄-alkyl or -N(C₁-C₄-alkyl)₂ ), -SO₂NH₂, -SO₂NR¹⁷R¹⁸ (where R¹⁷ and R¹⁸ are as defined above), -SO₂NHR¹⁸
(where R¹⁸ is as defined above) and -CH₂(OCH₂CH₂)ₓ-OC₁-C₄-alkyl (where x is as defined above);
Y is
-OCO-, -CH₂CO- or -CH₂CH(OH)- (where the Y substituent is inserted into formula I clockwise from left to right);
Wis O or NR²³, where R²³ is -H or C₁-C₄-alkyl;
Z is -NH₂, -OH, -OPO₃H₂, -OCOR²², -OCO-OR²² (where R²² is 5-indanyl or C₁-C₆-alkyl optionally substituted with Ph, -SO₃H, -CO₂H, -PO₃H₂, -NH₂, -NH(C₁-C₄-alkyl), -N(C₁-C₄-alkyl)₂, -N(C₁-C₄-alkyl)₃⁺X⁻ where X- is defined above), -OCHR22a-_{OCOR}22b (where R²²a and R^{22b} are C₁-C₄-alkyl); 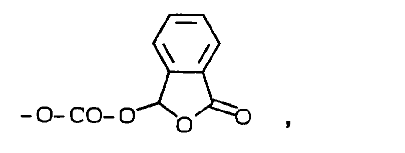
or -O-CO-CH₂O-(CH₂CH₂O)ₓ-C₁-C₄-alkyl or -O-CO-O(CH₂CH₂O)ₓ-C₁-C₄-alkyl (where x is as defined above); and
D is absent, -CH₂0, or -CH₂S-.
2. A compound according to Claim 1 in which Het is selected from the group consisting of piperidine, pyrryl, pyrrolinyl, quinuclidinyl, isoquinuclidinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolidinyl, isoxazolyl, isoxazolidinyl, morpholinyl, thiazolyl, thiazolidinyl isothiazolyl, isothiazolidinyl, indolyl, quinolinyl, isoquinolinyl, benzimidazolyl, benzothiazolyl, benzoxazolyl, furyl, thienyl or benzothienyl.
3. The compound of claim 1 wherein A is selected from the group consisting of -H or -CH₃.
4. The compound of claim 1 wherein A is selected from the group consisting of:
Boc- ,
Et OC-,
i-PrSO₂- ,
CH₃( OCH₂CH₂)₃OCO-, 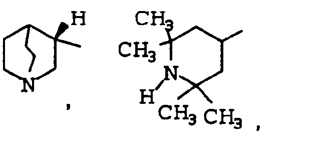 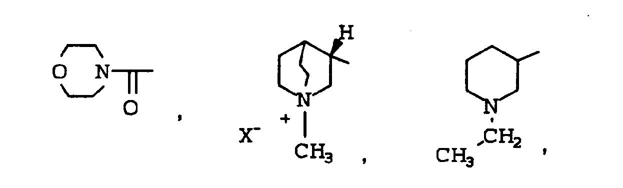 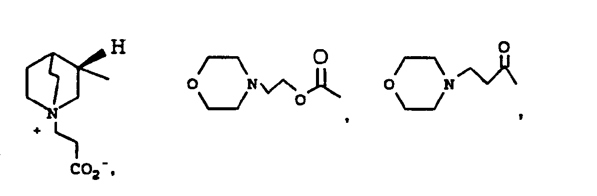 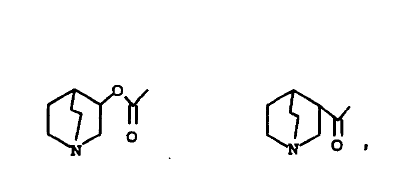 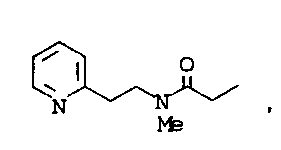 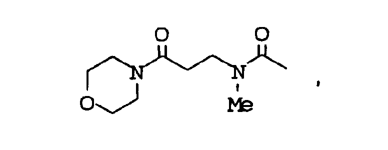 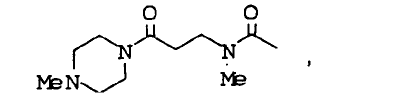 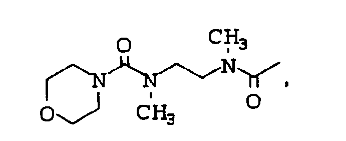 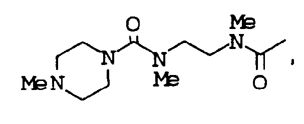
or 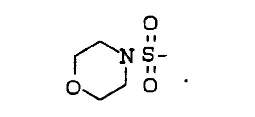
5. The compound of claim 1 wherein B is selected from the group consisting of: 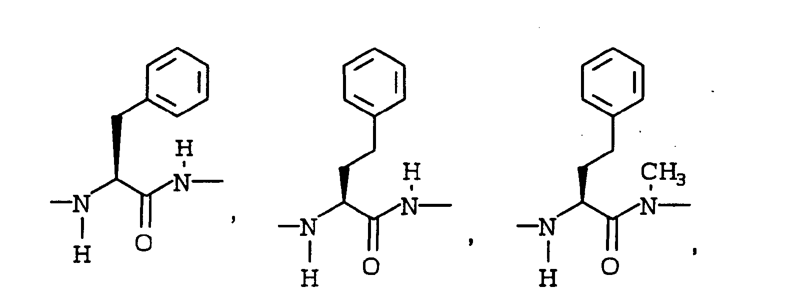 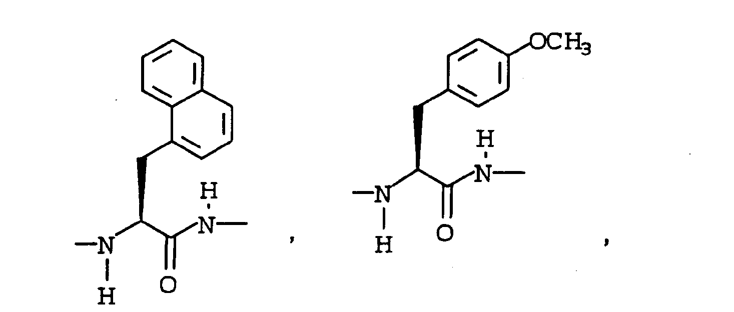 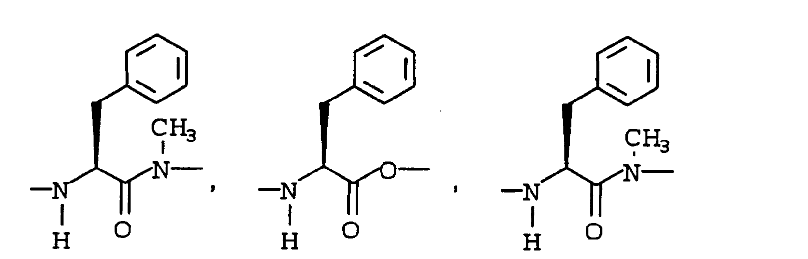 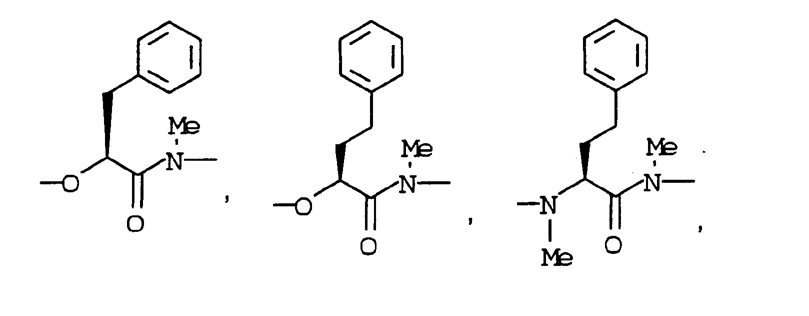 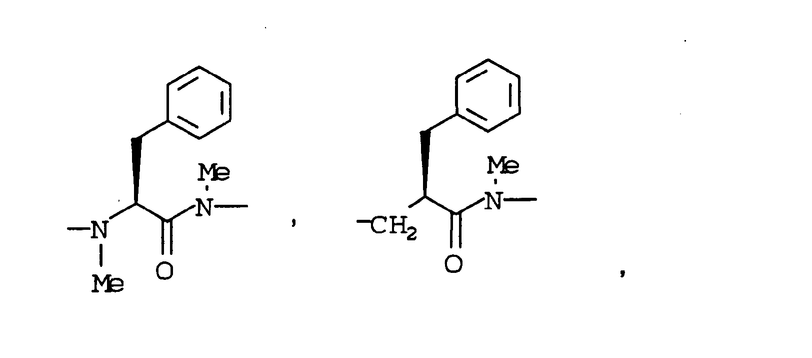 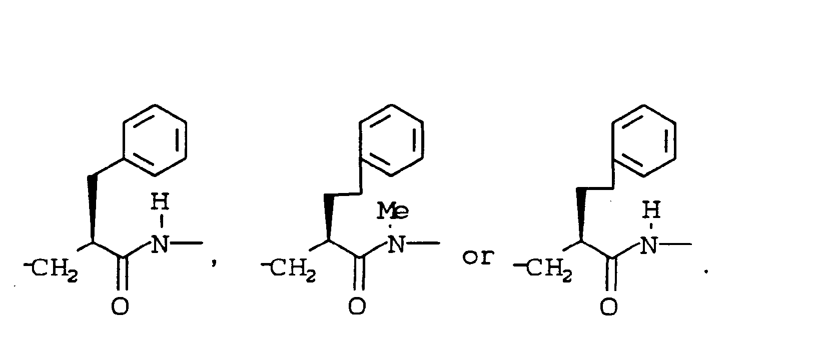
6. The compound of claim 1 wherein A and B taken together are selected from the group consisting of: 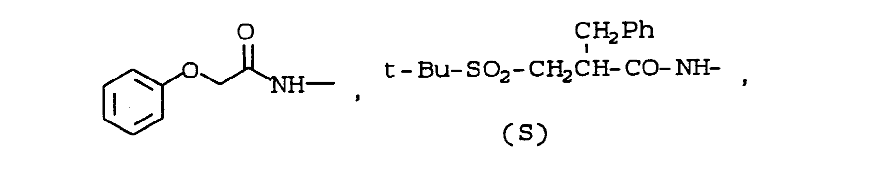 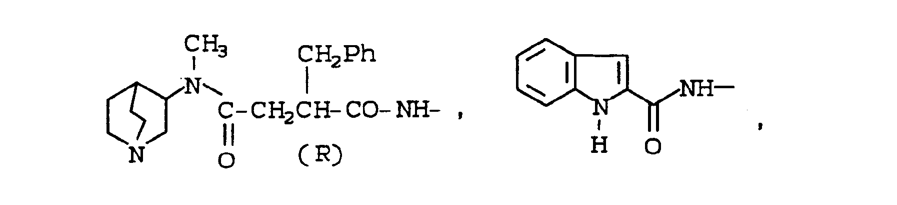 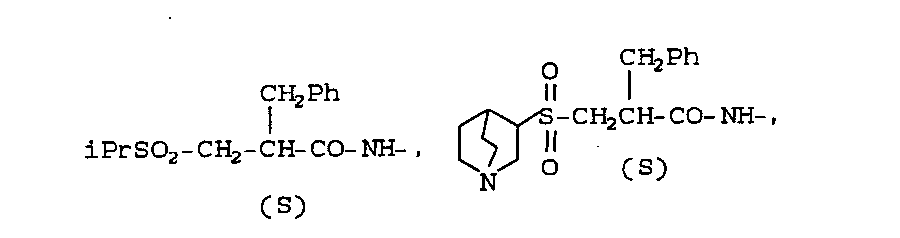 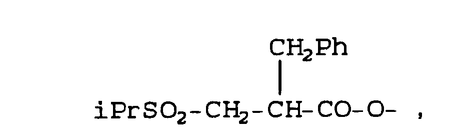 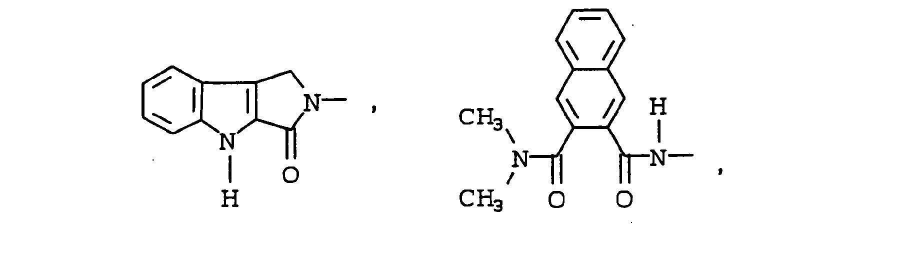 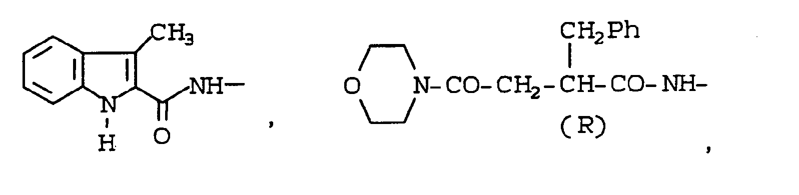 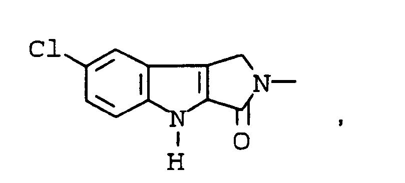 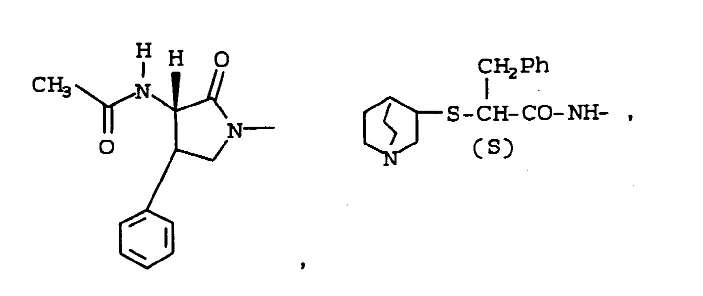 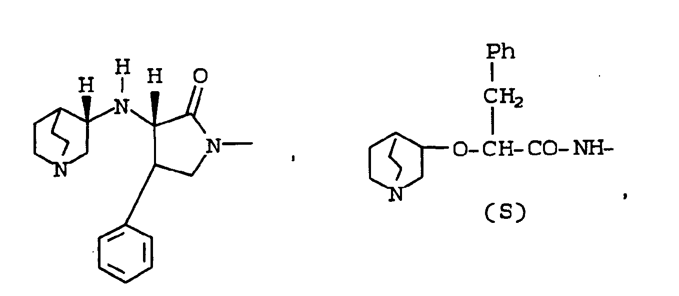 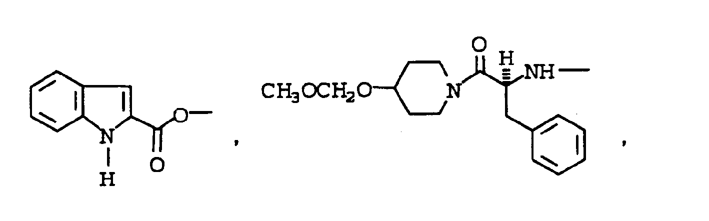 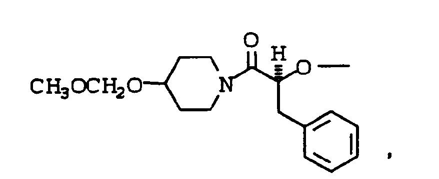 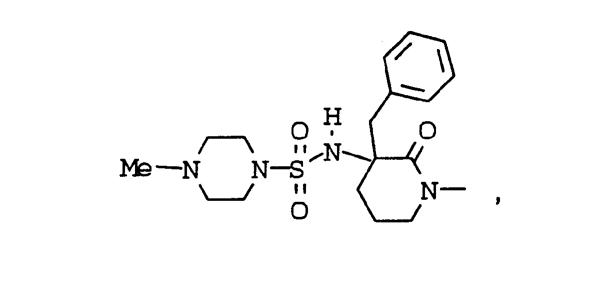 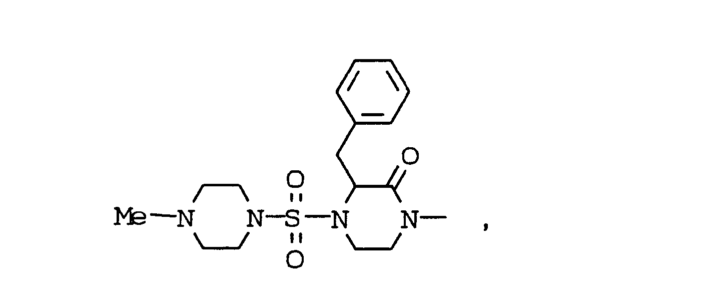 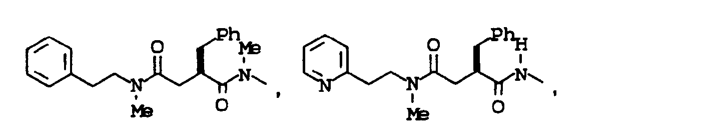 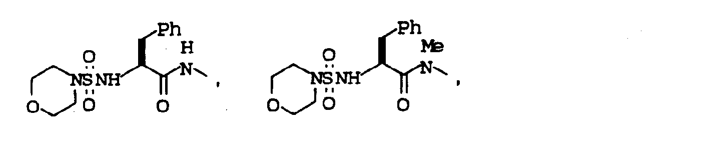 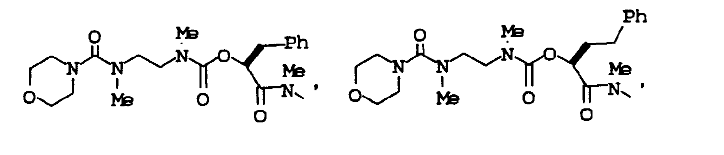 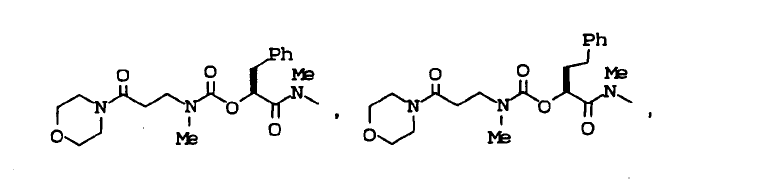 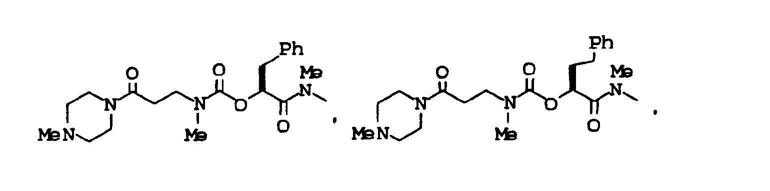 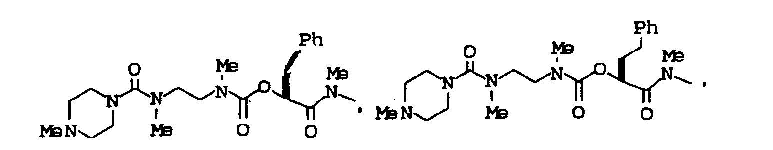 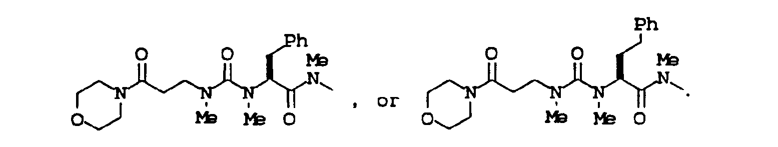
7 .A compound of the formula: 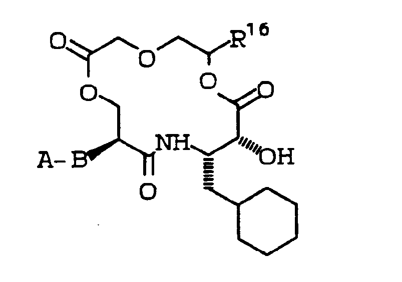
where A-B and R¹ are selected from 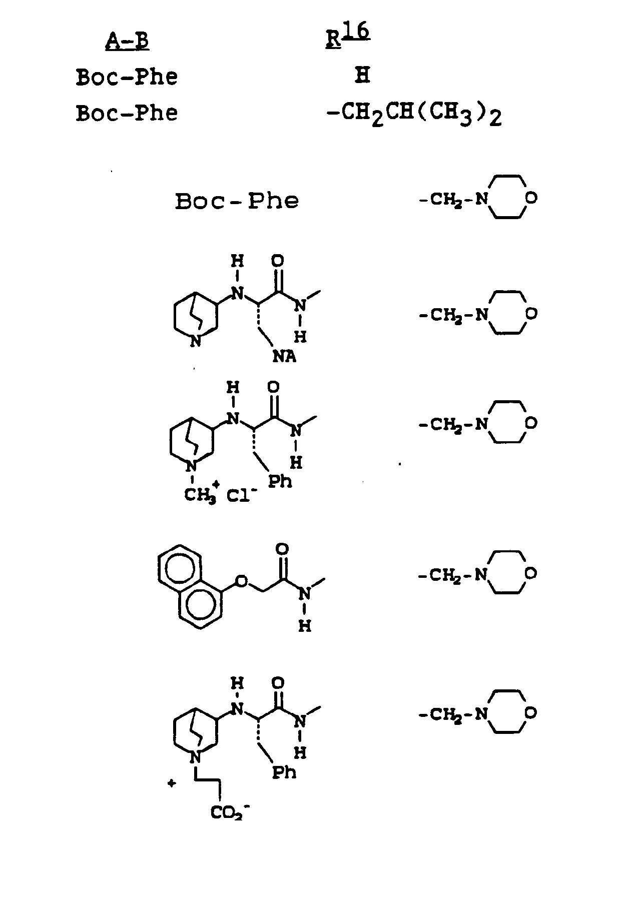 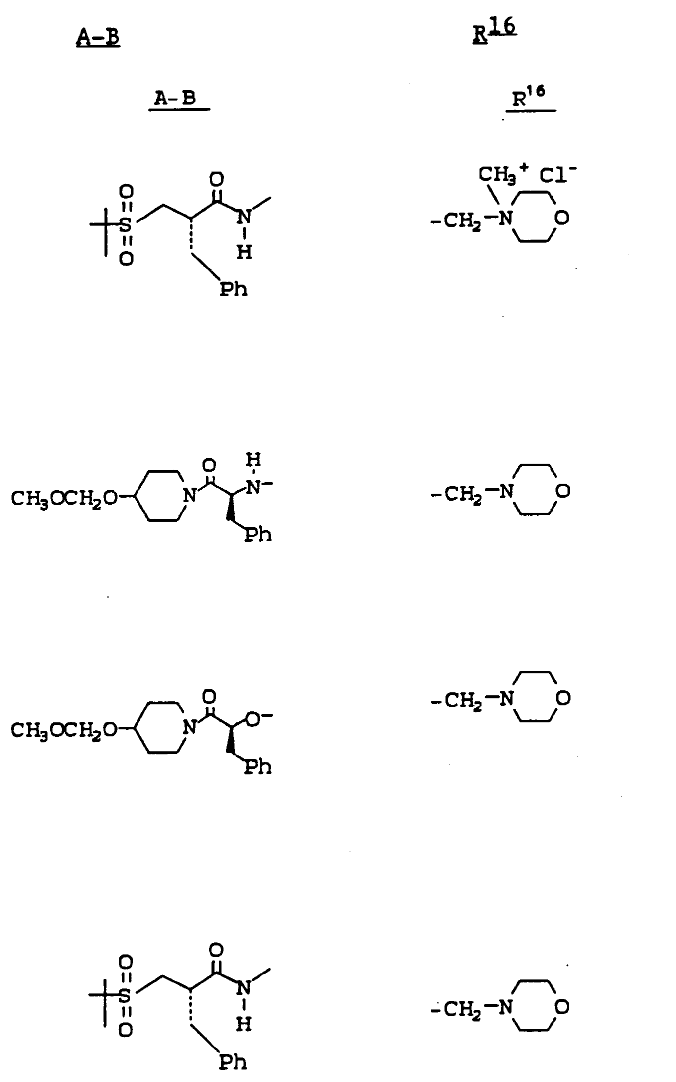 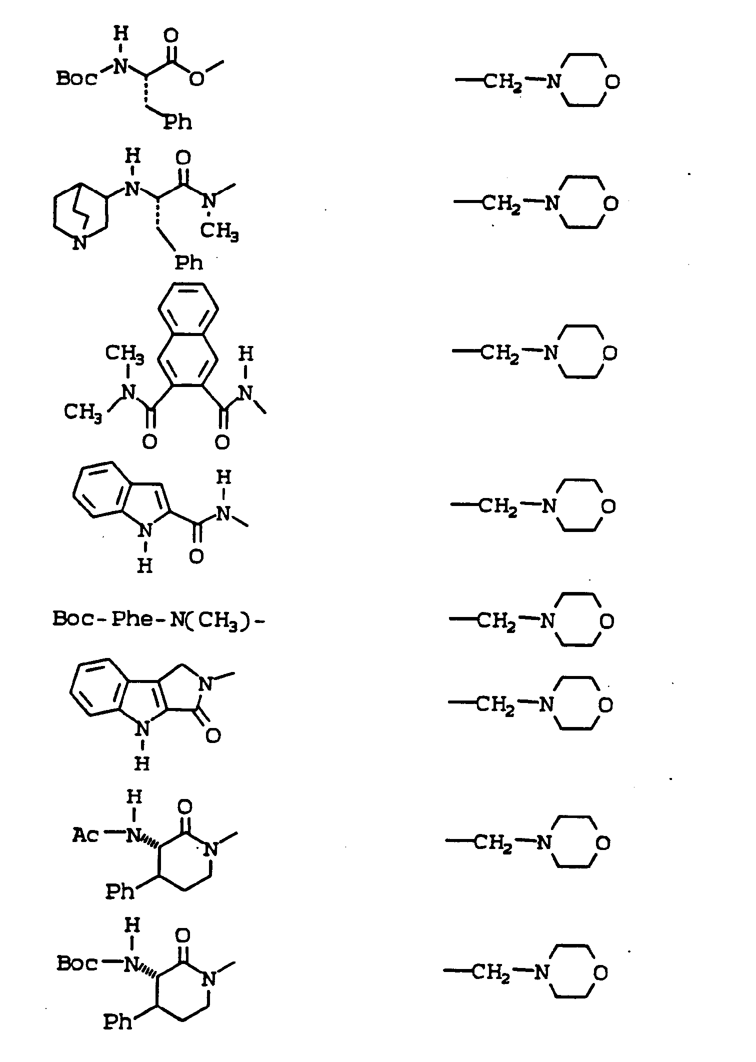 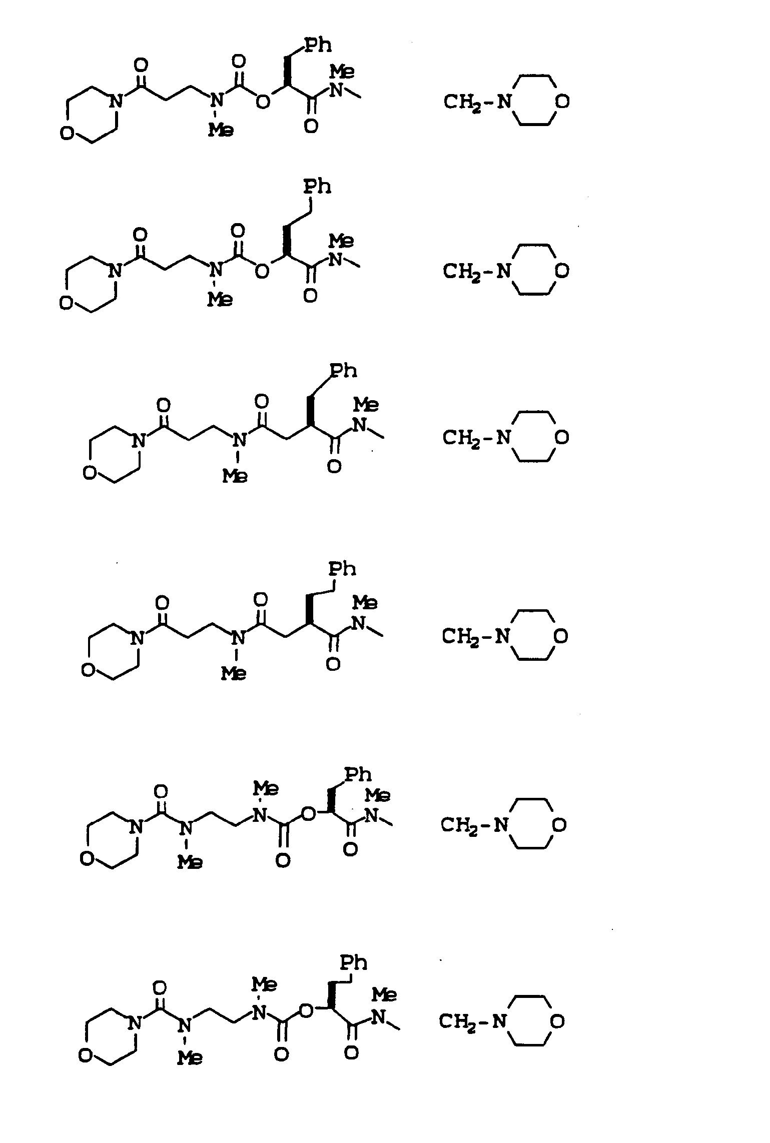 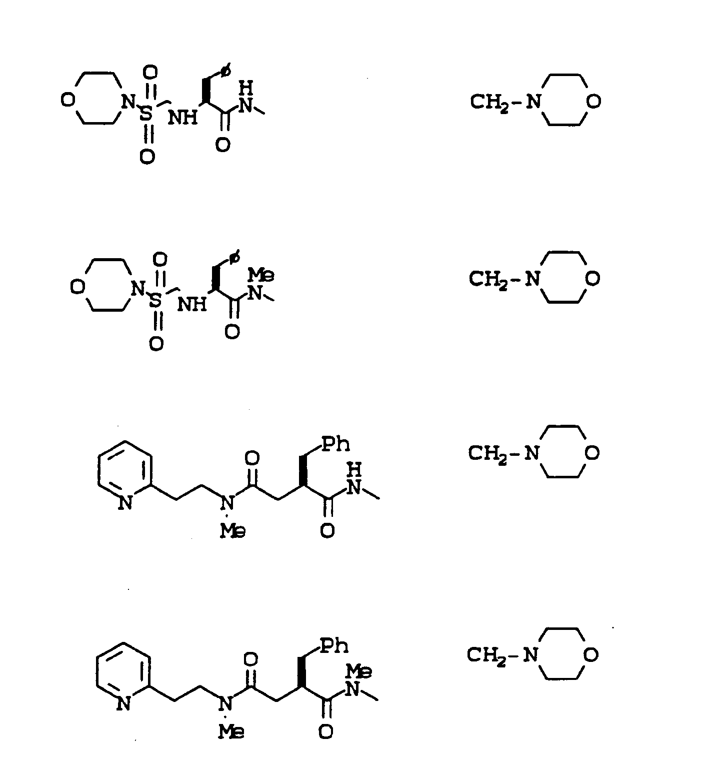
8. A pharmaceutical composition useful for treating renin associated hypertension, hyperaldosteronism, congestive heart failure and glaucoma comprising a pharmaceutically acceptable carrier and a therapeutically effective amount of a compound according to Claims 1-7.
9. The use of a compound according to Claims 1-7 for the manufacture of a medicament for treating renin associated hypertension, hypoaldosteronism, congestive heart failure and glaucoma.
